# EUROPEAN PATENT APPLICATION

(11) **EP 4 365 170 A1**
(43) Date of publication of application: **08.05.2024**
(21) Application number: 22832059.4
(22) Date of filing: 28.06.2022
(51) Int. Cl.: C07D 401/04, C07D 401/14, A61K 31/454, A61K 31/4545, A61P 35/00

(54) **AMIDE COMPOUND AND USE THEREOF**

(30) Priority: 28.06.2021 CN 202110722075
(71) Applicant: Chengdu Fendi Pharmaceutical Co. Ltd., Chengdu, Sichuan 610200 (CN)
(72) Inventor: HU, Wei, Chengdu, Sichuan 610200 (CN); WANG, Liqiang, Chengdu, Sichuan 610200 (CN); CAI, Xin, Chengdu, Sichuan 610200 (CN)
(74) Representative: Elzaburu S.L.P.
(86) International application number: PCT/CN2022/102001
(87) International publication number: WO 2023/274246

(57) **Abstract**

Provided are a compound as represented by formula (I), and a pharmaceutically acceptable salt, tautomer, mesomer, racemate, stereoisomer, metabolite, metabolic precursor, or drug precursor thereof, and application thereof as a protein regulator, in particular a GSPT1 protein regulator, and a preparation method.

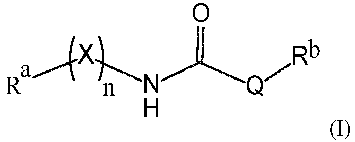

## Description

### TECHNICAL FIELD

The present invention relates to the field of medicine, in particular to an amide compound, its use and preparation method.

### BACKGROUND

Protein mutations, expression imbalances, allostery and dysfunction can cause many diseases. Protein synthesis, cell growth and proliferation are strictly regulated processes in space and time. Abnormal regulation of these processes may lead to uncontrolled growth, proliferation, and migration of cells, resulting in the occurrence of diseases such as cancer, aging, and viral infections.

GSPT1 is closely associated with diseases such as cancer, and the translation termination factor GSPT1 (eRF3a) mediates stop codon recognition and promotes the release of nascent peptides from ribosomes. In addition to its role in translational termination, GSPT1 is involved in several other key cellular processes, such as cell cycle regulation, cytoskeletal organization, and apoptosis. GSPT1 has been found to be an oncogenic driver in several cancer types, including breast, hepatocellular carcinoma, gastric and prostate cancers.

GSPT1 is significantly upregulated in cancer tissues and cell lines, and high GSPT1 expression is positively correlated with tumor size. For example, GSPT1 depletion can effectively inhibit the proliferation and migration of cancer cells and induce apoptosis of invasive colon cancer cells in vitro, and inhibit the tumorigenicity of HCT116 colon cancer cells in vivo (Aging (Albany NY), 2021, 13(7): 10354).

At present, there are no GSPT1-based protein-modulating medicaments on the market, and the only such protein-modulating medicament under clinical development is Celegen's CC-90009, which is being developed by intravenous administration and is undergoing preclinical phase I development. Other protein-modulating medicaments are currently in early development, such as BioTheryX Inc's protein modulator BTX-1188 (WO2017201069), which is still in the preclinical stage. Oral administration is the most commonly used method of administration because it is convenient, safe, and economical compared to injectable administration. In addition, the small size of the protein modulator molecule and the structural changes can greatly alter the metabolic and biological activity of the medicament. For example, the change of one atom in the molecular glue or PROTACs molecule may cause the compound to lose the degradation activity of a specific protein, therefore, changing the chemical structure of the modulator may improve the bioavailability and safety of the medicament.

Generally, protein dysfunction is a direct result of protein overexpression, underexpression, or changes in protein sequence and structure. In addition, wild-type proteins with normal function and expression levels can also cause functional abnormalities in a cell environment. Therefore, regulating certain wild-type proteins in abnormal cells is necessary for the treatment and prevention of disease. At present, it has not been reported that propiolamide or acrylamide compounds are used as small molecule regulators for GSPT1 and other protein, therefore, it is of great significance to develop new propiolamide or acrylamide compounds as small molecule regulators for GSPT1 and other protein.

### SUMMARY OF THE INVENTION

The purpose of the present invention is to provide a compound that is used to modulate proteins.

Another purpose of the present invention is to provide a chemical compound, or a pharmaceutically acceptable salt, tautomer, mesomer, racemate, stereoisomer, metabolite, metabolic precursor or prodrug, and intermediates thereof, manufacturing methods, and pharmaceutical composition comprising the compound, which have obvious protein regulatory effects and the protein includes GSPT1. The compounds disclosed are capable of effectively alleviating or treating cancer and other related diseases.

Another purpose of the present invention provides a protein modulator to regulate expression levels of protein, including GSPT1.

The present invention also provides methods for modulating protein-mediated diseases, such as GSPT1-mediated diseases, disorders, conditions, or responses.

The present invention also provides methods for treating, improving, or preventing protein-mediated diseases, conditions, and symptoms, such as GSPT1-mediated diseases, conditions, and symptoms.

Specifically, the present invention provides a propiolamide-based and acrylamide-based compound that are completely different from the prior art, and intermediate, manufacturing method, pharmaceutical composition and use thereof. The compounds containing propiolamide and acrylamide described herein have obvious regulatory effects on GSPT1 and other proteins, and is useful as an agent for alleviating or treating cancer and other related diseases.

Specifically, the present invention provides the compound of formula (I), or a pharmaceutically acceptable salt, tautomer, mesomer, racemate, stereoisomer, metabolite, metabolic precursor, or prodrug thereof.

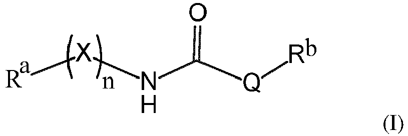

Wherein, R^{a} is represented by the formula (II), 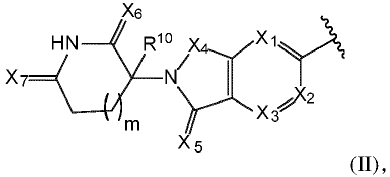
X₁ is Nor CR¹;
X₂ is N or CR²;
X₃ is N or CR³;
X₄ is CH₂, O, S, NR⁴, C=O, or C=S;
X₁, X₂, X₃, and X₄ are not N at the same time;
X₅ and X₇ are each independently O or S;
X₆ is O, S, or X₆ is absent;
R¹, R², R³, and R⁴ are each independently selected from hydrogen, deuterium, C₁₋₃ alkyl, C₁₋₃ alkoxy, C₃₋₆ cycloalkyl, 3- to 6- membered heterocycloalkyl, 4- to 7- membered aryl, 4- to 7- membered heteroaryl, hydroxyl, halogen, cyano, or -N(R⁵)(R⁶);
R⁵ and R⁶ are each independently selected from hydrogen, C₁₋₃ alkyl, C₁₋₃ alkoxy, C₃₋₆ cycloalkyl, 4- to 7- membered heterocycloalkyl, 4- to 7- membered heteroaryl, or R⁵ and R⁶ together with the nitrogen atom to which they are attached form a 3- to 6- membered mono saturated nitrogen-containing ring;
R¹⁰ is hydrogen, deuterium, C₁₋₃ alkyl, C₁₋₃ alkoxy, hydroxyl, halogen, or cyano;

In one embodiment, R¹⁰ is hydrogen, deuterium, or halogen.

In one embodiment, R¹⁰ is hydrogen, fluorine.

m is an integer from 0-3; preferably an integer from 0 to 2, and preferably m is 1.

R^{b} is C₁₋₁₂ alkyl, Si₁₋₃ alkyl, C₁₋₁₂ alkoxy, C₂₋₁₂ alkenyl, C₂₋₁₂ alkyne, C₃₋₁₂ cycloalkyl, 3- to 12-membered heterocycloalkyl, 5- to 12- membered aryl, 5- to 12- membered heteroaryl, 6- to 12- membered dicyclic carbon ring, 6- to 12- membered partially unsaturated bicyclic carbon ring, 8- to 12- membered benzoheterocyclic, 6- to 12- membered bicyclic heteroaryl, 10- to 15- membered tricyclic carbon ring or 10- to 15- membered partially unsaturated tricyclic carbon ring, wherein the C₁₋₁₂ alkyl, C₁₋₁₂ alkoxy, C_{2 12} alkenyl, C₂₋₁₂ alkyne, C₃₋₁₂ cycloalkyl, 3- to 12- membered heterocycloalkyl, 5- to 12- membered aryl, 5- to 12- membered heteroaryl, 6- to 12- membered bicyclic carbon rings, 6- to 12- membered partially unsaturated bicyclic carbon rings, 6- to 12- membered bicyclic heteroaryl, 10- to 15- membered tricyclic carbon rings, and the 10- to 15- membered partially unsaturated tricyclic carbon rings, are optionally substituted with one or more R;

In one embodiment, R^{b} is C₁₋₁₂ alkyl, C₁₋₁₂ alkoxy, C₂₋₁₂ alkenyl, C₂₋₁₂ alkyne, C₃₋₁₂ cycloalkyl, 3- to 12- membered heterocycloalkyl, 5- to 12- membered aryl, 5- to 12- membered heteroaryl, 6-12 bicyclic carbon ring, 6- to 12- membered partial unsaturated bicyclic carbon ring, 8- to 12- membered benzoheterocyclic, 6- to 12- membered bicyclic heteroaryl, 10- to 15- membered tricyclic carbon ring and 10- to 15- membered partially unsaturated tricyclic carbon rings, wherein the C₁₋₁₂ alkyl, C₁₋₁₂ alkoxy, C₂₋₁₂ alkenyl, C₂₋₁₂ alkyne, C₃₋₁₂-cycloalkyl, 3- to 12- membered heterocycloalkyl, 5- to 12- membered aryl, 5- to 12- membered heteroaryl, 6- to 12- membered bicyclic carbon ring, 6- 12 membered partial unsaturated bicyclic carbon ring, the 6- to 12- membered bicyclic heteroaryl, the 10- to 15- membered tricyclic carbon ring, or the 10- to 15- membered partial unsaturated tricyclic carbon ring are optionally substituted with one or more R.
X is methylene, each hydrogen on the methylene is substituted with one or more deuterium or halogen;
Preferably, one or more hydrogen atoms on the methylene is optionally substituted with deuterium or fluorine.
Q is a bond, -CR⁷=C-, -C≡C-,-C(R⁷)=N-,-N=C(R⁷)- or -N=N-; preferably, Q is -C=C-;

In one embodiment, Q is -C≡C-,-C(R⁷)=N-,-N=C(R⁷)- or -N=N-.

In one embodiment, Q is -C=C-.
R⁷ is independently selected from hydrogen, deuterium, C₁₋₆ alkyl, C₁₋₆ alkoxy, C₃₋₉ cycloalkyl, 3- to 9- membered heterocycloalkyl, 5- to 9- membered aryl, 5- to 9- membered heteroaryl, hydroxyl, halogen, cyano, wherein the C₁₋₆ alkyl, C₁₋₆ alkoxy, C₃₋₉ cycloalkyl, 3- to 9- membered heterocycloalkyl, 5- to 9-membered aryl, and 5- to 9- membered heteroaryl are optionally substituted with one or more R;
R is selected from deuterium, phosphonates, C₁₋₆ alkyl, C₁₋₆ alkoxy, S₁₋₆ alkoxy, C₁₋₆ alkoxy carbonyl, -C(O)-C₁₋₆ alkyl, -C(O)-C₁₋₆ alkoxy, -O-C(O)-C₁₋₆ alkyl, -O-C(O)-3- to 9- membered heterocycloalkyl, C₃₋₉ cycloalkyl, 3- to 9- membered heterocycloalkyl, 5- to 9- membered aryl, 5- to 9-membered heteroaryl, carboxy, hydroxyl, halogen, cyano, amino, nitro, or -N(R⁸)(R⁹), wherein C₁₋₆ alkyl, S₁₋₆ alkoxy, ₋C(O)-C₁₋₆ alkyl, -C(O)-C₁₋₆ alkoxy, ₋O-C(O)-C₁₋₆ alkyl, -O-C(O)-C₆₋₁₂ heterocycloalkyl, 5-to 9- membered aryl, and 3- to 9- membered heterocycloalkyl are optionally substituted with one or more halogen, carboxyl, nitro, amino, C₁₋₆ alkyl, and 6-membered heterocycloalkyllor -O-C(O)-C₁₋₆ alkyl; and H atoms on the phosphonate are optionally substituted with one or more R¹¹;
R¹¹ is selected from methyl or phenyl, or two R¹¹ together with the atoms to which they are attached O and P form a dioxophosphate heterocyclic alkyl ring;

In one embodiment, R is selected from deuterium, C₁₋₆ alkyl, C₁₋₆ alkoxy, C1-6 alkoxycarbonyl, C₃₋₉ cycloalkyl, 3- to 9- membered heterocycloalkyl, 5- to 9- membered aryl, 5- to 9- membered heteroaryl, carboxy, hydroxyl, halogen, cyano, -N(R⁸)(R⁹).
R⁸ and R⁹ are each independently selected from hydrogen, C₁₋₆ alkyl, C₁₋₆ alkoxy, C₃₋₉ cycloalkyl, 4-to 7- membered heterocycloalkyl or 4- to 7- membered heteroaryl, wherein the C₁₋₆ alkyl is optionally substituted with one or more hydroxyl, halogen, cyano or C₁₋₃ alkoxy; or R⁸ and R⁹ together with the nitrogen atom to which they are attached form 3- to 6- membered mono saturated nitrogen-containing ring;

n is an integer from 0 to 5.

In one embodiment, n is an integer from 0 to 3.

In one embodiment, n is an integer selected from 0 to 2.

In one embodiment, n is 0 or 1.

In one specific embodiment, n is 1.

Any one of the aforementioned compounds, or a pharmaceutically acceptable salt, tautomer, mesomer, racemate, stereoisomer, metabolite, metabolic precursor, or prodrug thereof, wherein R^{a} is defined as in formula (IIa) or (IIb);

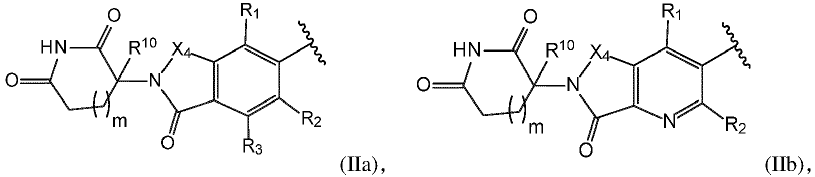

Wherein,
X₄ is -CH₂ or -C=O;
R¹, R², R³ and m are defined as in formula (II);
Any one of the aforementioned compounds, or a pharmaceutically acceptable salt, tautomer, mesomer, racemate, stereoisomer, metabolite, metabolic precursor, or prodrug thereof, wherein R^{a} is selected from:

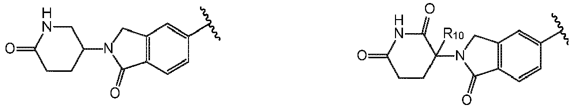

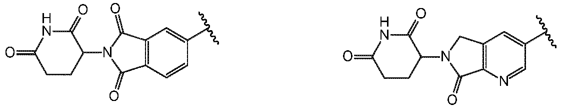

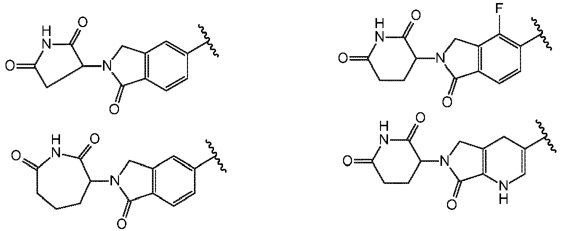

Any one of the aforementioned compounds, or a pharmaceutically acceptable salt, tautomer, mesomer , racemate, stereoisomer, metabolite, metabolic precursor or prodrug thereof, wherein R^{b} is Si₁₋₃ alkyl, C₁₋₆ alkyl, C₁₋₆ alkoxy, C₂₋₆ alkenyl, C₂₋₆ alkynyl, C₃₋₈ cycloalkyl, 5- to 8- membered heterocyclic alkyl, 5- to 7- membered aryl, 5- to 7- membered heteroaryl, 8- to 12- membered bicyclic carbon ring, 8-to 12- membered partially unsaturated bicyclic carbon ring, 8- to 12- membered benzoheterocyclics, 8-to 12- membered bicyclic heteroaryl, or 10- to 15- membered tricyclic carbon rings, wherein C₁₋₆ alkyl, C₁₋₆ alkoxy, C₂₋₆ alkenyl, C₂₋₆ alkyne, C₃₋₈ cycloalkyl, 5- to 8- membered heterocycloalkyl, 5- to 7-membered aryl, 5- to 7- membered heteroaryl, 8- to 12- membered bicyclic carbon ring, 8- to 12-membered partially unsaturated bicyclic carbon ring, 8- to 12- membered bicyclic heteroaryl, and 10- to 15- membered tricyclic carbon ring are optionally substituted with one or more R.

In one embodiment, R^{b} is C₁₋₆ alkyl, C₁₋₆ alkoxy, C₂₋₆ alkenyl, C₂₋₆ alkyne, C₃₋₈ cycloalkyl, 5- to 8-membered heterocyclic alkyl, 5- to 7- membered aryl, 5- to 7- membered heteroaryl, 8- to 12- membered bicyclic carbon rings, 8- to 12- membered partial unsaturated bicyclic carbon rings, 8- to 12- membered benzoheterocyclic, 8- to 12- membered bicyclic heteroaryl, 10- to 15- membered tricyclic carbon rings, wherein the C₁₋₆ alkyl, C₁₋₆ alkoxy, C₂₋₆ alkenyl, C₂₋₆ alkyne, C₃₋₈ cycloalkyl, 5- to 8- membered heterocyclic alkyl, 5- to 7- membered aryl, 5- to 7- membered heteroaryl, 8- to 12- membered bicyclic carbon rings, 8- to 12- membered partially unsaturated bicyclic carbon rings, 8- to 12- membered bicyclic heteroaryl, and 10- to 15- membered tricyclic carbon rings are optionally substituted with one or more R.

In one embodiment, R^{b} is silyl, C₁₋₆ alkyl, C₃₋₆ cycloalkyl, 5- to 6- membered heterocyclicalkyl, 5-to 6- membered aryl, 5- to 6- membered heteroaryl, 8- to 12- membered benzoheterocyclic, 10-membered tricyclic carbon ring, wherein the C₁₋₆ alkyl, C₃₋₆ cycloalkyl, 5- to 6- membered heterocyclicalkyl, 5- to 6- membered aryl, 5- to 6- membered heteroaryl, 8- to 12- membered benzoheterocyclic, and 10-membered tricyclic carbon ring are optionally substituted with one or more R.

In one embodiment, R^{b} is C₁₋₆ alkyl, C₃₋₆ cycloalkyl, 5- to 6- membered heterocyclicalkyl, 5- to 6-membered aryl, 5- to 6- membered heteroaryl, 8- to 12- membered benzoheterocyclic, 10 membered n tricyclic carbon ring, wherein the C₁₋₆ alkyl, C₃₋₆ cycloalkyl, 5- to 6- membered heterocyclic alkyl, 5- to 6- membered aryl, 5- to 6- membered heteroaryl, 8- to 12- membered benzoheterocyclic, and 10-membered tricyclic carbon ring are optionally substituted with one or more R.

In one embodiment, preferably, R^{b} is methyl, ethyl, propyl, tert-butyl, methoxy, trimethylsilyl, cyclopropyl, cyclopentyl, cyclohexyl, pyrrolidyl, piperidinyl, phenyl, pyridyl, pyrimidinyl, thienyl, pyrazolyl, imidazolyl, pyrazinyl, benzofuranyl, benzothiazolyl, benzimidazolyl, benzoxazolyl, indolyl, indazolyl, quinolinyl, quinolinonyl, 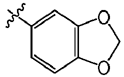 or 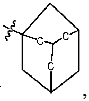 wherein methyl, ethyl, propyl, tert-butyl, methoxy, phenyl, pyridyl, pyrimidinyl, pyrazinyl, thienyl, pyrazolyl, imidazolyl, pyrazinyl, benzofuranyl, benzothiazolyl, benzimidazoly, benzoxazolyl, indolyl, indazolyl, quinolinyl, and quinolinonyl are optionally substituted with one or more R.

In one embodiment, R^{b} is methyl, ethyl, propyl, tert-butyl, methoxy, cyclopropyl , cyclohexyl, piperidinyl, phenyl, pyrimidinyl, pyrimidinyl, thienyl, pyrazolyl, imidazolyl, 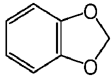 or 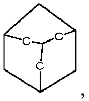 wherein the methy, ethyl, propyl, tert-butyl, methoxy, phenyl, pyridyl, pyrimidinyl, pyrazinyl, thienyl, pyrazolyl, and imidazolyl are optionally substituted with one or more R.

In one preferred embodiment, R^{b} is phenyl or thienyl.

Any one of the aforementioned compounds, or a pharmaceutically acceptable salt, tautomer, mesomer, racemate, stereoisomer, metabolite, metabolic precursor or prodrug thereof, wherein, R is selected from deuterium, phosphate ester group, C₁₋₄ alkyl, Si₁₋₄ alkoxy, C₁₋₄ alkoxy, C₁₋₄ alkoxycarbonyl, -C(O)-C₁₋₄ alkyl, -C(O)-C₁₋₄ alkoxy, -OC(O)-C₁₋₄ alkyl, -OC(O)-6-membered heterocycloalkyl, pyrrolidinyl, Phenyl, carboxyl, hydroxyl, halogen, cyano, amino or nitro; said C1-4 alkyl, Si 1 -4 alkoxy, -C(O)-C₁₋₄ alkyl, -C(O)-C₁₋₄ alkoxy, -OC(O)-C₁₋₄ alkyl, -OC(O)-6-membered heterocycloalkyl and phenyl are optionally substituted by one or more halogen , methyl, tert-butyl, carboxyl, nitro, amino, piperidinyl or -OC(O)-C₁₋₄ alkyl;

In one embodiment, R is selected from deuterium, C₁₋₄ alkyl, C₁₋₄ alkoxy, C₁₋₄ alkoxycarbonyl, phenyl, carboxy, hydroxyl and halogen.

In one embodiment, R is selected from methyl, ethyl, hydroxyl, cyano, amino, nitro, methoxycarbonyl, ethoxycarbonyl, tert-butoxycarbonyl, carboxy, phenyl, fluorine, chlorine, bromine, trifluoromethyl, nitrophenyl, aminophenyl, -CH₂CF₂, -C(O)CH₃, -C(O) CF₃, 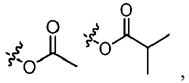 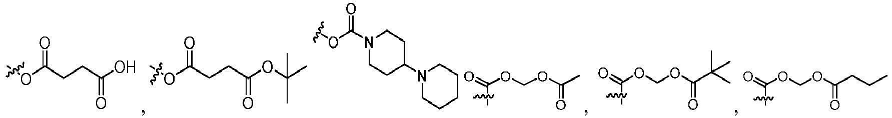 or

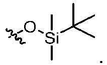

In one embodiment, R is selected from methyl, ethyl, fluorine, chlorine, bromine, nitro, amino, ethoxycarbonyl, trifluoromethyl, methoxy, tert-butoxycarbonyl, nitrophenyl, aminophenyl, -CH₂CF₂, or -C(O)CH₃.

In one embodiment, R is selected from methyl, ethyl, methoxy, ethoxycarbonyl, tert-butoxycarbonyl, carboxy, phenyl, fluorine, chlorine.

Any one of the aforementioned compounds, or a pharmaceutically acceptable salt, tautomer, mesomer, racemate, stereoisomer, metabolite, metabolic precursor, or prodrug thereof, wherein R^{b} is - CH₃, -CH₂CH₃, -CH₂CH₂ CH₃, -CH₂CH₂CH₂CH₂CH₃, -C(CH₃)₃, -CH₂OH, cyclopropyl, cyclopentyl, cyclohexyl, phenyl, trimethylsilyl, 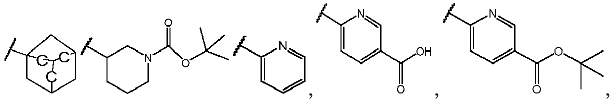 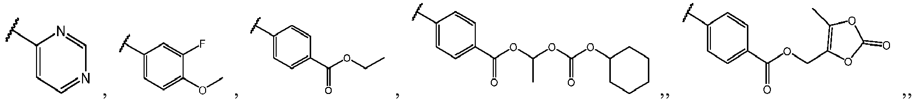 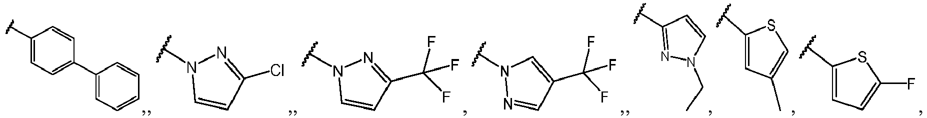 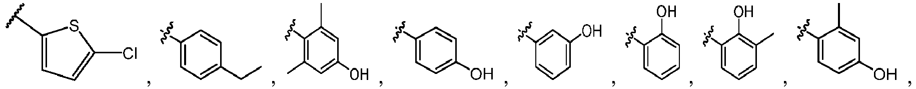 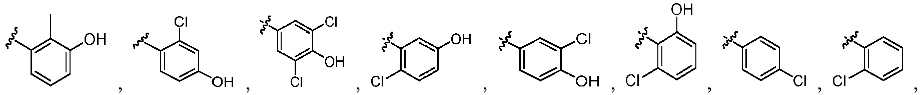 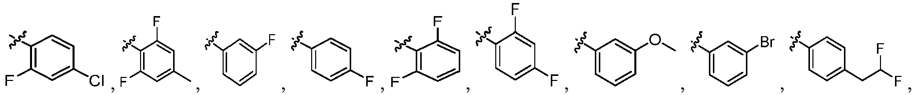 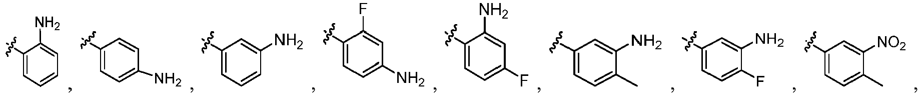 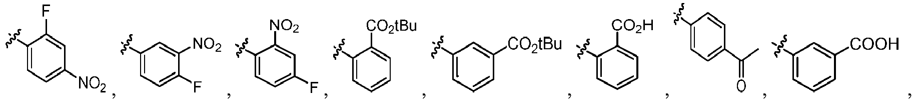 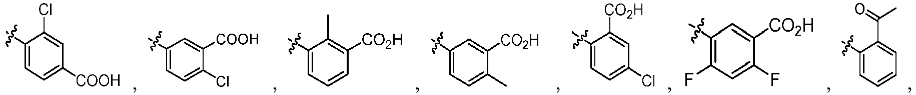 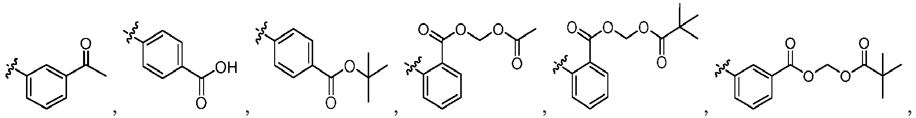 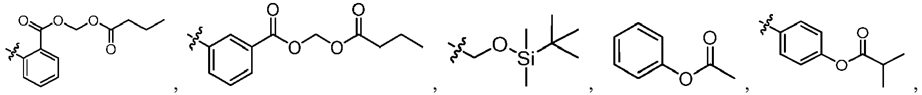 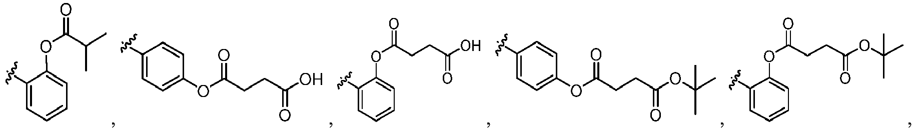 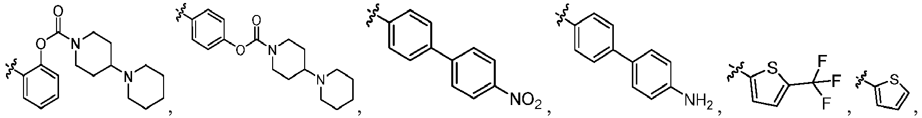 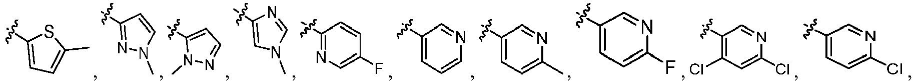 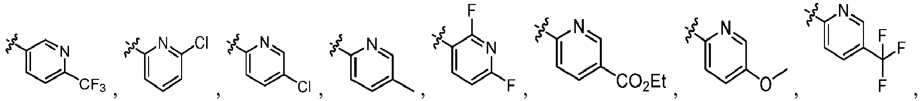 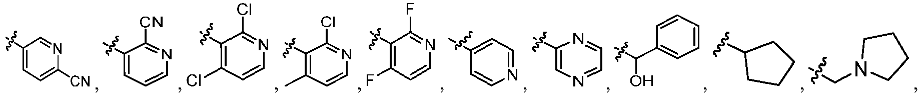 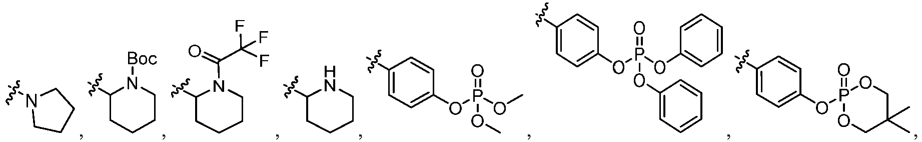 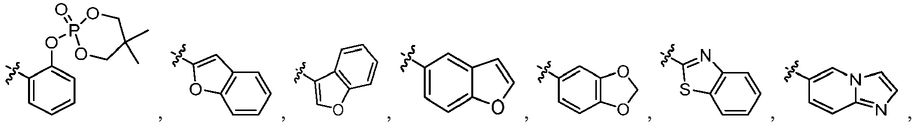 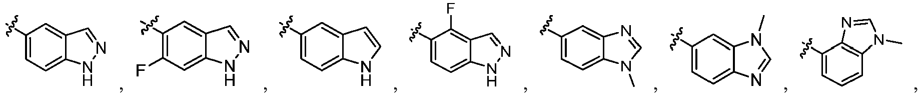 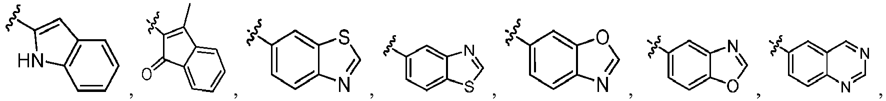 or

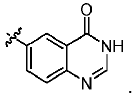

In one embodiment, R^{b} is -CH₃, -CH₂CH₃, -CH₂CH₂CH₃, -CH₂CH₂CH₂CH₂CH₃, -C(CH₃)₃, - CH₂OH, cyclopropyl, cyclohexyl, phenyl,

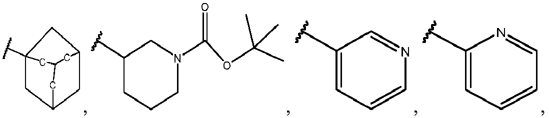

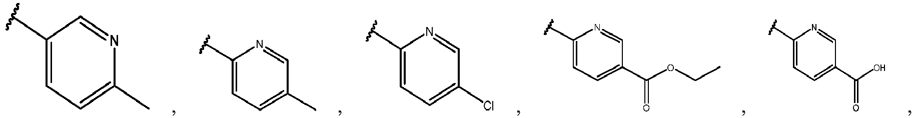

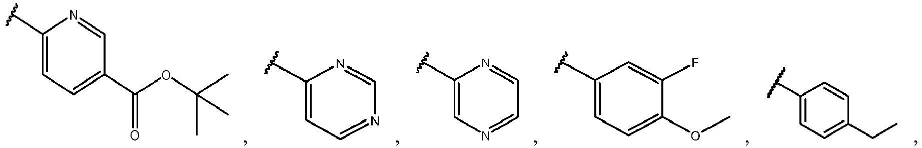

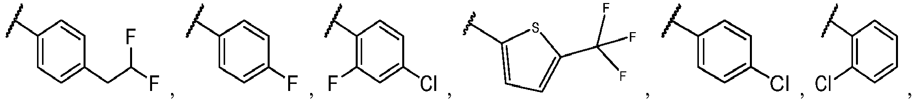

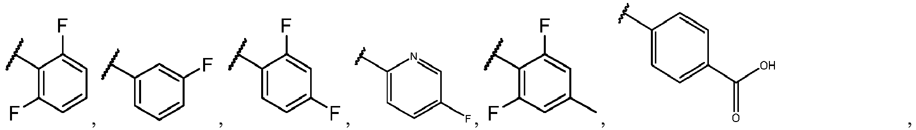

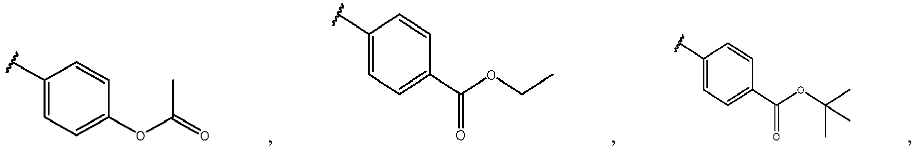

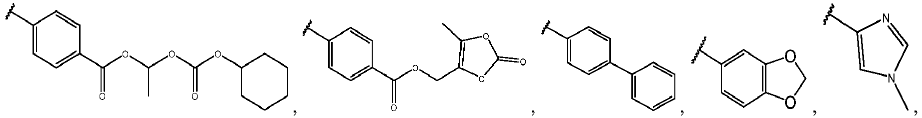

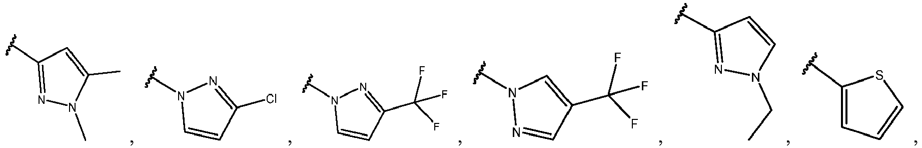

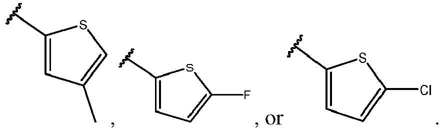

In one embodiment, R^{b} is ethyl, -CH₂OH, trimethylsilyl, cyclopropyl, cyclopentyl, cyclohexyl, phenyl,

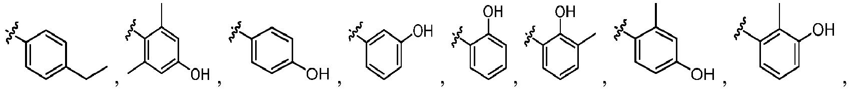

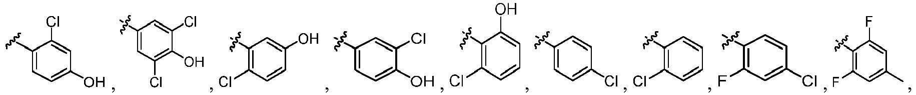

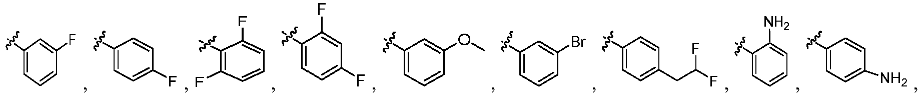

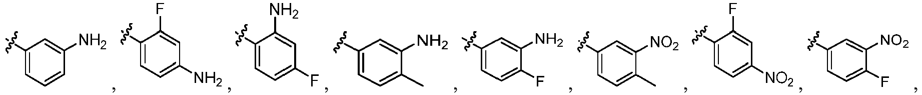

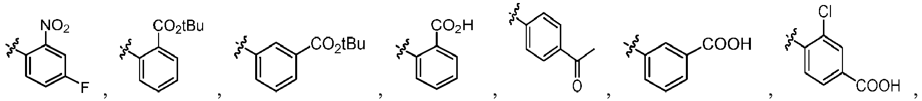

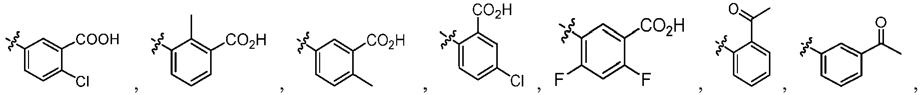

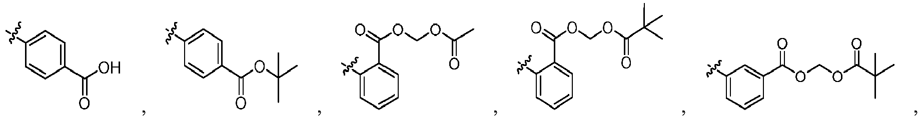

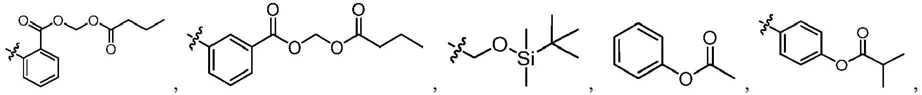

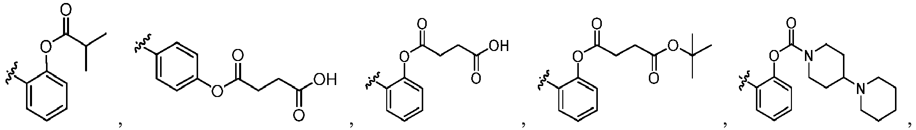

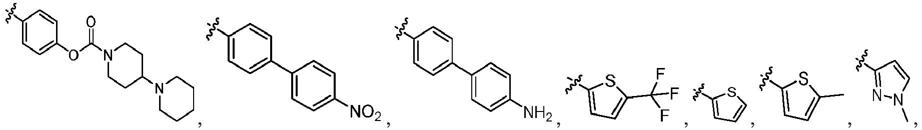

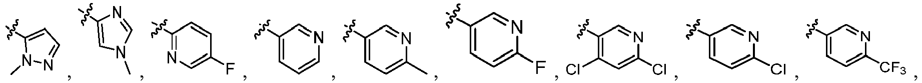

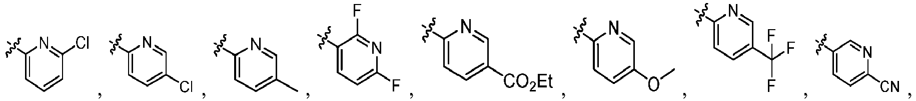

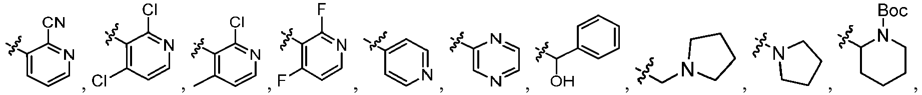

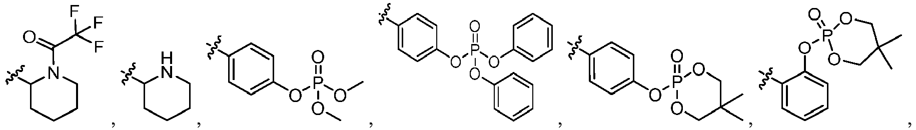

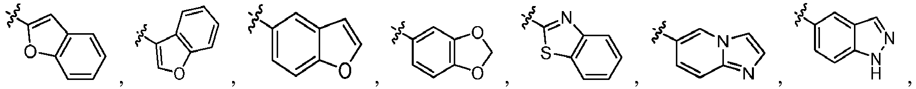

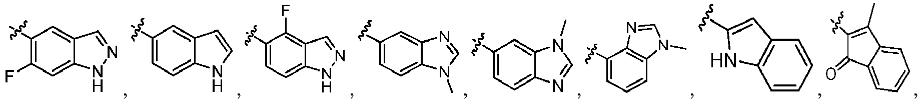

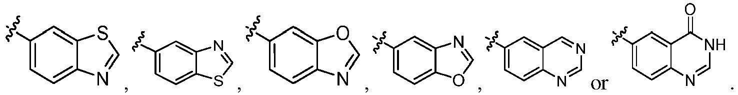

The compound is defined as in formula (III), or a pharmaceutically acceptable salt, tautomer, mesomer, racemate, stereoisomer, metabolite, metabolic precursor, or prodrug thereof.

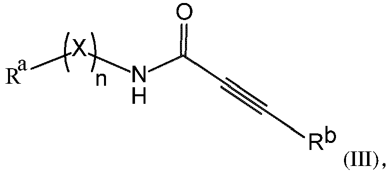

Wherein
n is an integer from 0 to 2.

In one embodiment, n is 0 or 1.

In one embodiment, n is 1.

The compound is defined as in formula (IIIa), or a pharmaceutically acceptable salt, tautomer, mesomer, racemate, stereoisomer, metabolite, metabolic precursor, or prodrug thereof.

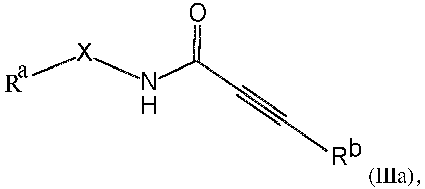

Wherein, R^{a} and R^{b} are as defined in formula (I), and X is as defined in formula (I).

Any one of the aforementioned compounds are defined as in formula (Ia), or a pharmaceutically acceptable salt, tautomer, mesomer, racemate, stereoisomer, metabolite, metabolic precursor, or prodrug thereof.

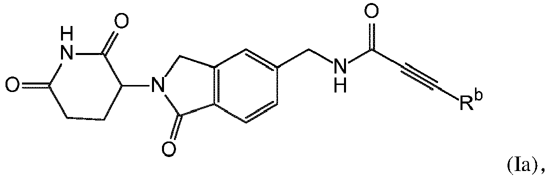

Wherein, R^{b} is as defined in formula (I);
In one embodiment of a compound of Formula (Ia), R^{b} is phenyl optionally substituted with one or more R, wherein R is selected from methyl, ethyl, carboxyl, hydroxyl, halogen, cyano, amino, nitro, methoxycarbonyl, methoxycarbonyl, ethoxycarbonyl, tert-butoxycarbonyl, carboxy, phenyl, fluorine, chlorine, bromine, trifluoromethyl, nitrophenyl, aminophenyl, -CH₂CF₂, -C(O)CH₃,-C(O)CF₃

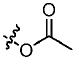

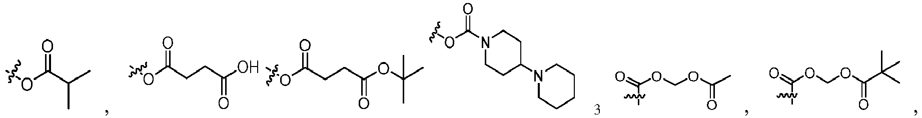

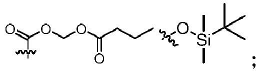

In one embodiment of a compound of the formula (Ia), wherein R is selected from methyl, ethyl, fluorine, chlorine, bromine, nitro, amino, ethoxycarbonyl, trifluoromethyl, methoxy, tert-butoxycarbonyl, nitrophenyl, aminophenyl, -CH₂CF₂, or -C(O)CH₃.

The present invention also provides the following compounds, or a pharmaceutically acceptable salt, tautomer, mesomer, racemate, stereoisomer, metabolite, metabolic precursor, or prodrug thereof,

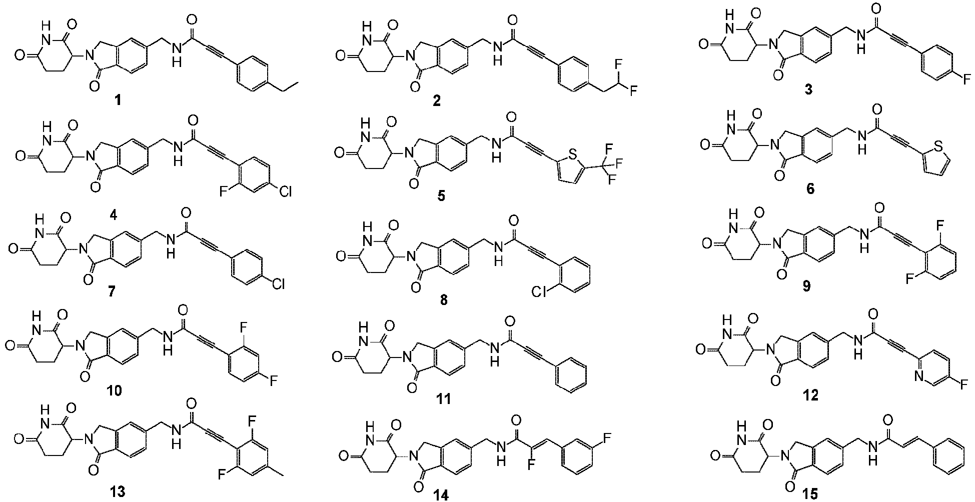

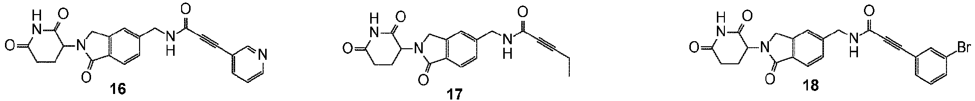

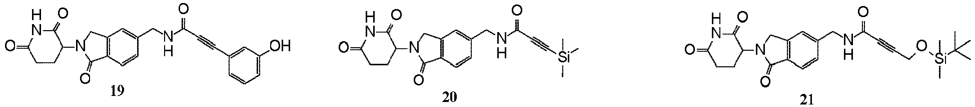

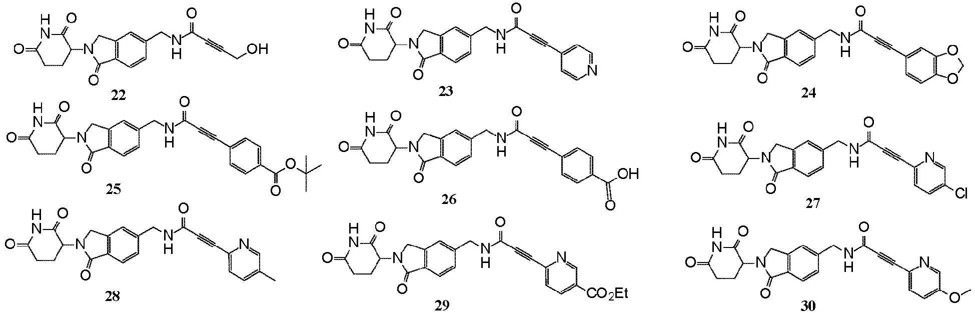

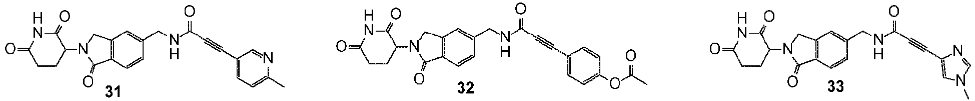

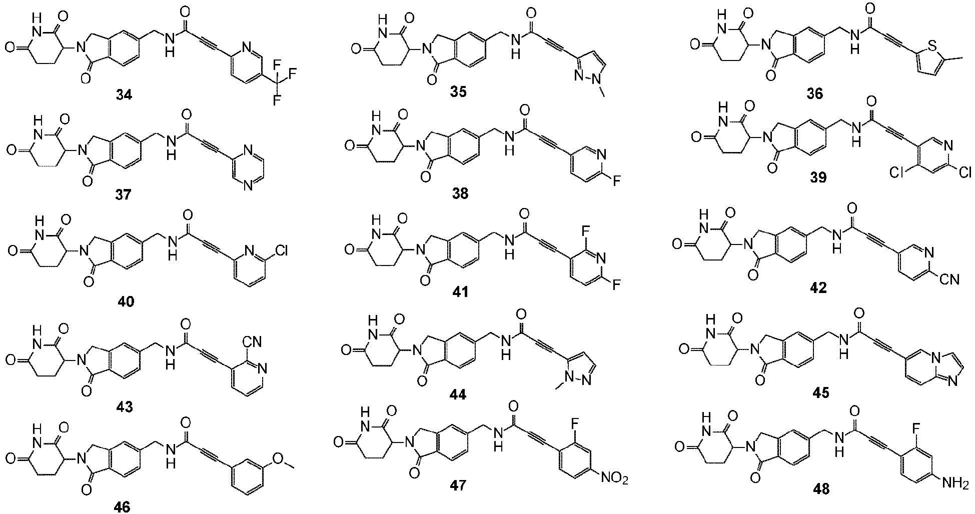

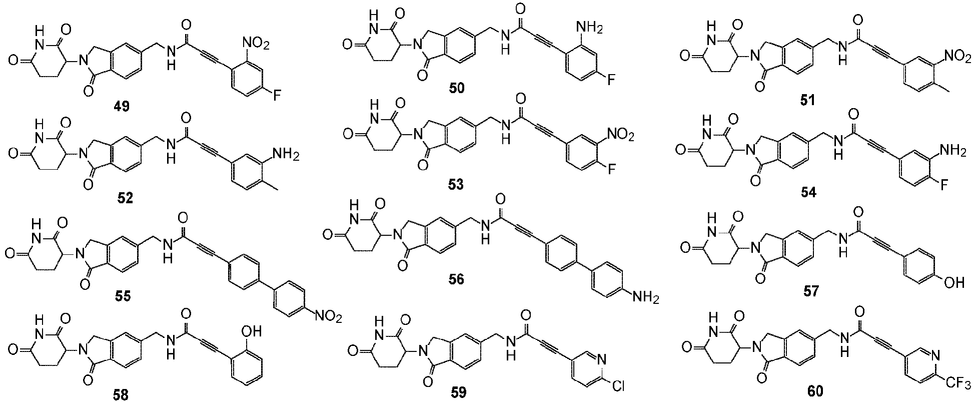

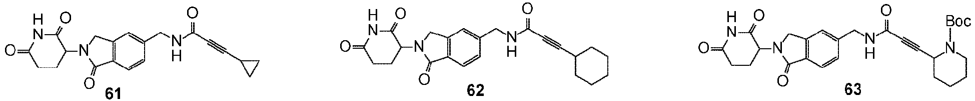

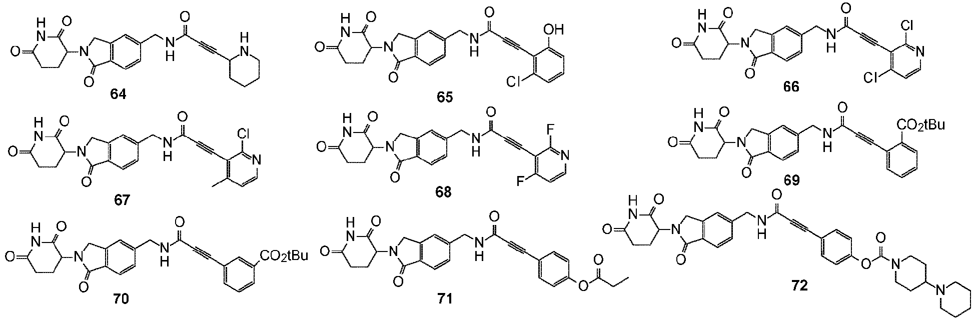

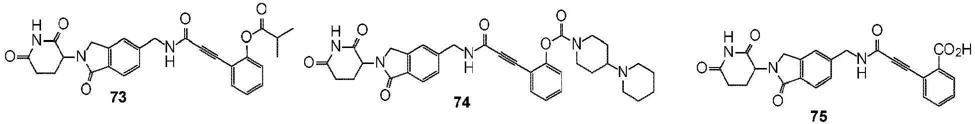

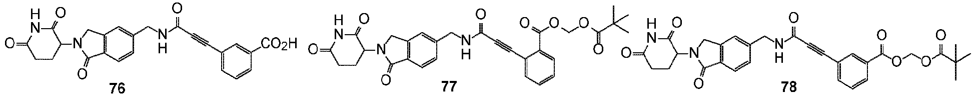

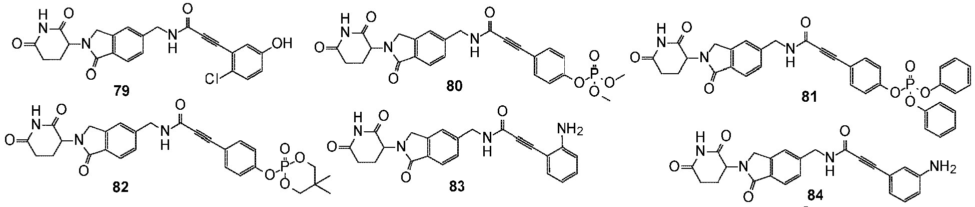

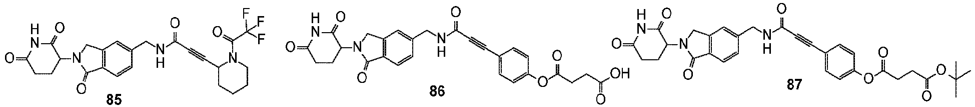

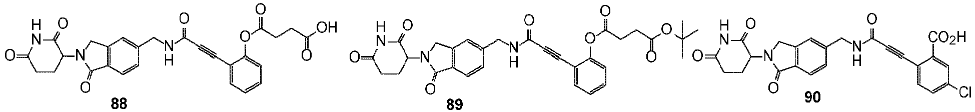

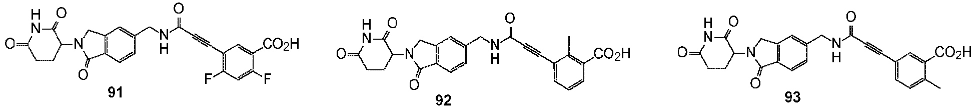

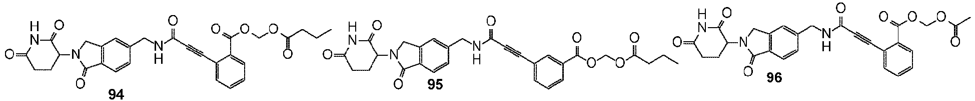

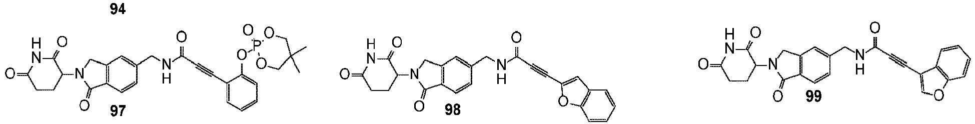

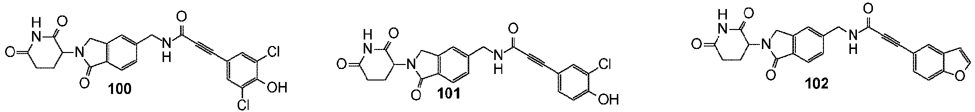

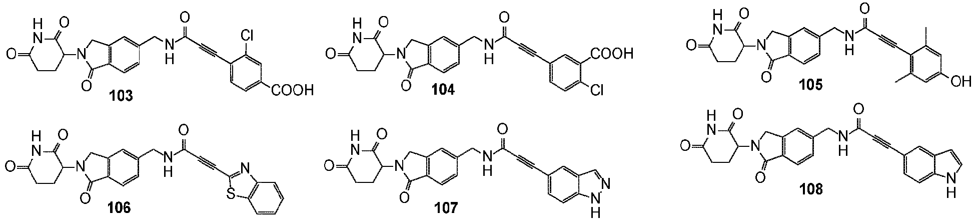

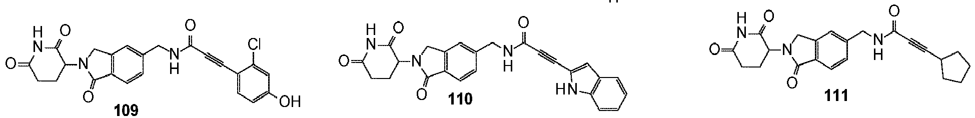

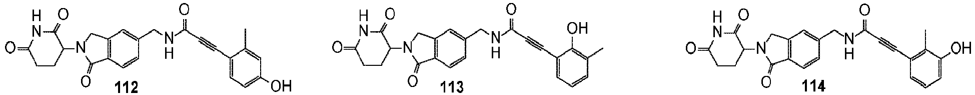

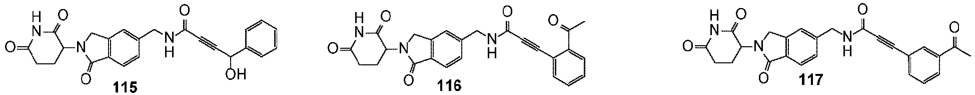

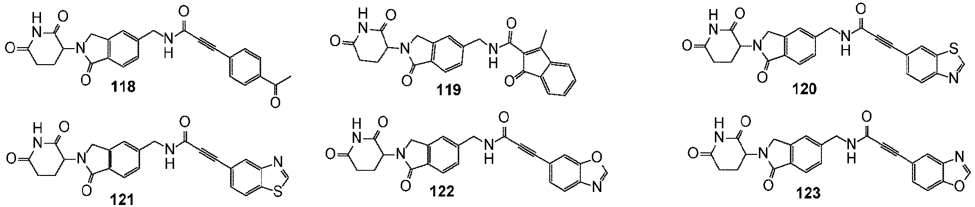

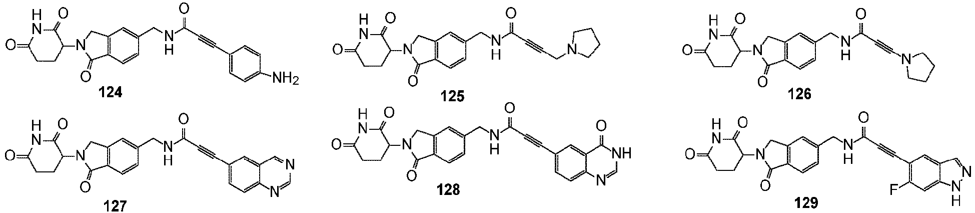

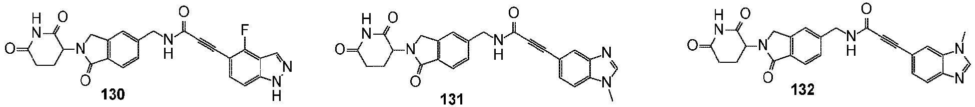

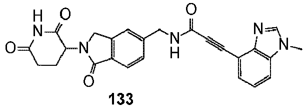

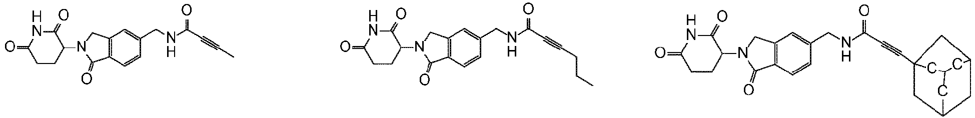

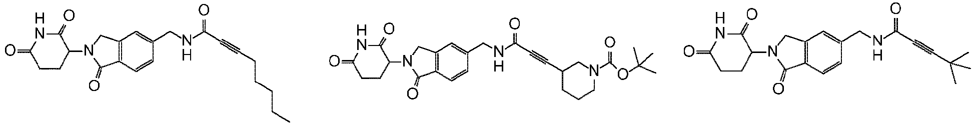

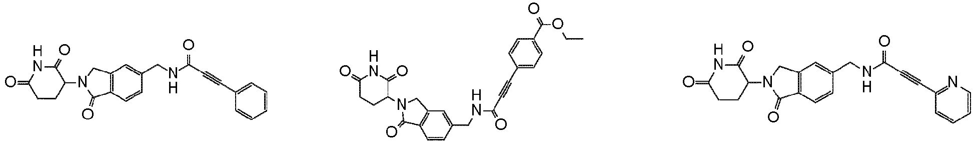

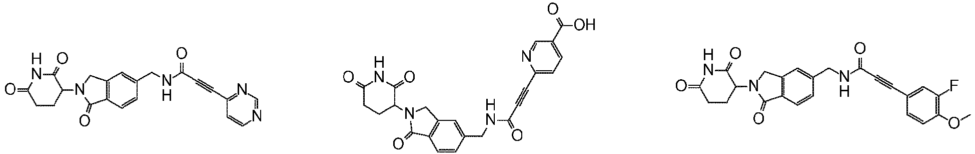

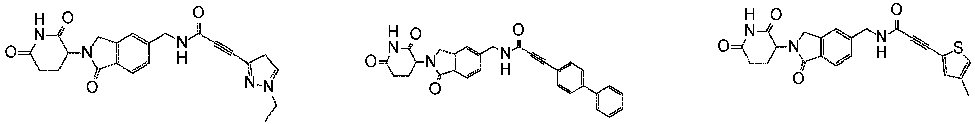

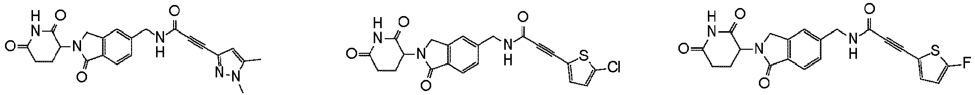

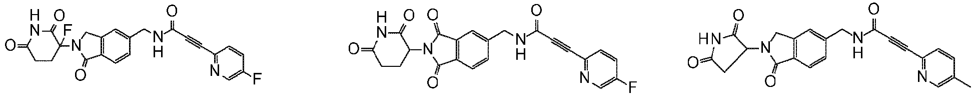

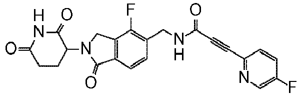

The method for preparing any one of the compounds of the aforementioned compounds characterized by comprising the steps:

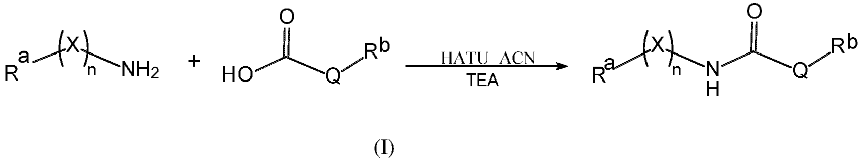

Amine compound, carboxylic acid compound, HATU, and TEA are dissolved in acetonitrile, stirred and reacted at room temperature, the reaction solution is concentrated under reduced pressure, extracted, combined with organic phases, concentrated, and purified to obtain the compound of formula (I).

The present invention also provides a pharmaceutical composition comprising the compound according to the aforementioned compounds, or a pharmaceutically acceptable salt, tautomer, mesomer, racemate, stereoisomer, metabolite, metabolic precursors or prodrug thereof, and pharmaceutically acceptable excipients.

The present invention also provides the use of any of the aforementioned compounds, or a pharmaceutically acceptable salt, tautomer, mesomer, racemate, stereoisomer, metabolite, metabolic precursor, prodrug thereof, or the pharmaceutical composition comprising the above compounds, in the preparation of drugs for the treatment of GSPT1-mediated conditions or disorders.

In one embodiment, GSPT1-mediated disease or disorder include, but are not limited to, cancer, viral infection, aging, immune diseases, neurological diseases. Among them, the cancers are selected from acute myeloid leukemia, liver cancer, acute lymphoblastic leukemia, bladder cancer, bone cancer, brain cancer, breast cancer, cervical cancer, chorionic cancer, chronic lymphocytic leukemia (CLL), chronic myeloid leukemia (CML), colon cancer, endometrial cancer, esophageal cancer, gallbladder cancer, gastric cancer, gastrointestinal stromal tumor, head and neck cancer, Hodgkin lymphoma, laryngeal cancer, leukemia, lung cancer, melanoma, mesothelioma, multiple myeloma, ovarian cancer, pancreatic cancer, prostate cancer, rectal cancer, kidney cancer, Sarcoma, skin cancer, small cell lung cancer, testicular cancer, throat cancer, thyroid cancer, uterine cancer.

The present invention also provides the use of any of the aforementioned compounds, or a pharmaceutically acceptable salt, enantiomer, stereoisomer, solvate and polymorphs or N-oxides, or the aforementioned pharmaceutical composition in the preparation of GSPT1 degrader.

The present invention also provides the use of any of the aforementioned compounds, or a pharmaceutically acceptable salt, tautomer, mesomer, racemate, stereoisomer, metabolite, metabolic precursor, or prodrug thereof, or the pharmaceutical composition comprising the above compounds for the manufacture of GSPT1 degrader.

The present invention also provides a method for degrading the GSPT1 protein in patient, which comprises administering to the patient any of the aforementioned compounds, or a pharmaceutically acceptable salt, enantiomer, stereoisomer, solvate or polymorph or N-oxide, or a pharmaceutical composition comprising the aforementioned compound.

The present invention also provides a method for degrading the GSPT1 protein in patient, which comprises the administration of any of the foregoing compounds, their pharmaceutically acceptable salts, tautomers, mesomers, racemates, stereoisomers, metabolites, metabolic precursors, prodrugs, or aforementioned drug compositions to the patients.

The present invention also provides a method for the treatment of a condition or disorder caused by the accumulation of GSPT1 protein in patient in need, consisting of administering to the patient any of the aforementioned compounds, or a pharmaceutically acceptable salt, enantiomer, stereoisomer, solvate or polymorph, or N-oxide, or a combination of the aforementioned compounds.

The present invention also provides a method for the treatment of a condition or disorder caused by the accumulation of GSPT1 protein in a patient in need, which consists of administering to the patient any of the aforementioned compounds, or a pharmaceutically acceptable salt, tautomers, mesomer, racemate, stereoisomer, metabolite, metabolic precursor, prodrug, or a combination of the aforementioned compounds.

In one embodiment, GSPT1-mediated illnesses or disorders include, but are not limited to, cancer, viral infection, aging, immune diseases, neurological diseases. Among them, the cancers are selected from acute myeloid leukemia, liver cancer, acute lymphoblastic leukemia, bladder cancer, bone cancer, brain cancer, breast cancer, cervical cancer, chorionic cancer, chronic lymphocytic leukemia (CLL), chronic myeloid leukemia (CML), colon cancer, endometrial cancer, esophageal cancer, gallbladder cancer, gastric cancer, gastrointestinal stromal tumor, head and neck cancer, Hodgkin lymphoma, laryngeal cancer, leukemia, lung cancer, melanoma, multiple myeloma, ovarian cancer, pancreatic cancer, prostate cancer, rectal cancer, kidney cancer, sarcoma, Skin cancer, small cell lung cancer, testicular cancer, throat cancer, thyroid cancer, uterine cancer.

In one embodiment, the above lung cancer is non-small cell lung cancer.

In one embodiment, the above sarcomas are selected from Kaposi's sarcoma, soft tissue sarcoma, mesothelioma, osteosarcoma, non-Hodgkin lymphoma.

In one embodiment, the viral infection includes coronaviruses such as SARS-CoV-2, HCoV-229E, HCoV-OC43, HCoV-NL63, HCoV-HKU1, SARS-CoV, MERS-CoV, SADS-COV, PEDV, PDCoV and FIPV, and viral infections such as hepatitis B virus, HIV virus, Ebola virus, ASFV, etc.

In one embodiment, the above GSPT1-mediated condition or disorder is selected from acute myeloid leukemia or liver cancer or coronavirus infection.

The propiolamide or acrylamide compound in the present invention could be used to regulate the level of GSPT1 protein, and have obvious activity for inhibiting the proliferation of tumor cells, such as acute myeloid leukemia, liver cancer and other cells and can effectively alleviate or treat cancer and other related diseases.

### BRIEF DESCRIPTION OF DRAWINGS

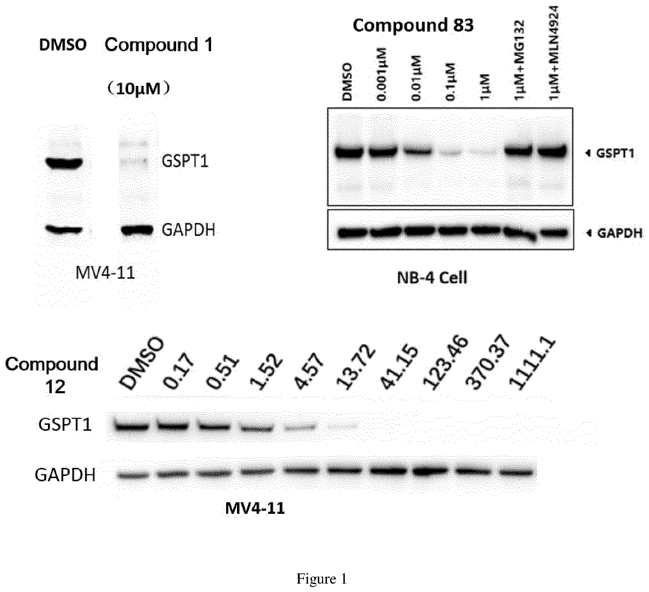

Figure 1 shows the western blot analysis results of embodiment 1.

### DETAILED DESCRIPTION OF THE INVENTION

In accordance with the above-mentioned contents of the present invention, and in accordance with the common technical knowledge and customary means in the art, and on the premise of not departing from the above-mentioned basic technical ideas of the present invention, other forms of modification, replacement or change may also be made.

### I. Definitions

Unless otherwise expressly stated, throughout the description and claims, the terms "including" or their transformations such as "consisting " or "comprising ", etc., are to be construed to include the stated element or component without excluding the other elements or other components.

The present invention compounds may be asymmetrical, e.g., with one or more stereoisomers. Unless otherwise stated, all stereoisomers are included, such as enantiomers and diastereomers. The compounds containing asymmetric carbon atoms disclosed in this invention can be isolated in optically active pure form or racemic form. The optically active pure form can be separated from racemic mixtures or synthesized by using chiral raw materials or chiral reagents. Racemates, diastereomers, and enantiomers are included in the scope of this invention.

Thepresent invention compounds also include a tautomeric form. The tautomeric form is derived from the exchange of a single bond with an adjacent double bond and the migration of a proton along with it.

The term "optional" or "optionally" refers to the subsequent description of the event or circumstance which may or may not occur, which includes both the occurrence of said event or circumstance and the non-occurrence of said event or circumstance.

The numerical range in the present invention refers to the integers in a given range. For example, "C1-C6" means that the group may have 1 carbon atom, 2 carbon atoms, 3 carbon atoms, 4 carbon atoms, 5 carbon atoms, or 6 carbon atoms, and "C3-C6" means that the group may have 3 carbon atoms, 4 carbon atoms, 5 carbon atoms, or 6 carbon atoms.

The term "substituted" refers to the substitution of any one or more hydrogen atoms on a particular atom or group as long as the valence state of the particular atom or group is normal and the substituted compound is stable. When the substituent is a ketone group (i.e. =O), it means that two hydrogen atoms are substituted. Unless otherwise specified, the type and number of substituents may be arbitrary on a chemically achievable basis.

When any variable, such as Rₙ or Rⁿ, appears more than once in the composition or structure of a compound, its definition in each case is independent. So, for example, if a group is replaced by 1-5 R's, the group can be optionally replaced by up to 5 R's, and there are independent options for R in each case. In addition, combinations of substituents and/or their variants are only allowed if such a combination results in a stable compound.

The term "alkyl" refers to saturated aliphatic hydrocarbon groups, including linear or branched saturated hydrocarbon groups with the indicated number of carbon atoms. For example, the term "C₁-C₆ alkyl" includes C₁ alkyl, C₂ alkyl, C₃ alkyl, C₄ alkyl, C₅ alkyl, C₆ alkyl, examples include, but are not limited to, methyl, ethyl, n-propyl, isopropyl, n-butyl, isobutyl, tert-butyl, n-amyl, 2-pentyl, 3-amyl, n-hexyl, 2-hexyl, 3-hexyl, etc. It can be divalent, e.g., methylene, ethylene.

As used in present invention, the term "C₁-C₆ linear or branched divalent hydrocarbon chains" refers to the divalent alkyre, alkenyl and alkyne chains that are linear or branched as defined in present invention.

The term "halogenated" refers to being replaced by one or more halogen atoms, examples of halogen atoms include fluorine atoms, chlorine atoms, bromine atoms, iodine atoms.

The term " cycloalkyl"" refers to a monocyclic saturated hydrocarbon system with no heteroatoms and no double bonds. Examples of the term "C₃-C₆ cycloalkyl" include, but are not limited to, cyclopropyl, cyclobutyl, cycloamyl, cyclohexyl.

The term "aryl" refers to an aromatic ring group of whole carbon single ring or fused polycyclic aromatic ring group with a conjugated π-electron system, which is obtained by removing a hydrogen atom from a single carbon atom of the parent aromatic ring system. For example, aryl groups can have 6-20 carbon atoms, 6-14 carbon atoms, or 6-10 carbon atoms, including bicyclic groups containing aromatic rings fused with saturated, partially unsaturated rings, or aromatic carbon rings. Examples include, but are not limited to, phenyl, naphthalene, anthracene, indene, indane, 1,2-dihydronaphthalene, 1,2,3,4-tetrahydronaphthalene.

The term "heteroaryl" refers to a monovalent aryl group containing at least one 5-, 6-, 7-member ring independently with heteroatoms selected from nitrogen, oxygen, and sulfur, and a fused ring system consisting of 5-10 atoms (at least one of which is aromatic). Examples of aryl heterogroups include, but are not limited to, Pyridyl, thiophenyl, imidazolyl, pyridyl, pyridyl, furanyl, pyrazinyl, thiazolyl, quinolinyl, isoquinolinyl, indolyl, benzimidazolyl, imidazolpyridyl, benzofuranyl, pyridyl, isoindolyl.

The term "membered" refers to the number of skeleton atoms that make up the ring. For example, "5- to 10- membered" means that the number of skeleton atoms that make up the ring is 5, 6, 7, 8, 9, or 10. So, for example, pyridine, piperidine, piperazine, and benzene are six-membered rings, while thiophene and pyrrole are five-membered rings.

The term "heterocycle" refers to a 5- to 12- membered saturated non-aromatic system comprising of carbon atoms and 1 to 2 heteroatoms, where the heteroatoms are independently selected from nitrogen, sulfur, or oxygen atoms. In heterocyclic groups containing one or more nitrogen atoms, the junction point can be carbon or nitrogen atoms, as long as the valence allows. Heterocycles can be single- or multi-rings, such as dicycles, in which two or more rings exist in the form of spiral ring, bridge ring, and fused ring, where at least one ring contains one or more heteroatoms.

As used in present invention, the term "partially unsaturated" means a ring that includes at least one double or triple bond. The term "partially unsaturated" is intended to cover rings with multiple unsaturated sites, but is not intended to include aryl or heteroaryl fractions as defined in present invention.

The substituent Rₙ can bond to any atom on the ring as long as the atomic valence allows. Combinations of substituents and/or their variants are only permissible if such a combination results in a stable compound. It can be understood that those skilled in the art can understand that for any group containing one or more Rₙ substituents, substitution or substitution modes that are spatially impossible to exist and/or synthesize will not be introduced.

The terms "deuterium", "D" denote a single deuterium atom. It should be understood that the compounds disclosed in present invention may be isotopically labeled. The use of isotope substitutions such as deuterium may be preferred in some cases because of certain therapeutic advantages resulting in greater metabolic stability, such as increasing in vivo half-life or decreasing dose requirements. In the structure of the compound disclosed in present invention, the hydrogen atom may be unambiguously disclosed or understood to exist in the compound. Hydrogen atoms can be any isotope of hydrogen anywhere in a compound where hydrogen atoms can be present, including but not limited to: ¹H (deuterium) and ²H (deuterium). Deuterium substitution can be partial or complete, with partial deuterium substitution being substituted with at least one hydrogen or at least one deuterium.

The compounds described in present invention may be deuterium-substituted compounds. The terms "deuterium substitution", "deuteration generation" refer to the substitution of one or more C-H bonds by C-D bonds in a compound or group, and deuterium substitution can be one-substituted, di-substituted, multi-substituted, or fully substituted, as in the term "one or more hydrogen in methylene is replaced by deuterium" refers to the substitution of one or more C-H bonds in -CH₂- by C-D bonds, which include, but are not limited to, -CDH-,-CD2-. The "deuterated" method adopts a conventional method in the art, such as the introduction of deuterium into a compound in accordance with the method disclosed in the prior art. 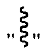 refers to the junction of chemical bonds.

### Medicament or pharmaceutical composition

The term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication commensurate with a reasonable benefit/risk ratio.

The term "pharmaceutically acceptable salt" refers to a salt that retains the biological efficacy of the free acid and base of a specific compound without biological adverse effects. For example, acid (including organic acid and inorganic acid) addition salt or base (including organic base and inorganic base) addition salt.

The pharmaceutically acceptable salts disclosed in the present may be synthesized by conventional chemical methods from parent compounds containing acid groups or bases. In general, such salts are prepared by reacting these compounds in the form of free acids or bases with appropriate bases or acids in stoichioimetric form in water or organic solvents, or a mixture of both.

The medicament or pharmaceutical composition disclosed in present invention may be administered orally, locally, parenterally, or mucosally (e.g., by mouth, by inhalation, or rectum) in dose units containing conventional non-toxic medicament-acceptable vehicles. The oral route is usually preferred. The active reagent may be administered orally in the form of capsules, tablets, etc. (see Remington: The Science and Practice of Pharmacy, 20th Edition).

For oral administration in tablet or capsule form, the active medicament component may be combined with non-toxic, pharmaceutically acceptable excipients such as binders (e.g., pregelatinized corn starch, polyvinylpyrrolidone, or hydroxypropyl methylcellulose), fillers (e.g., lactose, sucrose, glucose, mannitol, sorbitol, and other reducing and non-reducing sugars, microcrystalline cellulose, calcium sulfate, or dicalcium phosphate), lubricants (e.g., magnesium stearate, talc or silica, stearic acid, sodium stearate fumarate, glyceryl dodecanoate, calcium stearate, etc.), disintegrants (e.g., potato starch or sodium starch glycolate), or wetting agents (e.g., sodium lauryl sulfate), colorants and flavorings, gelatin, sweeteners, natural and synthetic gums (e.g., gum arabic, tragacanth gum, or alginate), buffered salts, carboxymethylcellulose, polyethylene glycol, wax, etc. For oral administration in liquid form, the drug components may be combined with a non-toxic, pharmaceutically acceptable inert carrier (e.g., ethanol, glycerol, water), anti-settling agents (e.g., sorbitol syrup, cellulose derivatives, or hydrogenated edible fats), emulsifiers (e.g., lecithin or gum arabic), non-aqueous carriers (e.g., almond oil, oleoesters, ethanol, or fractionated vegetable oils), preservatives (e.g., methyl p-hydroxybenzoate or propyl p-hydroxybenzoate, or sorbic acid). Stabilizers such as antioxidants (BHA, BHT, Propyl hesperate, sodium ascorbate, citric acid) may also be added to stabilize the dosage form.

Tablets containing active compounds can be coated by methods that are well known in the art. The composition of the present invention containing the formula I compound that is the active compound may also introduce beads, microspheres, or microcapsules, such as those constructed from polyglycolic acid/lactic acid (PGLA). Formulations of liquids for oral administration may take the form of, for example, solutions, syrups, emulsions or suspensions, or they may be presented as dry products reconstituted with water or other suitable excipients prior to use. Formulations for oral administration may be formulated appropriately to allow for controlled or delayed release of the active compound.

The medicament or pharmaceutical composition disclosed in present invention may be administered parenterally, i.e., by intravenous (i.v.), intraventricular (i.c.v.), subcutaneous (s.c.), intraperitoneal (i.p.), intramuscular (i.m.), subcutaneous (s.d.) or intradermal (i.d.), by direct injection, by, for example, rapid concentration or continuous infusion. Formulations for injection can be presented in unit dosage forms, e.g. in ampoules or multi-dose containers with added preservatives. The composition may take the form of an excipient, a suspension, a solution or an emulsion in an oil or aqueous carrier, and may contain a formulation reagent such as an anti-settling agent, a stabilizer and/or a dispersant. Alternatively, the active ingredient can be reconstituted in powder form with a suitable carrier (e.g., sterile, pyrogen-free water) prior to use.

The medicament or pharmaceutical composition disclosed herein may also be formulated for rectal administration, such as suppositories or retaining enemas (e.g., containing conventional suppository bases such as cocoa butter or other glycerides).

The term "treatment" includes suppressing, alleviating, preventing, or eliminating one or more of the symptoms or side effects associated with the disease, condition, or disorder being treated.

The use of the terms "reduce", "inhibit", "alleviate" or "decrease" is relative to control. A person skilled in the art will easily determine the appropriate controls to use for each experiment. For example, a reduced response in subjects or cells treated with a compound is compared to a response in subjects or cells not treated with a compound.

As used in present invention, the term "effective amount " or "therapeutically effective amount" means a dosage sufficient to treat, inhibit, or alleviate one or more symptoms of an inflammatory response or autoimmune disease state being treated or to otherwise provide a desired pharmacologic and/or physiologic effect. The precise dosage will vary according to a variety of factors such as subject-dependent variables (e.g., age, immune system health, etc.), the disease or disease, and the treatment being administered. The effect of an effective amount can be relative to a control. These controls are known in the art and discussed herein, and may be, for example, the condition of the subject prior to or without administration of the drug or drug combination, or in the case of drug combinations, the combined effects may be compared to the effect of administering only one drug.

The term "excipient" is used herein to include any other compound that may be contained in or on the microparticles that is not a therapeutic or biologically active compound. Therefore, the excipient should be pharmaceutically or biologically acceptable or relevant, eg, the excipient is generally non-toxic to the subject. "Excipient" includes a single such compound and is also intended to include a plurality of compounds.

The term "pharmaceutical composition" means a composition comprising a compound of the present invention or a pharmaceutically acceptable salt thereof, and at least one pharmaceutically acceptable ingredient selected from the following, depending on the mode of administration and the nature of the dosage form, Including but not limited to: carriers, diluents, adjuvants, excipients, preservatives, fillers, disintegrants, wetting agents, emulsifiers, suspending agents, sweeteners, flavoring agents, flavors, antibacterial agents , antifungal agents, lubricants, dispersants, temperature-sensitive materials, temperature regulators, adhesives, stabilizers, suspending agents, etc.

### Uses and treatments

The terms "patient," "subject," "individual" and the like are used interchangeably herein and refer to any animal or cells thereof that is subject to the methods described herein, whether in vitro or in situ. In some non-limiting embodiments, the patient, subject or individual is a human.

According to the methods of the present invention, a compound or pharmaceutical composition may be administered in any amount and by any route of administration effective to treat or reduce the severity of a disease associated with GSPT1 protein.

The present invention relates to a method of reducing GSPT1 in a biological sample, comprising the step of contacting said biological sample with a compound of the present invention or a composition comprising said compound.

The term "biological sample" includes, but is not limited to, cell cultures, or extracts thereof; biopsy material obtained from mammals, or extracts thereof; and blood, saliva, urine, feces, semen, tears, or other body fluids, or extracts thereof things. Inhibition of enzymes in biological samples can be used to achieve a variety of purposes known to those skilled in the art. Examples of such purposes include, but are not limited to, biological analysis, gene expression studies, and biological target identification.

The method of the present invention for inhibiting GSPT1 protein in a patient, comprises the step of administering to said patient a compound of the present invention or a composition comprising said compound.

The compounds provided are GSPT1 protein modulators, so it may be used to treat one or more conditions associated with GSPT1 activity. Thus, in certain embodiments, the present invention provides a method for treating a GSPT1-mediated diseases, comprising administering to a patient a compound or pharmaceutically acceptable composition of the present invention.

As used in present invention, the term "GSPT1-mediated" disorder, disease and/or condition as used herein means any disease or other deleterious condition in which GSPT1 or it's mutants thereof are known to play a role.

GSPT1-mediated disorders are well established in the art. The relationship between GSPT1 and GSPT1-mediated disorders, diseases and/or conditions as described herein is well established in the relevant art.

In some embodiments, the present invention provides a method for treating one or more disorders, diseases, and/or conditions wherein the disorder, disease, or condition is a proliferative disease such as cancer, an inflammatory disorder, or a viral infection.

In certain embodiments, the present invention provides a method of treating cancer or another proliferative disorder, comprising administering a compound or composition of the present invention to a patient with cancer or another proliferative disorder. In certain embodiments, the method of treating cancer or another proliferative disorder comprises administering compounds and compositions of the present invention to a mammal. In certain embodiments, the mammal is a human.

As used herein, the terms "inhibition of cancer" and "inhibition of cancer cell proliferation" refer to the inhibition of the growth, division, maturation or viability of cancer cells, and/or causing the death of cancer cells, individually or in aggregate with other cancer cells, by cytotoxicity, nutrient depletion, or the induction of apoptosis.

Examples of tissues containing cancerous cells whose proliferation is inhibited by the compounds and compositions described herein and against which the methods described herein are useful include but are not limited to breast, prostate, brain, blood, bone marrow, liver, pancreas, skin, kidney, colon, ovary, lung, testicle, penis, thyroid, parathyroid, pituitary, thymus, retina, uvea, conjunctiva, spleen, head, neck, trachea, gall bladder, rectum, salivary gland, adrenal gland, throat, esophagus, lymph nodes, sweat glands, sebaceous glands, muscle, heart, and stomach.

In some embodiments, the cancer treated by compounds or compositions of the present invention is a melanoma, liposarcoma, lung cancer, breast cancer, prostate cancer, leukemia, kidney cancer, esophageal cancer, brain cancer, lymphoma or colon cancer. In certain embodiments, the cancer is a primary effusion lymphoma (PEL).

Compounds of the present invention are useful in the treatment of a proliferative disease selected from a benign or malignant tumor, carcinoma of the brain, kidney, liver, adrenal gland, bladder, breast, stomach, gastric tumors, ovaries, colon, rectum, prostate, pancreas, lung, vagina, cervix, testis, genitourinary tract, esophagus, larynx, skin, bone or thyroid, sarcoma, glioblastomas, neuroblastomas, multiple myeloma or gastrointestinal cancer, especially colon carcinoma or colorectal adenoma or a tumor of the neck and head, an epidermal hyperproliferation, psoriasis, prostate hyperplasia, a neoplasia, a neoplasia of epithelial character, adenoma, adenocarcinoma, keratoacanthoma, epidermoid carcinoma, large cell carcinoma, non-small-cell lung carcinoma, lymphomas, Hodgkins and Non-Hodgkins, Waldenström's macroglobulinemia, a mammary carcinoma, follicular carcinoma, undifferentiated carcinoma, papillary carcinoma, seminoma, melanoma, an MYD88-driven disorder, DLBCL, ABC DLBCL, an IL-1-driven disorder, Smoldering of indolent multiple myeloma, or a leukemia.

Cancer includes, in some embodiments, without limitation, leukemias (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia), polycythemia vera, lymphoma (e.g., Hodgkin's disease or non-Hodgkin's disease), Waldenstrom's macroglobulinemia, multiple myeloma, heavy chain disease, and solid tumors such as sarcomas and carcinomas (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, glioblastoma multiforme (GBM, also known as glioblastoma), medulloblastoma, craniopharyngioma, ependymoma, pinealoma, hemangioblastoma, acoustic neuroma, oligodendroglioma, schwannoma, neurofibrosarcoma, meningioma, melanoma, neuroblastoma, and retinoblastoma).

In some embodiments, the cancer is glioma, astrocytoma, glioblastoma multiforme (GBM, also known as glioblastoma), medulloblastoma, craniopharyngioma, ependymoma, pinealoma, hemangioblastoma, acoustic neuroma, oligodendroglioma, schwannoma, neurofibrosarcoma, meningioma, melanoma, neuroblastoma, or retinoblastoma.

In some embodiments, the cancer is acoustic neuroma, astrocytoma (e.g. Grade I-Pilocytic Astrocytoma, Grade II-Low-grade Astrocytoma, Grade III-Anaplastic Astrocytoma, or Grade IV-Glioblastoma (GBM)), chordoma, CNS lymphoma, craniopharyngioma, brain stem glioma, ependymoma, mixed glioma, optic nerve glioma, subependymoma, medulloblastoma, meningioma, metastatic brain tumor, oligodendroglioma, pituitary tumors, primitive neuroectodermal (PNET) tumor, or schwannoma. In some embodiments, the cancer is a type found more commonly in children than adults, such as brain stem glioma, craniopharyngioma, ependymoma, juvenile pilocytic astrocytoma (JPA), medulloblastoma, optic nerve glioma, pineal tumor, primitive neuroectodermal tumors (PNET), or rhabdoid tumor. In some embodiments, the patient is an adult human. In some embodiments, the patient is a child or pediatric patient.

Cancer includes, in another embodiment, without limitation, mesothelioma, hepatobilliary (hepatic and billiary duct), bone cancer, pancreatic cancer, skin cancer, cancer of the head or neck, cutaneous or intraocular melanoma, ovarian cancer, colon cancer, rectal cancer, cancer of the anal region, stomach cancer, gastrointestinal (gastric, colorectal, and duodenal), uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, testicular cancer, chronic or acute leukemia, chronic myeloid leukemia, lymphocytic lymphomas, cancer of the bladder, cancer of the kidney or ureter, renal cell carcinoma, carcinoma of the renal pelvis, non-Hodgkins's lymphoma, spinal axis tumors, brain stem glioma, pituitary adenoma, adrenocortical cancer, gall bladder cancer, multiple myeloma, cholangiocarcinoma, fibrosarcoma, neuroblastoma, retinoblastoma, or a combination of one or more of the foregoing cancers.

In some embodiments, the cancer is selected from hepatocellular carcinoma, ovarian cancer, ovarian epithelial cancer, or fallopian tube cancer; papillary serous cystadenocarcinoma or uterine papillary serous carcinoma (UPSC); prostate cancer; testicular cancer; gallbladder cancer; hepatocholangiocarcinoma; soft tissue and bone synovial sarcoma; rhabdomyosarcoma; osteosarcoma; chondrosarcoma; Ewing sarcoma; anaplastic thyroid cancer; adrenocortical adenoma; pancreatic cancer; pancreatic ductal carcinoma or pancreatic adenocarcinoma; gastrointestinal/stomach (GIST) cancer; lymphoma; squamous cell carcinoma of the head and neck (SCCHN); salivary gland cancer; glioma, or brain cancer; neurofibromatosis-1 associated malignant peripheral nerve sheath tumors (MPNST); Waldenstrom's macroglobulinemia; or medulloblastoma.

The term "primary tumor" is relative to secondary tumors. Primary tumors refer to tumors that first appear in a certain part such as the lung, liver, intestine, head, or skin, etc., which can be called Primary lung cancer, primary liver cancer, primary bowel cancer, etc.

### Combined Therapy

Also provided herein are combined therapies using any of the compositions described herein and other therapeutic agents. The term combination therapy, as used herein, embraces administration of these agents in a sequential manner, that is, wherein each therapeutic agent is administered at a different time, as well as administration of these therapeutic agents, or at least two of the agents, in a substantially simultaneous manner. Sequential or substantially simultaneous administration of each agent can be affected by any appropriate route including, but not limited to, oral routes, intravenous routes, intramuscular, subcutaneous routes, direct absorption through mucous membrane tissues, and pulmonary delivery routes. The agents can be administered by the same route or by different routes. For example, a first agent can be administered by pulmonary delivery routes, and a second agent can be administered intravenously. Additionally, selected combination agents may be administered by intravenous injection, while the other agents of the combination may be administered orally. Alternatively, for example, two or more agents may be administered by intravenous or subcutaneous injection.

### Abbreviation:

Et: ethyl; tBu: tert-butyl; HATU: O-(7-Aza-1H-benzotriazol-1-yl)-N,N,N',N'-tetramethyluronium hexafluorophosphate; TEA: triethanolamine; CAN: acetonitrile.

### II. Embodiments

The first aspect, the present invention relates to compounds that are useful as protein modulators.

The second aspect, the present invention provides a chemical compound, its pharmaceutically acceptable salts, tautomers, mesomers, racemates, stereoisomers, metabolites, metabolic precursors or prodrugs, and intermediates, thereof, preparation methods, and pharmaceutical composition comprising the compound, which has obvious protein regulatory effects, the protein includes GSPT1, and can effectively alleviate or treat cancer and other related diseases..

The third aspect, the present invention provides a protein modulator to regulate expression levels of protein, including GSPT1.

The fourth aspect, the present invention also provides methods for modulating protein-mediated diseases, such as GSPT1-mediated diseases, disorders, conditions, or responses.

The fifth aspect, the present invention also provides methods for the treatment, improvement or prevention of protein-mediated diseases, conditions and conditions, such as GSPT1-mediated diseases, conditions and pathologies.

In one specific embodiment, the present invention provides a propiolamide or acrylamide compound, its intermediate, preparation method, pharmaceutical composition and application that are completely different from the prior art. The propiolamide or acrylamide compounds disclosed in present invention have obvious regulatory effects on GSPT1 and other proteins, and can effectively alleviate or treat cancer and other related diseases.

The present invention is further explained below with reference to the embodiments. The description of the specific exemplary embodiments of the present invention is for illustrative and illustrative purposes. These descriptions are not intended to limit the present invention to the precise form in which it is disclosed, and it is clear that many alterations and variations can be made according to the teachings of the present application specification. The purpose of selecting and describing the exemplary embodiments is to explain the specific principles of the present invention and its practical applications, so that those skilled in the art can realize and make use of the different exemplary embodiments of the present invention and the various choices and variations.

The experimental methods used in the following embodiments are conventional methods unless otherwise specified.

The materials, reagents, etc. used in the following embodiments, unless otherwise specified, can be obtained from commercial means.

In the following embodiment, room temperature refers to 10 °C-30 °C, reflux refers to solvent reflux temperature, overnight refers to 8-24 hours, preferably 12-18 hours.

The structure of the compounds was determined by nuclear magnetic resonance (NMR) or mass spectrometry (MS), which was obtained by a Bruker Avance-500 instrument, and mass spectrometry was obtained by liquid chromatography-mass spectrometry (LC-MS) coupled to an Agilent Technologies 6110 using an ESI ion source. Other reagents and intermediate compounds are purchased directly unless otherwise specified.

Preparation example: the general synthesis method of propiolamide compounds in the present disclose:

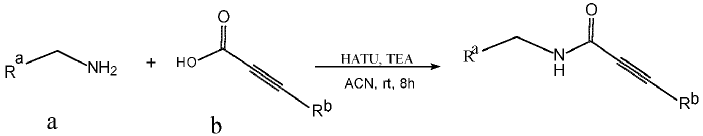

Ra and Rb are defined in the foregoing section of the present invention.

The mixed solution of amino compound (0.05mmol), propiolic acid compound (0.05 mmol), HATU (0.05mmol), TEA (0.2mmol), acetonitrile 10ml was stirred at room temperature for 8 hours, the reaction solution was concentrated under reduced pressure at 40 °C, ethyl acetate-water extraction was added, and the organic phase was concentrated, and the residue was purified by column chromatography (the solvent was dichloromethane : methanol = 20:1).or purified by reversed-phase high-performance liquid chromatography (eluent is acetonitrile-water, gradient 35-60%).

According to the method of the preparation example, by providing the corresponding precursor compound A and compound B, the embodiment compound 1-133 is synthesized for example, compound 12 is prepared by the following method:

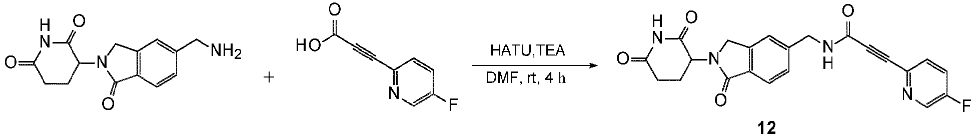

3-(5-fluoropyridine-2-yl) propiolic acid (64 mg, 0.39 mmol) was dissolved in 10 mL DMF, and 3-(6-aminomethylene-3-oxo-1H-isoindole-2-yl)piperidin-2,6-dione hydrochloride (120 mg, 0.39 mmol) and triethylamine (65 mg, 0.65 mmol) were slowly added to HATU (123 mg, 0.32 mmol) in batches. After about 4 h of reaction, LCMS showed that the raw material had disappeared. 20 mL of water and 10 mL of ethyl acetate were added to the reaction solution for extraction, and the extraction was repeated three times until the extraction was complete. The organic phase was combined and backwashed with saturated saline water, and then the organic phase was dried and concentrated with anhydrous Na₂SO₄ to obtain a crude brown concentrate. Column chromatography (MeOH/DCM = 1/100 ~ 1/30) was performed to obtain white solid compound 12 (68 mg, yield 50%).

### Embodiment 1: synthesis of compound 1

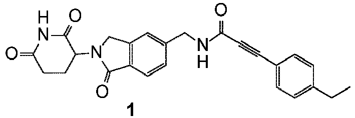

¹H NMR (400 MHz, DMSO-*d6*): 10.96 (s, 1H), 9.36 (t, *J* = 8 Hz, 1H), 7.69 (d, *J* = 8 Hz, 1H), 7.48-7.42 (m, 3H), 7.41 (d, *J* = 4 Hz, 1H), 7.29 (d, *J* = 8 Hz, 2H), 5.09 (dd, *J* = 8, 4Hz, 1H), 4.44-4.41 (m, 3H), 4.31 (d, *J* = 12 Hz, 1H), 2.89-2.86 (m, 1H), 2.62-2.56 (m, 3H), 2.39-2.37 (m, 1H), 1.99-1.92 (m, 1H), 1.16 (t, *J* = 8 Hz, 3H). Molecular Formula: C25H23N3O4, theoretical molecular weight: 429.48; LC-MS(ESI):m/z = [M+H]+: 430.48.

### Embodiment 2: compound 2 synthesis

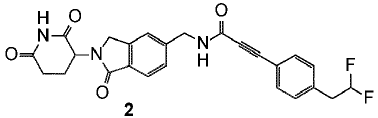

¹H NMR (400 MHz, DMSO-*d6*): 10.97 (s, 1H), 9.40 (t, J = 4 Hz, 1H), 7.69 (d, J = 4 Hz, 1H), 7.54 (d, J = 4 Hz, 2H), 7.50 (s, 1H), 7.41 (d, J = 4 Hz, 1H), 7.38 (d, J = 4 Hz, 2H), 6.26-6.23 (m, 1H), 5.09 (dd, J= 12, 4Hz, 1H), 4.44-4.41 (m, 3H), 4.31 (d, J = 12 Hz, 1H), 3.23-3.19 (m, 2H), 2.89-2.85 (m, 1H), 2.59 (d, J = 12 Hz, 1H), 2.39-2.34 (m, 1H), 2.00 (q, J = 8, 4 Hz, 1H). Molecular Formula: C25H21F2N3O4, theoretical molecular weight: 465.46; LC-MS(ESI):m/z = [M+H]+: 466.46.

### Embodiment 3: compound 3 synthesis

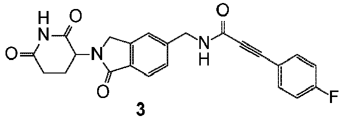

¹H NMR (400 MHz, DMSO-d6): 10.97 (s, 1H), 9.40 (t, J = 8 Hz, 1H), 7.69 (d, J = 8 Hz, 1H), 7.64 (d, J = 8 Hz, 2H), 7.49 (s, 1H), 7.41 (d, J = 8 Hz, 1H), 7.30 (t, J = 8 Hz, 2H), 5.09 (dd, J= 12, 4Hz, 1H), 4.44-4.41 (m, 3H), 4.31 (d, J = 12 Hz, 1H), 2.89-2.82 (m, 1H), 2.59 (d, J = 12 Hz, 1H), 2.39-2.36 (m, 1H), 1.99 (q, J = 8, 4 Hz, 1H). Molecular Formula: C23H18FN3O4, theoretical molecular weight: 419.41; LC-MS(ESI):m/z = [M+H]+: 420.41.

### Embodiment 4: compound 4 synthesis

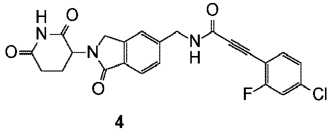

¹H NMR (400 MHz, DMSO-76): 10.95 (s, 1H), 9.53 (s, 1H), 7.67-7.62 (m, 4H), 7.49 (s, 1H), 7.40 (t, *J* = 8 Hz, 1H), 5.09 (dd, *J* = 12, 4Hz, 1H), 4.44-4.40 (m, 3H), 4.31 (d, *J* = 12 Hz, 1H), 2.92-2.89 (m, 1H), 2.58 (d, *J* = 16 Hz, 1H), 2.37-2.33 (m, 1H), 1.98-1.93 (m, 1H). Molecular Formula: C23H17C1FN3O4, theoretical molecular weight: 453.85; LC-MS(ESI):m/z=[M+H]+: 454.85.

### Embodiment 5: compound 5 synthesis

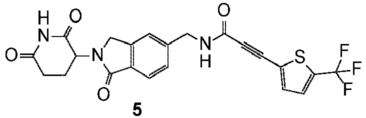

¹H NMR (400 MHz, DMSO-d6) : 10.97 (s, 1H), 9.58 (t, *J =* 4 Hz, 1H), 7.78 (d, *J* = 4 Hz, 1H), 7.69 (d, *J* = 8 Hz, 1H), 7.65 (d, *J* = 4 Hz, 1H), 7.49 (s, 1H), 7.41 (d, *J* = 8 Hz, 1H), 5.09 (dd, *J* = 12, 4Hz, 1H), 4.44-4.38 (m, 3H), 4.30 (d, *J* = 16 Hz, 1H), 2.89-2.82 (m, 1H), 2.58 (d, *J* = 16 Hz, 1H), 2.37-2.33 (m, 1H), 1.98-1.92 (m, 1H). Molecular Formula: C22H16F3N3O4S, theoretical molecular weight: 475.44; LC-MS(ESI):m/z = [M+H]+: 476.44.

### Embodiment 6: compound 6 synthesis

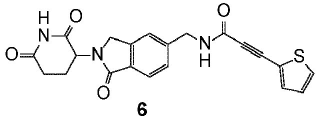

¹H NMR (400 MHz, DMSO-76): 10.96 (s, 1H), 9.42 (t, *J* = 4 Hz, 1H), 7.79 (d, *J* = 4 Hz, 1H), 7.69 (d, *J* = 8 Hz, 1H), 7.55 (d, *J* = 4 Hz, 1H), 7.49 (s, 1H), 7.41 (d, *J* = 4 Hz, 1H), 7.15-7.10 (m, 1H), 5.09 (dd, *J* = 12, 4Hz, 1H), 4.44-4.41 (m, 3H) , 4.30 (d, *J* = 12 Hz, 1H), 2.89-2.83 (m, 1H), 2.58 (d, *J* = 12 Hz, 1H), 2.37-2.32 (m, 1H), 1.98-1.94 (m, 1H). Molecular Formula: C21H17N3O4S, theoretical molecular weight: 407.44; LC-MS(ESI):m/z = [M+H]+: 408.44.

### Embodiment 7: compound 7 synthesis

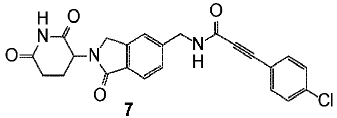

¹H NMR (400 MHz, DMSO-76): 10.96 (s, 1H), 9.43 (t, *J* = 4 Hz, 1H), 7.69 (d, *J* = 8 Hz, 1H), 7.59 (d, *J* = 4 Hz, 2H), 7.52-7.48 (m, 3H), 7.41 (d, *J* = 4 Hz, 1H), 5.09 (dd, *J*= *12, 4Hz, 1H), 4.45-4.40 (m, 3H), 4.30 (d, J* = 12 Hz, 1H), 2.89-2.81 (m, 1H), 2.58 (d, *J* = 16 Hz, 1H), 2.38-2.33 (m, 1H), 1.99-1.94 (m, 1H). Molecular Formula: C23H18C1N3O4, theoretical molecular weight: 435.86; LC-MS(ESI):m/z = [M+H]+: 436.86.

### Embodiment 8: compound 8 synthesis

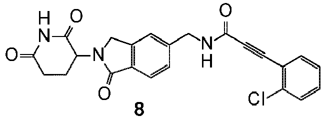

¹H NMR (400 MHz, DMSO-d6): 10.99 (s, 1H), 9.49 (t, J = 4 Hz, 1H), 7.71 (d, J = 8 Hz, 2H), 7.62 (d, J = 8 Hz, 1H), 7.52-7.49 (m, 2H), 7.44 (d, J = 8 Hz, 2H), 5.11 (dd, J= 12, 4Hz, 1H), 4.47-4.41 (m, 3H), 4.30 (d, J = 20 Hz, 1H), 2.90-2.85 (m, 1H), 2.60 (d, J = 16 Hz, 1H), 2.38-2.31 (m, 1H), 1.98-1.94 (m, 1H). Molecular Formula: C23H18C1N3O4, theoretical molecular weight: 435.86; LC-MS(ESI):m/z = [M+H]+: 436.86.

### Embodiment 9: compound 9 synthesis

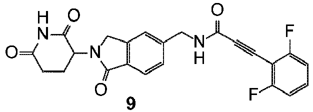

¹H NMR (400 MHz, DMSO-d6): 10.99 (s, 1H), 9.59 (t, J = 4 Hz, 1H), 7.71 (dd, J= 8, 4Hz, 1H), 7.64-7.60 (m, 1H), 7.52 (s, 1H), 7.43 (d, J = 8 Hz, 1H), 7.30 (dt, J = 8 Hz, 2H), 5.12 (d, J = 12Hz, 1H), 4.47-4.41 (m, 3H), 4.32 (d, J = 16 Hz, 1H), 2.91-2.85 (m, 1H), 2.60-2.55 (m, 1H), 2.38-2.31 (m, 1H), 1.98-1.93 (m, 1H). Molecular Formula: C23H17F2N3O4, theoretical molecular weight: 437.40; LC-MS(ESI):m/z = [M+H]+: 438.40.

### Embodiment 10: compound 10 synthesis

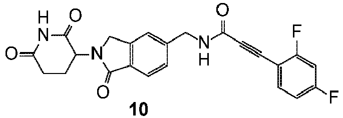

¹H NMR (400 MHz, DMSO-d6) : 10.97 (s, 1H), 9.49 (t, *J* = 4 Hz, 1H), 7.72-7.65 (m, 2H), 7.47-7.41 (m, 2H), 7.41 (d, *J* = 8 Hz, 1H) , 7.21 (t, *J* = 8 Hz, 1H), 5.09 (dd, *J* = 12, 4Hz, 1H), 4.46-4.39 (m, 3H), 4.31 (d, *J* = 12 Hz, 1H), 2.89-2.82 (m, 1H), 2.59 (d, *J* = 16 Hz, 1H), 2.38-2.33 (m, 1H), 1.98-1.94 (m, 1H). Molecular Formula: C23H17F2N3O4, theoretical molecular weight: 437.40; LC-MS(ESI):m/z = [M+H]+: 438.40.

### Embodiment 11: compound 11 synthesis

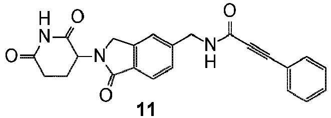

¹H NMR (400 MHz, DMSO-d6) : 10.96 (s, 1H), 9.40 (t, *J* = 4 Hz, 1H), 7.69 (d, *J* = 8 Hz, 1H), 7.57-7.50 (m, 2H), 7.49 (d, 2H), 7.45-7.41 (m, 3H), 5.10 (dd, *J* = 12, 4Hz, 1H), 4.46-4.40 (m, 3H), 4.31 (d, *J* = 12 Hz, 1H), 2.90-2.82 (m, 1H), 2.59 (d, *J* = 16 Hz, 1H), 2.37-2.31 (m, 1H), 1.99-1.86 (m, 1H). Molecular Formula: C23H19N3O4, theoretical molecular weight: 401.42; LC-MS(ESI):m/z=[M+H]+: 402.42.

### Embodiment 12: compound 12 synthesis

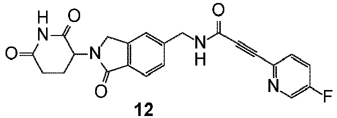

¹H NMR (400 MHz, DMSO-d6) : 10.99 (s, 1H), 9.60 (s, 1H), 8.68 (s, 1H), 7.83-7.78 (m, 2H), 7.71 (d, *J* = 8 Hz, 1H), 7.57 (s, 1H), 7.43 (d, *J* = 8 Hz, 1H), 5.11 (dd, *J*= *12, 4Hz, 1H), 4.46-4.41 (m, 3H), 4.32 (d, J* = *12* Hz, 1H), 2.90-2.81 (m, 1H), 2.59 (d, *J* = 16 Hz, 1H), 2.37-2.32 (m, 1H), 1.99-1.92 (m, 1H). Molecular Formula: C22H17FN4O4, theoretical molecular weight: 420.40; LC-MS(ESI):m/z= [M+H]+: 421.40.

### Embodiment 13: compound 13 synthesis

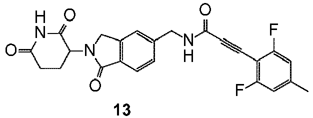

¹H NMR (400 MHz, DMSO-d6) : 10.99 (s, 1H), 9.56 (s, 1H), 7.71 (d, *J* = 8 Hz, 1H), 7.52 (s, 1H), 7.43 (d, *J =* 8 Hz, 1H), 7.15 (d, *J* = 12 Hz, 2H), 5.10-5.06 (m, 1H), 4.46-4.41 (m, 3H), 4.32 (d, *J* = 16 Hz, 1H), 2.90-2.82 (m, 1H), 2.59-2.52 (m, 1H), 2.37-2.31 (m, 4H), 2.02-1.96 (m, 1H). Molecular Formula: C24H19F2N3O4, theoretical molecular weight: 451.43; LC-MS(ESI):m/z = [M+H]+: 452.43.

### Embodiment 14: compound 14

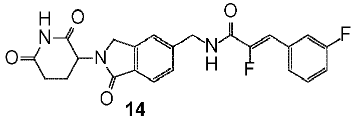

¹H NMR (400 MHz, DMSO-d6) : 10.96 (s, 1H), 9.30 (s, 1H), 7.66(d, *J* = 4 Hz, 1H), 7.56 (d, *J* = 8 Hz, 1H), 7.46 (s, 1H), 7.40 (d, *J* = 4 Hz, 1H), 7.32-7.28 (m, 2H), 7.13 (s, 1H), 6.87 (d, *J* = 16 Hz, 1H), 5.09 (dd, *J* = 8, 4Hz, 1H), 4.47-4.42 (m, 2H), 4.35 (dd, *J* = 20 Hz, 2H), 2.90-2.84 (m, 1H), 2.59 (d, *J* = 16 Hz, 1H), 2.37-2.33 (m, 1H), 1.98-1.92 (m, 1H). Molecular Formula: C23H19F2N3O4, theoretical molecular weight: 439.42; LC-MS(ESI):m/z = [M+H]+: 440.42.

### Embodiment 15: compound 15

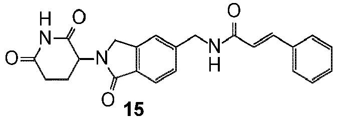

¹H NMR (400 MHz, DMSO-*d6*) : 10.96 (s, 1H), 8.72 (s, 1H), 7.50-7.42 (m, 9H), 6.69 (d, *J* = 8 Hz, 1H), 5.09 (d, *J*= *4Hz, 1H), 4.51 (s, 2H), 4.36 (dd, J* = 52, 12 Hz, 2H), 2.89-2.84 (m, 1H), 2.59 (d, *J* = 12 Hz, 1H), 2.38-2.33 (m, 1H), 1.98-1.92 (m, 1H). Molecular Formula: C23H21N3O4, theoretical molecular weight: 403.44; LC-MS(ESI):m/z=[M+H]+: 404.44.

### Embodiment 16: compound 16 synthesis

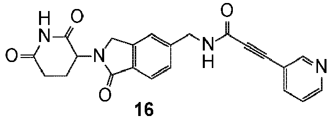

¹H NMR (400 MHz, DMSO-*d6*) : 10.99 (s, 1H), 9.53 (s, 1H), 8.78 (dd, J = 2.3, 1.0 Hz, 1H), 8.67 (dd, J = 4.9, 1.7 Hz, 1H), 8.03 (dt, J = 7.9, 1.9 Hz, 1H), 7.71 (d, J = 7.7 Hz, 1H) , 7.55-7.47 (m, 2H), 7.47-7.40 (m, 1H), 5.11 (dd, J = 13.3, 5.1 Hz, 1H), 4.51-4.42 (m, 3H), 4.32 (d, J = 17.4 Hz, 1H), 2.97-2.87 (m, 1H), 2.62-2.49 (m, 1H), 2.39 (dd, J = 13.1, 4.5 Hz, 1H), 2.04-1.97 (m, 1H). Molecular Formula: C22H18N4O4, theoretical molecular weight: 402.41; LC-MS(ESI):m/z=[M+H]+: 403.41.

### Embodiment 17: compound 17 synthesis

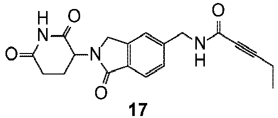

¹H NMR (400 MHz, DMSO-*d6*) : 10.98 (s, 1H), 9.11 (s, 1H), 7.69 (d, J = 7.8 Hz, 1H), 7.45 (s, 1H), 7.37 (d, J = 8.2 Hz, 1H), 5.10 (dd, J = 13.3, 5.1 Hz, 1H), 4.44 (d, J = 17.3 Hz, 1H), 4.38 (d, J = 6.1 Hz, 2H), 4.30 (d, J = 17.3 Hz, 1H), 2.96-2.87 (m, 1H), 2.63-2.57 (m, 1H), 2.41-2.32 (m, 3H), 2.04-1.95 (m, 1H), 1.11 (t, J = 7.5 Hz, 3H). Molecular Formula: C19H19N3O4, theoretical molecular weight: 353.38; LC-MS(ESI):m/z = [M+H]+: 354.38.

### Embodiment 18: compound 18 synthesis

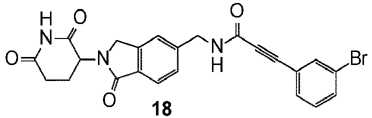

¹H NMR (400 MHz, DMSO-*d6*): 10.99 (s, 1H), 9.47 (s, 1H), 7.80 (t, J = 1.8 Hz, 1H), 7.77-7.68 (m, 2H), 7.61 (dt, J = 7.8, 1.3 Hz, 1H), 7.52 (s, 1H), 7.47-7.37 (m, 2H), 5.11 (dd, J = 13.3, 5.1 Hz, 1H), 4.52-4.41 (m, 3H), 4.32 (d, J = 17.4 Hz, 1H), 2.93-2.85 (m, 1H), 2.68-2.56 (m, 1H), 2.42-2.38 (m, 1H), 2.03-1.98 (m, 1H). Molecular Formula: C23H18BrN3O4, theoretical molecular weight: 480.32; LC-MS(ESI):m/z= [M+H]+: 481.32.

### Embodiment 19: compound 19 synthesis

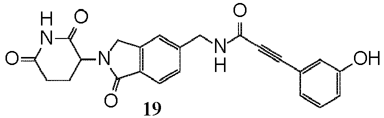

¹H NMR (400 MHz, DMSO-*d6*): 10.99 (s, 1H), 9.86 (s, 1H), 9.40 (s, 1H), 7.71 (d, J = 7.8 Hz, 1H), 7.51 (s, 1H), 7.46-7.39 (m, 1H), 7.25 (t, J = 7.8 Hz, 1H), 7.00 (d, J = 8.0 Hz, 1H), 6.95-6.86 (m, 2H), 5.11 (dd, J = 13.3, 5.1 Hz, 1H), 4.52-4.37 (m, 3H), 4.32 (d, J = 17.4 Hz, 1H), 2.96-2.87 (m, 1H), 2.65-2.55 (m, 1H), 2.47-2.32 (m, 1H), 2.04-1.96 (m, 1H). Molecular Formula: C23H19N3O5, theoretical molecular weight: 417.42; LC-MS(ESI):m/z = [M+H]+: 418.42.

### Embodiment 20: compound 20 synthesis

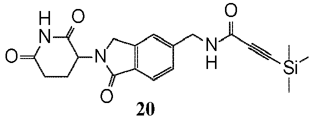

¹H NMR (400 MHz, DMSO-d6): 10.96 (s, 1H), 9.25 (s, 1H), 7.67 (d, J = 7.8 Hz, 1H), 7.42 (s, 1H), 7.42-7.35 (m, 1H), 5.10 (dd, J = 13.3, 5.1 Hz, 1H), 4.53-4.37 (m, 3H), 4.34 (d, J = 17.5 Hz, 1H), 2.98-2.86 (m, 1H), 2.63-2.59 (m, 1H), 2.43-2.39 (m, 1H), 2.05-1.95 (m, 1H), 0.07 (s, 9H). Molecular Formula: C20H23N3O4Si, theoretical molecular weight: 397.51; LC-MS(ESI):m/z= [M+H]+: 398.51.

### Embodiment 21: compound 21 synthesis

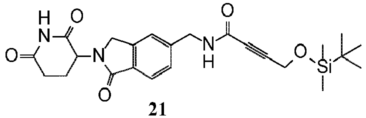

¹H NMR (400 MHz, DMSO-d6) : 10.96 (s, 1H), 9.24 (s, 1H), 7.65 (d, J = 7.8 Hz, 1H), 7.44 (s, 1H), 7.40-7.35 (m, 1H), 5.10 (dd, J = 13.3, 5.1 Hz, 1H), 4.52-4.36 (m, 3H), 4.32 (d, J = 17.5 Hz, 1H), 4.20 (d, J = 6.1 Hz, 2H), 2.99-2.86 (m, 1H), 2.62-2.55 (m, 1H), 2.41-2.39 (m, 1H), 2.05-1.93 (m, 1H) 0.21 (s, 6H), 0.06 (s, 9H). Molecular Formula: C24H31N3O5Si, theoretical molecular weight: 469.61; LC-MS(ESI):m/z = [M+H]+: 470.61.

### Embodiment 22: compound 22 synthesis

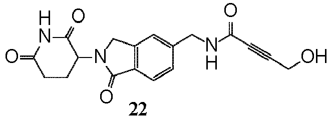

¹H NMR (400 MHz, DMSO-*d6*): 10.98 (s, 1H), 9.26 (s, 1H), 7.69 (d, J = 7.8 Hz, 1H), 7.46 (s, 1H), 7.41-7.34 (m, 1H), 5.47 (t, J = 6.0 Hz, 1H), 5.11 (dd, J = 13.3, 5.1 Hz, 1H), 4.51-4.35 (m, 3H), 4.31 (d, J = 17.5 Hz, 1H), 4.23 (d, J = 6.1 Hz, 2H), 2.97-2.85 (m, 1H), 2.64-2.57 (m, 1H), 2.42-2.38 (m, 1H), 2.06-1.94 (m, 1H). Molecular Formula: C18H17N3O5, theoretical molecular weight: 355.35; LC-MS(ESI):m/z = [M+H]+: 356.35.

### Embodiment 23: compound 23 synthesis

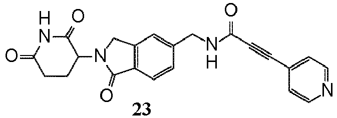

¹H NMR (400 MHz, DMSO-*d6*): 10.98 (s, 1H), 9.60 (s, 1H), 8.71-8.63 (m, 2H), 7.71 (d, J = 7.8 Hz, 1H), 7.60-7.53 (m, 2H), 7.52 (s, 1H), 7.43 (dd, J = 7.9, 1.5 Hz, 1H), 5.11 (dd, J = 13.3, 5.1 Hz, 1H), 4.51-4.42 (m, 3H), 4.32 (d, J = 17.4 Hz, 1H), 2.97-2.86 (m, 1H), 2.65-2.56 (m, 1H), 2.42-2.38 (m, 1H), 2.04-1.96 (m, 1H). Molecular Formula: C22H18N4O4, theoretical molecular weight: 402.41; LC-MS(ESI):m/z = [M+H]+: 403.41.

### Embodiment 24: compound 24 synthesis

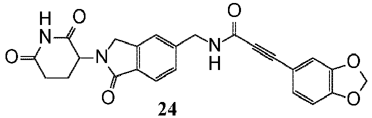

¹H NMR (400 MHz, DMSO-d6) : 10.99 (s, 1H), 9.33 (s, 1H), 7.71 (d, J = 7.8 Hz, 1H), 7.50 (s, 1H), 7.46-7.39 (m, 1H), 7.17-7.09 (m, 2H), 7.00 (d, J = 7.9 Hz, 1H), 6.11 (s, 2H), 5.11 (dd, J = 13.3, 5.1 Hz, 1H), 4.50-4.41 (m, 3H), 4.32 (d, J = 17.4 Hz, 1H), 2.96-2.87 (m, 1H), 2.64-2.57 (m, 1H), 2.42-2.38 (m, 1H), 2.05-1.95 (m, 1H). Molecular Formula: C24H19N3O6, theoretical molecular weight: 445.43; LC-MS(ESI):m/z=[M+H]+: 446.43.

### Embodiment 25: compound 25 synthesis

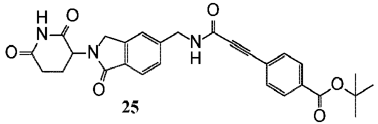

¹H NMR (400 MHz, DMSO-*d6*) : 10.98 (s, 1H), 9.53 (s, 1H), 8.02-7.89 (m, 2H), 7.78-7.59 (m, 3H), 7.52 (s, 1H), 7.43 (d, J = 7.9 Hz, 1H), 5.11 (dd, J = 13.2,5.1 Hz, 1H), 4.56- 4.38 (m, 3H), 4.32 (d, J = 17.4 Hz, 1H), 2.96-2.86 (m, 1H), 2.63- 2.56 (m, 1H), 2.43-2.35 (m, 1H), 2.03-1.95 (m, 1H), 1.55 (s, 9H). Molecular Formula: C28H27N3O6, theoretical molecular weight: 501.54; LC-MS(ESI):m/z = [M+H]+: 502.54.

### Embodiment 26: compound 26 synthesis

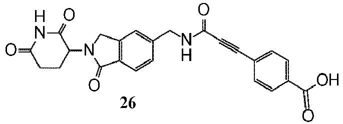

¹H NMR (400 MHz, DMSO-*d6*): 12.08 (s, 1H), 10.97 (s, 1H), 9.52 (s, 1H), 8.01-7.88 (m, 2H), 7.79-7.59 (m, 3H), 7.51 (s, 1H), 7.44 (d, J = 7.9 Hz, 1H), 5.12 (dd, J = 13.2, 5.1 Hz, 1H), 4.54-4.38 (m, 3H), 4.33 (d, J = 17.4 Hz, 1H), 2.97-2.85 (m, 1H), 2.62-2.57 (m, 1H), 2.43-2.36 (m, 1H), 2.04-1.97 (m, 1H). Molecular Formula: C24H19N3O6, theoretical molecular weight: 445.43; LC-MS(ESI):m/z= [M+H]+: 446.43.

### Embodiment 27: compound 27 synthesis

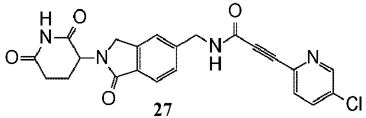

¹H NMR (400 MHz, DMSO-d6): 11.00 (s, 1H), 9.65 (s, 1H), 8.72 (d, J = 2.5 Hz, 1H), 8.07 (dd, J = 8.4, 2.6 Hz, 1H), 7.74-7.65 (m, 2H), 7.52 (s, 1H), 7.43 (d, J = 7.8 Hz, 1H), 5.12 (dd, J = 13.3, 5.0 Hz, 1H), 4.51-4.41 (m, 3H), 4.32 (d, J = 17.4 Hz, 1H), 2.94-2.86 (m, 1H), 2.66-2.58 (m, 1H), 2.43-2.35 (m, 1H), 2.04-1.97 (m, 1H). Molecular Formula: C22H17C1N4O4, theoretical molecular weight: 436.85; LC-MS(ESI):m/z = [M+H]+: 437.85.

### Embodiment 28: compound 28 synthesis

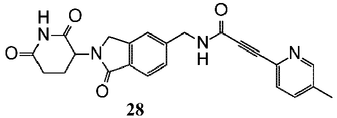

¹H NMR (400 MHz, DMSO-*d6*): 10.99 (s, 1H), 9.56 (s, 1H), 8.50 (d, J = 2.2 Hz, 1H), 7.71 (dd, J = 7.8, 3.0 Hz, 2H), 7.60 (d, J = 8.0 Hz, 1H), 7.51 (s, 1H), 7.43 (dd, J = 7.7, 1.4 Hz, 1H), 5.11 (dd, J = 13.3, 5.1 Hz, 1H), 4.50-4.42 (m, 3H), 4.32 (d, J = 17.3 Hz, 1H), 2.96-2.87 (m, 1H), 2.66-2.58 (m, 1H), 2.42-2.37 (m, 1H), 2.34 (s, 3H), 2.04-1.97 (m, 1H). Molecular Formula: C23H20N4O4, theoretical molecular weight: 416.44; LC-MS(ESI):m/z = [M+H]+: 417.44.

### Embodiment 29: compound 29 synthesis

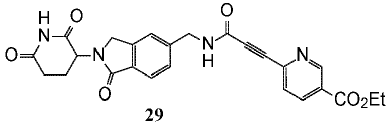

¹H NMR (400 MHz, DMSO-*d6*): 10.99 (s, 1H), 9.56 (s, 1H), 8.50 (d, J = 2.2 Hz, 1H), 7.71 (dd, J = 7.8, 3.0 Hz, 2H), 7.60 (d, J = 8.0 Hz, 1H), 7.51 (s, 1H), 7.43 (dd, J = 7.7, 1.4 Hz, 1H), 5.11 (dd, J = 13.3, 5.1 Hz, 1H), 4.50-4.42 (m, 3H), 4.32 (d, J = 17.3 Hz, 1H), 4.05-4.00 (m, 2H), 2.96-2.87 (m, 1H), 2.66-2.58 (m, 1H), 2.42-2.37 (m, 1H), 2.04-1.97 (m, 1H), 1.53 (t, J = 11.3 Hz, 3H). Molecular Formula: C25H22N4O6, theoretical molecular weight: 474.47; LC-MS(ESI):m/z = [M+H]+: 475.47.

### Embodiment 30: compound 30 synthesis

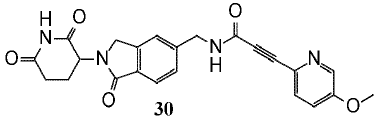

¹H NMR (400 MHz, DMSO-*d6*): 10.99 (s, 1H), 9.51 (s, 1H), 8.35 (d, J = 2.9 Hz, 1H), 7.69 (dd, J = 13.1, 8.3 Hz, 2H), 7.51 (s, 1H), 7.49-7.35 (m, 2H), 5.11 (dd, J = 13.3, 5.1 Hz, 1H), 4.54-4.39 (m, 3H), 4.32 (d, J = 17.3 Hz, 1H), 3.88 (s, 3H), 2.95-2.86 (m, 1H), 2.63-2.61 (m, 1H), 2.43-2.38 (m, 1H), 2.04-1.97 (m, 1H). Molecular Formula: C23H20N4O5, theoretical molecular weight: 432.44; LC-MS(ESI):m/z = [M+H]+: 433.44.

### Embodiment 31: compound 31 synthesis

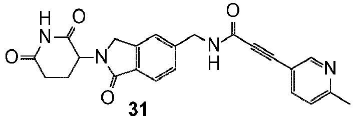

¹H NMR (400 MHz, DMSO-*d6*): 10.98 (s, 1H), 9.49 (s, 1H), 8.65 (d, J = 2.3 Hz, 1H), 7.90 (dd, J = 8.0, 2.3 Hz, 1H), 7.71 (d, J = 7.8 Hz, 1H), 7.51 (s, 1H), 7.43 (d, J = 7.9 Hz, 1H), 7.37 (d, J = 8.1 Hz, 1H), 5.11 (dd, J = 13.3, 5.1 Hz, 1H), 4.53-4.40 (m, 3H), 4.32 (d, J = 17.4 Hz, 1H), 2.96-2.87 (m, 1H), 2.63-2.57 (m, 1H), 2.52 (s, 3H), 2.44-2.36 (m, 1H), 2.05-1.95 (m, 1H). Molecular Formula: C23H20N4O4, theoretical molecular weight: 416.44; LC-MS(ESI):m/z = [M+H]+: 417.44.

### Embodiment 32: compound 32 synthesis

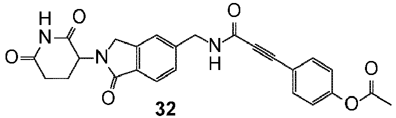

¹H NMR (400 MHz, DMSO-*d6*): 10.96 (s, 1H), 9.43 (s, 1H), 7.69 (d, J = 8 Hz, 1H), 7.59 (d, J = 4 Hz, 2H), 7.52-7.50 (m, 3H), 7.41 (d, J = 4 Hz, 1H), 5.09 (dd, J= 12, 4Hz, 1H), 4. 45-4.43 (m, 3H), 4.30 (d, J = 12 Hz, 1H), 2.89-2.83 (m, 1H), 2.58 (d, J = 16 Hz, 1H), 2.38-2.33 (m, 1H), 2.02 (s, 3H), 1.99-1.92 (m, 1H). Molecular Formula: C25H21N3O6, theoretical molecular weight: 459.46; LC-MS(ESI):m/z = [M+H]+: 460.46.

### Embodiment 33: compound 33 synthesis

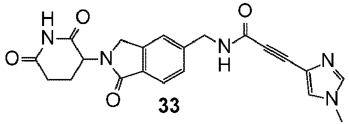

¹H NMR (400 MHz, DMSO-d6): 10.98 (s, 1H), 9.34 (s, 1H), 7.74-7.66 (m, 3H), 7.49 (s, 1H), 7.41 (d, J = 7.8 Hz, 1H), 5.11 (dd, J = 13.3, 5.1 Hz, 1H), 4.50-4.39 (m, 3H), 4.31 (d, J = 17.4 Hz, 1H), 3.67 (s, 3H), 2.97-2.85 (m, 1H), 2.63-2.57 (m, 1H), 2.43-2.38 (m, 1H), 2.04-1.96 (m, 1H). Molecular Formula: C21H19N5O4, theoretical molecular weight: 405.41; LC-MS(ESI):m/z = [M+H]+: 406.41.

### Embodiment 34: compound 34 synthesis

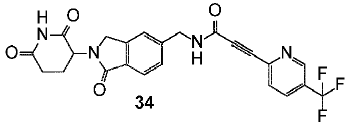

¹H NMR (400 MHz, DMSO-*d6*) : 10.99 (s, 1H), 9.72 (s, 1H), 9.07 (d, J = 2.3 Hz, 1H), 8.35 (dd, J = 8.4, 2.4 Hz, 1H), 7.95 (d, J = 8.2 Hz, 1H), 7.72 (d, J = 7.8 Hz, 1H), 7.53 (s, 1H), 7.48-7.41 (m, 1H), 5.11 (dd, J = 13.3, 5.1 Hz, 1H), 4.50 (d, J = 6.5 Hz, 2H), 4.44 (s, 1H), 4.32 (d, J = 17.3 Hz, 1H), 2.95-2.86 (m, 1H), 2.67-2.62 (m, 1H), 2.43-2.38 (m, 1H), 2.06-1.96 (m, 1H). Molecular Formula: C23H17F3N4O4, theoretical molecular weight 470.41 LC-MS(ESI):m/z = [M+H]+: 471.41.

### Embodiment 35: compound 35 synthesis

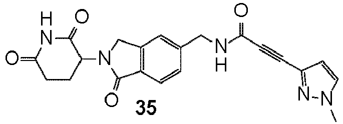

¹H NMR (400 MHz, DMSO-d6) : 10.97 (s, 1H), 9.54 (s, 1H), 7.71 (d, J = 7.8 Hz, 1H), 7.54 (d, J = 2.1 Hz, 1H), 7.51 (s, 1H), 7.42 (d, J = 7.9 Hz, 1H), 6.73 (d, J = 2.0 Hz, 1H), 5.10 (dd, J = 13.3, 5.1 Hz, 1H), 4.51-4.41 (m, 3H), 4.31 (d, J = 17.4 Hz, 1H), 3.92 (s, 3H), 2.96-2.88 (m, 1H), 2.63-2.59 (m, 1H), 2.42-2.37 (m, 1H), 2.01-1.96 (m, 1H). Molecular Formula: C21H19N5O4, theoretical molecular weight: 405.41; LC-MS(ESI):m/z= [M+H]+: 406.41.

### Embodiment 36: compound 36 synthesis

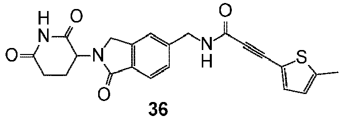

¹H NMR (400 MHz, DMSO-d6) : 10.99 (s, 1H), 9.39 (s, 1H), 7.70 (d, J = 7.8 Hz, 1H), 7.50 (s, 1H), 7.45-7.39 (m, 1H), 7.38 (d, J = 3.6 Hz, 1H), 6.89-6.87 (m, 1H), 5.11 (dd, J = 13.3, 5.1 Hz, 1H), 4.52-4.38 (m, 3H), 4.31 (d, J = 17.4 Hz, 1H), 2.95-2.86 (m, 1H), 2.62-2.60 (m, 1H), 2.52 (s, 3H), 2.43-2.34 (m, 1H), 2.05-1.94 (m, 1H). Molecular Formula C22H19N3O4S, theoretical molecular weight: 421.47; LC-MS(ESI):m/z = [M+H]+: 422.47.

### Embodiment 37: compound 37 synthesis

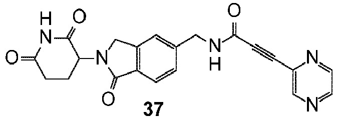

¹H NMR (400 MHz, DMSO-*d6*) : 11.02 (s, 1H), 9.53 (s, 1H), 8.51 (d, J = 2.4 Hz, 1H), 8.22 (td, J = 8.1, 2.4 Hz, 1H), 7.71 (d, J = 7.8 Hz, 1H), 7.53 (s, 1H), 7.46 (d, J = 7.8 Hz, 1H), 7.33 (dd, J = 8.5, 2.8 Hz, 1H), 5.11 (dd, J = 13.3, 5.0 Hz, 1H), 4.56-4.38 (m, 3H), 4.33 (d, J = 17.4 Hz, 1H), 2.95-2.89 (m, 1H), 2.63-2.57 (m, 1H), 2.43-2.31 (m, 1H), 2.03-1.97 (m, 1H). Molecular Formula: C21H17N5O4, theoretical molecular weight: 403.4; LC-MS(ESI):m/z= [M+H]+: 404.40.

### Embodiment 38: compound 38 synthesis

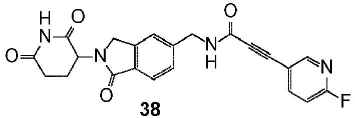

¹H NMR (400 MHz, DMSO-d6) : 11.00 (s, 1H), 9.54 (s, 1H), 8.52 (d, J = 2.4 Hz, 1H), 8.23 (td, J = 8.1, 2.4 Hz, 1H), 7.72 (d, J = 7.8 Hz, 1H), 7.52 (s, 1H), 7.43 (d, J = 7.8 Hz, 1H), 7.34 (dd, J = 8.5, 2.8 Hz, 1H), 5.12 (dd, J = 13.3, 5.0 Hz, 1H), 4.57-4.39 (m, 3H), 4.32 (d, J = 17.4 Hz, 1H), 2.97-2.87 (m, 1H), 2.63-2.55 (m, 1H), 2.44-2.32 (m, 1H), 2.05-1.96 (m, 1H). Molecular Formula: C22H17FN4O4, theoretical molecular weight: 420.4; LC-MS(ESI):m/z = [M+H]+: 421.40.

### Embodiment 39: compound 39 synthesis

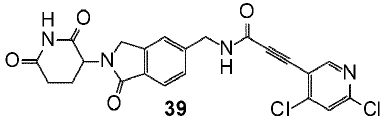

¹H NMR (400 MHz, DMSO-*d6*) : 10.99 (s, 1H), 9.62 (s, 1H), 8.72 (s, 1H), 8.06 (s, 1H), 7.72 (d, J = 7.8 Hz, 1H), 7.52 (s, 1H), 7.44 (d, J = 7.7 Hz, 1H), 5.11 (dd, J = 13.3, 5.1 Hz, 1H), 4.52 - 4.42 (m, 3H), 4.32 (d, J = 17.4 Hz, 1H), 2.97-2.85 (m, 1H), 2.64-2.57 (m, 1H), 2.43-2.38 (m, 1H), 2.04 - 1.96 (m, 1H). Molecular Formula: C22H16C12N4O4, theoretical molecular weight: 471.29; LC-MS(ESI):m/z = [M+H]+: 472.29.

### Embodiment 40: compound 40 synthesis

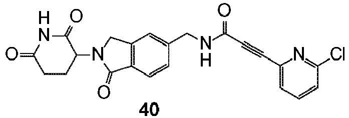

¹H NMR (400 MHz, DMSO-*d6*) : 10.99 (s, 1H), 9.67 (s, 1H), 7.98 (t, J = 7.8 Hz, 1H), 7.80-7.59 (m, 3H), 7.53 (d, J = 8.1 Hz, 1H), 7.43 (d, J = 7.9 Hz, 1H), 5.11 (dd, J = 13.2, 5.1 Hz, 1H), 4.58-4.39 (m, 3H), 4.32 (d, J = 17.4 Hz, 1H), 2.96-2.86 (m, 1H), 2.64-2.60 (m, 1H), 2.43-2.38 (m, 1H), 2.04-1.94 (m, 1H). Molecular Formula: C22H17C1N4O4, theoretical molecular weight: 436.85; LC-MS(ESI):m/z = [M+H]+: 437.85.

### Embodiment 41: compound 41 synthesis

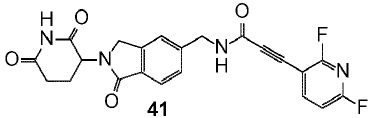

¹H NMR (400 MHz, DMSO-*d6*): 10.99 (s, 1H), 9.59 (s, 1H), 8.50-8.39 (m, 1H), 7.73-7.69 (m, 1H), 7.51 (d, J = 6.7 Hz, 1H), 7.46-7.40 (m, 1H), 7.34 (dd, J = 8.3, 2.3 Hz, 1H), 5.11 (dd, J = 13.3, 5.0 Hz, 1H), 4.53-4.40 (m, 3H), 4.32 (d, J = 17.4 Hz, 1H), 2.97-2.87 (m, 1H), 2.64-2.60 (m, 1H), 2.42-2.35 (m, 1H), 2.04-1.96 (m, 1H). Molecular Formula: C22H16F2N4O4, theoretical molecular weight: 438.39; LC-MS(ESI):m/z = [M+H]+: 439.39.

### Embodiment 42: compound 42 synthesis

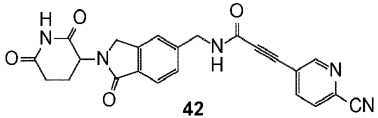

¹H NMR (400 MHz, DMSO-*d6*): 10.97 (s, 1H), 9.48 (s, 1H), 8.65 (d, J = 2.3 Hz, 1H), 7.88 (dd, J = 8.0, 2.3 Hz, 1H), 7.70 (d, J = 7.8 Hz, 1H), 7.48 (s, 1H), 7.42 (d, J = 7.9 Hz, 1H), 7.36 (d, J = 8.1 Hz, 1H), 5.07 (dd, J = 13.3, 5.1 Hz, 1H), 4.51-4.40 (m, 3H), 4.30 (d, J = 17.4 Hz, 1H), 2.94-2.87 (m, 1H), 2.61-2.57 (m, 1H), 2.41-2.35 (m, 1H), 2.04-1.95 (m, 1H). Molecular Formula: C23H17N5O4, theoretical molecular weight: 427.42; LC-MS(ESI):m/z = [M+H]+: 428.42.

### Embodiment 43: compound 43 synthesis

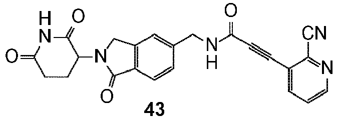

¹H NMR (400 MHz, DMSO-*d6*): 10.98 (s, 1H), 9.47 (s, 1H), 8.67 (d, J = 2.3 Hz, 1H), 7.88 (t, J = 8.0, 1H), 7.72 (d, J = 7.8 Hz, 1H), 7.46 (s, 1H), 7.41 (d, J = 7.9 Hz, 1H), 7.33 (d, J = 8.1 Hz, 1H), 5.08 (dd, J = 13.3, 5.12 Hz, 1H), 4.51-4.43 (m, 3H), 4.32 (d, J = 17.4 Hz, 1H), 2.94-2.89 (m, 1H), 2.62-2.57 (m, 1H), 2.41-2.37 (m, 1H), 2.06-1.95 (m, 1H). Molecular Formula: C23H17N5O4, theoretical molecular weight: 427.42; LC-MS(ESI):m/z=[M+H]+: 428.42.

### Embodiment 44: compound 44 synthesis

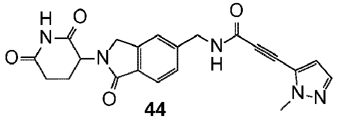

¹H NMR (400 MHz, DMSO-*d6*): 10.99 (s, 1H), 9.56 (s, 1H), 7.72 (d, J = 7.8 Hz, 1H), 7.56 (d, J = 2.1 Hz, 1H), 7.52 (s, 1H), 7.44 (d, J = 7.9 Hz, 1H), 6.75 (d, J = 2.0 Hz, 1H), 5.11 (dd, J = 13.3, 5.1 Hz, 1H), 4.52-4.43 (m, 3H), 4.32 (d, J = 17.4 Hz, 1H), 3.94 (s, 3H), 2.95-2.86 (m, 1H), 2.62-2.58 (m, 1H), 2.43-2.38 (m, 1H), 2.03-1.97 (m, 1H). Molecular Formula: C21H19N5O4, theoretical molecular weight:405.41; LC-MS(ESI):m/z = [M+H]+: 406.41.

### Embodiment 45: compound 45 synthesis

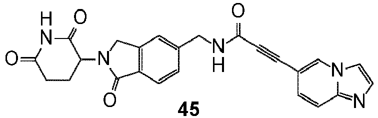

¹H NMR (400 MHz, DMSO-*d6*): 10.98 (s, 1H), 9.48 (s, 1H), 8.80-8.75 (m, 1H), 8.70-8.67 (m, 1H), 8.67 (d, J = 2.3 Hz, 1H), 7.89 (d, J = 8.0, 1H), 7.72 (d, J = 7.8 Hz, 1H), 7.49 (s, 1H), 7.41 (d, J = 7.9 Hz, 1H), 7.35 (d, J = 8.1 Hz, 1H), 5.06 (dd, J = 13.3, 5.1 Hz, 1H), 4.51-4.43 (m, 3H), 4.32 (d, J = 17.4 Hz, 1H), 2.94-2.89 (m, 1H), 2.62-2.57 (m, 1H), 2.40-2.36 (m, 1H), 2.04-1. 97 (m, 1H). Molecular Formula: C24H19N5O4, theoretical molecular weight: 441.45; LC-MS(ESI):m/z= [M+H]+: 442.45.

### Embodiment 46: compound 46 synthesis

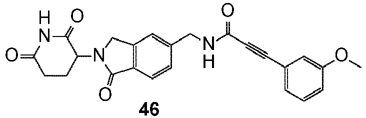

¹H NMR (400 MHz, DMSO-*d6*): 10.99 (s, 1H), 9.43 (s, 1H), 7.71 (d, J = 7.9 Hz, 1H), 7.51 (s, 1H), 7.43 (d, J = 8.0 Hz, 1H), 7.38 (t, J = 8.0 Hz, 1H), 7.21-7.05 (m, 3H), 5.11 (dd, J = 13.3, 5.1 Hz, 1H), 4.50-4.42 (m, 3H), 4.32 (d, J = 17.4 Hz, 1H), 3.78 (s, 3H), 2.96-2.87 (m, 1H), 2.65-2.55 (m, 1H), 2.43-2.38 (m, 1H), 2.05-1.94 (m, 1H). Molecular Formula: C24H21N3O5, theoretical molecular weight: 431.45; LC-MS(ESI):m/z = [M+H]+: 432.45. Embodiment 47: compound 47 synthesis 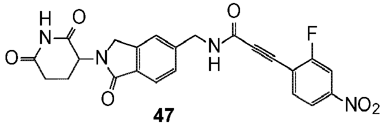 ¹H NMR (400 MHz, DMSO-*d6*): 10.97 (s, 1H), 9.18 (s, 1H), 7.69 (d, J = 7.9 Hz, 1H), 7.46 (s, 1H), 7.40 (d, J = 7.8 Hz, 1H), 7.18 (t, J = 8.4 Hz, 1H), 6.41-6.32 (m, 2H), 5.10 (dd, J = 13.3, 5.1 Hz, 1H), 4.53-4.37 (m, 3H), 4.30 (d, J = 17.3 Hz, 1H), 2.93-2.85 (m, 1H), 2.62-2.60 (m, 1H), 2.46-2.39 (m, 1H), 2.04-1.97 (m, 1H). Molecular Formula: C23H17FN4O6, theoretical molecular weight: 464.41; LC-MS(ESI):m/z = [M+H]+: 464.41. Embodiment 48: compound 48 synthesis 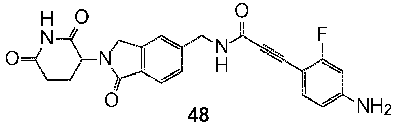 ¹H NMR (400 MHz, DMSO-*d6*): 10.99 (s, 1H), 9.20 (s, 1H), 7.70 (d, J = 7.9 Hz, 1H), 7.49 (s, 1H), 7.41 (d, J = 7.8 Hz, 1H), 7.21 (t, J = 8.4 Hz, 1H), 6.43-6.33 (m, 2H), 6.17 (s, 2H), 5.11 (dd, J = 13.3, 5.1 Hz, 1H), 4.54-4.38 (m, 3H), 4.31 (d, J = 17.3 Hz, 1H), 2.95-2.86 (m, 1H), 2.63-2.61 (m, 1H), 2.43-2.38 (m, 1H), 2.03-1.96 (m, 1H). Molecular Formula: C23H19FN4O4, theoretical molecular weight: 434.43; LC-MS(ESI):m/z=[M+H]+: 435.43. Embodiment 49: compound 49 synthesis 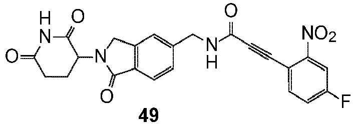 ¹H NMR (400 MHz, DMSO-*d6*): 10.99 (s, 1H), 9.53 (s, 1H), 8.20 (dd, J = 8.7, 2.6 Hz, 1H), 7.97 (dd, J = 8.7, 5.5 Hz, 1H), 7.82-7.75 (m, 1H), 7.71 (t, J = 6.8 Hz, 1H), 7.52 (s, 1H), 7.44 (d, J = 8.0 Hz, 1H), 5.11 (dd, J = 13.3, 5.0 Hz, 1H), 4.49-4.43 (m, 3H), 4.36-4.27 (m, 1H), 2.95-2.84 (m, 1H), 2.65 -2.55 (m, 1H), 2.43-2.38 (m, 1H), 2.02-1.99 (m, 1H). Molecular Formula: C23H17FN4O6, theoretical molecular weight: 464.41; LC-MS(ESI):m/z= [M+H]+: 465.41.

### Embodiment 50: compound 50 synthesis

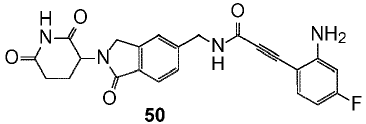

¹H NMR (400 MHz, DMSO-*d6*) : 10.98 (s, 1H), 9.27 (s, 1H), 7.72 (d, J = 7.8 Hz, 1H), 7.53 (s, 1H), 7.45 (d, J = 7.8 Hz, 1H), 7.30 (dd, J = 8.6, 6.6 Hz, 1H), 6.46 (dd, J = 11.7, 2.5 Hz, 1H), 6.34 (td, J = 8.6, 2.5 Hz, 1H), 6.11 (s, 2H), 5.11 (dd, J = 13.3, 5.1 Hz, 1H), 4.55-4.39 (m, 3H), 4.32 (d, J = 17.4 Hz, 1H), 2.97-2.85 (m, 1H), 2.63-2.57 (m, 1H), 2.43-2.38 (m, 1H), 2.05-1.95 (m, 1H). Molecular Formula: C23H19FN4O4, theoretical molecular weight: 434.43; LC-MS(ESI):m/z = [M+H]+: 435.43.

### Embodiment 51: compound 51 synthesis

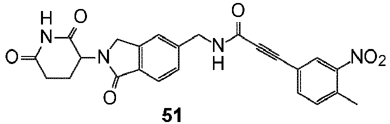

¹H NMR (400 MHz, DMSO-*d6*) : 11.00 (s, 1H), 9.52 (s, 1H), 8.18 (d, J = 1.8 Hz, 1H), 7.83 (dd, J = 7.8, 1.8 Hz, 1H), 7.72 (d, J = 7.8 Hz, 1H), 7.61 (d, J = 8.1 Hz, 1H), 7.52 (s, 1H), 7.44 (d, J = 7.9 Hz, 1H), 5.12 (dd, J = 13.3, 5.0 Hz, 1H), 4.59-4.38 (m, 3H), 4.32 (d, J = 17.4 Hz, 1H), 2.95-2.86 (m, 1H), 2.63-2.57 (m, 1H), 2.55 (s, 3H), 2.43-2.38 (m, 1H), 2.05-1.95 (m, 1H). Molecular Formula: C24H20N4O6, theoretical molecular weight: 460.45; LC-MS(ESI):m/z = [M+H]+: 461.45.

### Embodiment 52: compound 52 synthesis

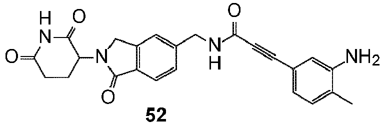

¹H NMR (400 MHz, DMSO-*d6*) : 10.99 (s, 1H), 9.33 (s, 1H), 7.70 (d, J = 7.8 Hz, 1H), 7.50 (s, 1H), 7.42 (d, J = 7.8 Hz, 1H), 6.98 (d, J = 7.7 Hz, 1H), 6.77 (d, J = 1.7 Hz, 1H), 6.66 (dd, J = 7.5, 1.7 Hz, 1H), 5.20 (s, 2H), 5.12 (dd, J = 13.3, 5.0 Hz, 1H), 4.53-4.39 (m, 3H), 4.32 (d, J = 17.3 Hz, 1H), 2.95-2.86 (m, 1H), 2.63-2.57 (m, 1H), 2.55 (s, 3H), 2.43-2.38 (m, 1H), 2.07 (s, 3H), 2.04-1.95 (m, 1H). Molecular Formula: C24H22N4O4, theoretical molecular weight: 430.46; LC-MS(ESI):m/z= [M+H]+: 431.46.

### Embodiment 53: compound 53 synthesis

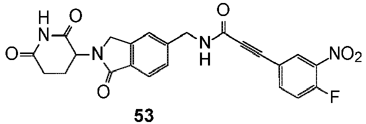

¹H NMR (400 MHz, DMSO-*d6*) : 10.99 (s, 1H), 9.53 (s, 1H), 8.36 (dd, J = 7.1, 2.2 Hz, 1H), 8.05 - 8.01 (m, 1H), 7.74-7.69 (m, 2H), 7.51 (d, J = 6.4 Hz, 1H), 7.44 (d, J = 7.8 Hz, 1H), 5.11 (dd, J = 13.3, 5.0 Hz, 1H), 4.55-4.39 (m, 3H), 4.32 (d, J = 17.4 Hz, 1H), 2.97-2.85 (m, 1H), 2.64-2.56 (m, 1H), 2.43-2.35 (m, 1H), 2.07-1.96 (m, 1H). Molecular Formula: C23H17FN4O6, theoretical molecular weight: 464.41; LC-MS(ESI):m/z=[M+H]+: 465.41.

### Embodiment 54: compound 54 synthesis

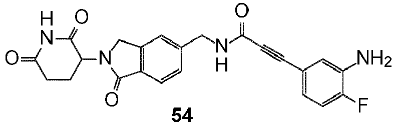

¹H NMR (400 MHz, DMSO-*d6*) : 10.99 (s, 1H), 9.36 (s, 1H), 7.70 (d, J = 7.8 Hz, 1H), 7.50 (s, 1H), 7.42 (d, J = 7.9 Hz, 1H), 7.06 (dd, J = 11.5, 8.3 Hz, 1H), 6.93 (dd, J = 8.6, 2.2 Hz, 1H), 6.75-6.71 (m, 1H), 5.45 (s, 2H), 5.11 (dd, J = 13.3, 5.2 Hz, 1H), 4.52-4.40 (m, 3H), 4.32 (d, J = 17.4 Hz, 1H), 2.95-2.87 (m, 1H), 2.64-2.61 (m, 1H), 2.43-2.35 (m, 1H), 2.04-1.95 (m, 1H). Molecular Formula: C23H19FN4O4, theoretical molecular weight: 434.43; LC-MS(ESI):m/z = [M+H]+: 435.43.

### Embodiment 55: compound 55 synthesis

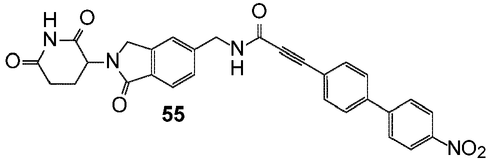

¹H NMR (400 MHz, DMSO-*d6*) : 10.95 (s, 1H), 9.38 (s, 1H), 7.70 (d, J = 7.9 Hz, 1H), 7.62 (d, J = 8.5 Hz, 2H), 7.58-7.49 (m, 3H), 7.50-7.39 (m, 3H), 6.63 (d, J = 8.6 Hz, 2H), 5.10 (dd, J = 13.2, 5.0 Hz, 1H), 4.48-4.42 (m, 3H), 4.31 (d, J = 17.4 Hz, 1H), 2.95-2.90 (m, 1H), 2.65-2.61 (m, 1H), 2.43-2.36 (m, 1H), 2.04-1.97 (m, 1H). Molecular Formula: C29H22N4O6, theoretical molecular weight: 522.52; LC-MS(ESI):m/z = [M+H]+: 523.52.

### Embodiment 56: compound 56 synthesis

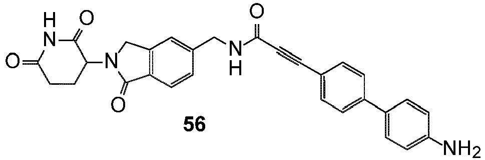

¹H NMR (400 MHz, DMSO-*d6*) : 10.97 (s, 1H), 9.39 (s, 1H), 7.71 (d, J = 7.9 Hz, 1H), 7.64 (d, J = 8.5 Hz, 2H), 7.60-7.49 (m, 3H), 7.50-7.38 (m, 3H), 6.64 (d, J = 8.6 Hz, 2H), 5.39 (s, 2H), 5.11 (dd, J = 13.2, 5.0 Hz, 1H), 4.50-4.42 (m, 3H), 4.32 (d, J = 17.4 Hz, 1H), 2.94-2.90 (m, 1H), 2.63-2.61 (m, 1H), 2.43-2.35 (m, 1H), 2.04-1.95 (m, 1H). Molecular Formula: C29H24N4O4, theoretical molecular weight: 492.54; LC-MS(ESI):m/z=[M+H]+: 493.54.

### Embodiment 57: compound 57 synthesis

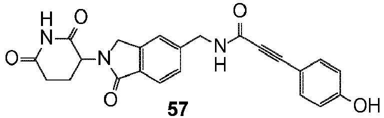

¹H NMR (400 MHz, DMSO-*d6*) : 11.00 (s, 1H), 10.18 (s, 1H), 9.28 (s, 1H), 7.70 (d, J = 7.9 Hz, 1H), 7.50 (s, 1H), 7.43-7.39 (m, 3H), 6.86-6.76 (m, 2H), 5.11 (dd, J = 13.3, 5.1 Hz, 1H), 4.51-4.38 (m, 3H), 4.31 (d, J = 17.4 Hz, 1H), 2.97-2.86 (m, 1H), 2.62-2.57 (m, 1H), 2.43-2.35 (m, 1H), 2.06-1.96 (m, 1H). Molecular Formula: C23H19N3O5, theoretical molecular weight: 417.42; LC-MS(ESI):m/z = [M+H]+: 418.42.

### Embodiment 58: compound 58 synthesis

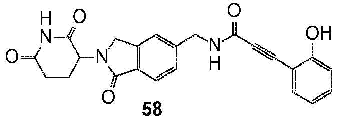

¹H NMR (400 MHz, DMSO-*d6*) : 10.99 (s, 1H), 10.27 (s, 1H), 9.29 (s, 1H), 7.71 (d, J = 7.8 Hz, 1H), 7.51 (s, 1H), 7.47-7.35 (m, 2H), 7.32-7.27 (m, 1H), 6.93 (d, J = 8.2 Hz, 1H), 6.88-6.80 (m, 1H), 5.11 (dd, J = 13.3, 5.1 Hz, 1H), 4.53-4.37 (m, 3H), 4.32 (d, J = 17.4 Hz, 1H), 2.96-2.86 (m, 1H), 2.64-2.57 (m, 1H), 2.43-2.35 (m, 1H), 2.04-1.96 (m, 1H). Molecular Formula: C23H19N3O5, theoretical molecular weight: 417.42; LC-MS(ESI):m/z = [M+H]+: 418.42.

### Embodiment 59: compound 59 synthesis

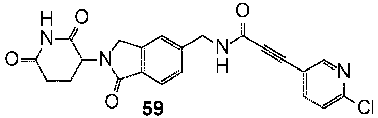

¹H NMR (400 MHz, DMSO-*d6*) : 11.00 (s, 1H), 9.56 (s, 1H), 8.64 (dd, J = 2.4, 0.8 Hz, 1H), 8.08 (dd, J = 8.4, 2.4 Hz, 1H), 7.71 (d, J = 7.8 Hz, 1H), 7.66 (dd, J = 8.4, 0.8 Hz, 1H), 7.51 (s, 1H), 7.47 -7.37 (m, 1H), 5.11 (dd, J = 13.3, 5.1 Hz, 1H), 4.54-4.40 (m, 3H), 4.32 (d, J = 17.4 Hz, 1H), 2.96-2.87 (m, 1H), 2.65-2.55 (m, 1H), 2.43-2.35 (m, 1H), 2.04-1.96 (m, 1H). Molecular Formula: C22H17C1N4O4, theoretical molecular weight: 436.85; LC-MS(ESI):m/z = [M+H]+: 437.85.

### Embodiment 60: compound 60 synthesis

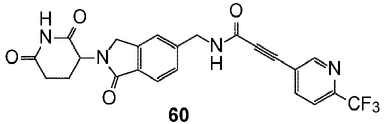

¹H NMR (400 MHz, DMSO-*d6*) : 11.00 (s, 1H), 9.65 (s, 1H), 8.97 (d, J = 2.0 Hz, 1H), 8.33 (dd, J = 8.4, 2.1 Hz, 1H), 8.03 (d, J = 8.4 Hz, 1H), 7.72 (d, J = 7.9 Hz, 1H), 7.53 (s, 1H), 7.44 (d, J = 7.9 Hz, 1H), 5.12 (dd, J = 13.3, 5.1 Hz, 1H), 4.54-4.41 (m, 3H), 4.33 (d, J = 17.4 Hz, 1H), 2.96-2.86(m, 1H), 2.64-2.57 (m, 1H), 2.43-2.35 (m, 1H), 2.05-1.95 (m, 1H). Molecular Formula: C23H17F3N4O4, theoretical molecular weight: 470.41; LC-MS(ESI):m/z = [M+H]+: 471.41.

### Embodiment 61: compound 61 synthesis

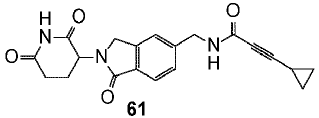

¹H NMR (400 MHz, DMSO-*d6*) : 10.99 (s, 1H), 9.06 (s, 1H), 7.68 (d, J = 7.8 Hz, 1H), 7.44 (s, 1H), 7.37 (d, J = 7.6 Hz, 1H), 5.11 (dd, J = 13.3, 5.1 Hz, 1H), 4.44 (d, J = 17.4 Hz, 1H), 4.36 (d, J = 6.2 Hz, 2H), 4.30 (d, J = 17.3 Hz, 1H), 2.96-2.87 (m, 1H), 2.64-2.55 (m, 1H), 2.43-2.35 (m, 1H), 2.03-1.96 (m, 1H), 1.51-1.45 (m, 1H), 0.96-0.86 (m, 2H), 0.81-0.70 (m, 2H). Molecular Formula: C20H19N3O4, theoretical molecular weight: 365.39; LC-MS(ESI):m/z= [M+H]+: 366.39.

### Embodiment 62: compound 62 synthesis

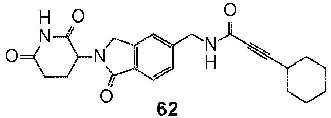

¹H NMR (400 MHz, DMSO-*d6*): 10.99 (s, 1H), 9.10 (s, 1H), 7.69 (d, J = 7.8 Hz, 1H), 7.46 (s, 1H), 7.38 (d, J = 7.9 Hz, 1H), 5.11 (dd, J = 13.3, 5.1 Hz, 1H), 4.44 (d, J = 17.4 Hz, 1H), 4.37 (d, J = 6.1 Hz, 2H), 4.30 (d, J = 17.3 Hz, 1H), 2.95-2.84 (m, 1H), 2.62-2.59 (m, 2H), 2.38 (dd, J = 13.2, 4.5 Hz, 1H), 2.06-1.94 (m, 1H), 1.81-1.72 (m, 2H), 1.66-1.63 (m, 2H), 1.56-1.35 (m, 4H), 1.35-1.08 (m, 4H). Molecular Formula: C23H25N3O4, theoretical molecular weight: 407.47; LC-MS(ESI):m/z= [M+H]+: 408.47.

### Embodiment 63: compound 63 synthesis

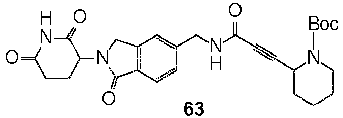

¹H NMR (400 MHz, DMSO-*d6*): 10.99 (s, 1H), 9.28 (s, 1H), 7.69 (d, J = 7.8 Hz, 1H), 7.48 (s, 1H), 7.43-7.36 (m, 1H), 5.11 (dd, J = 13.1, 4.9 Hz, 2H), 4.53-4.36 (m, 3H), 4.31 (d, J = 17.4 Hz, 1H), 3.83 (d, J = 13.3 Hz, 1H), 2.97-2.87 (m, 2H), 2.62-2.58(m, 1H), 2.43-2.38(m, 1H), 2.34-2.32 (m, 1H), 2.05-1.95 (m, 1H), 1.74 (s, 1H), 1.63 (d, J = 6.6 Hz, 4H), 1.40 (s, 9H). Molecular Formula: C27H32N4O6, theoretical molecular weight: 508.58; LC-MS(ESI):m/z=[M+H]+: 509.58.

### Embodiment 64: compound 64 synthesis

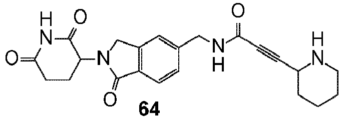

¹H NMR (400 MHz, DMSO-*d6*): 10.98 (s, 1H), 9.29 (s, 1H), 7.68 (d, J = 7.8 Hz, 1H), 7.47 (s, 1H), 7.42-7.36 (m, 1H), 5.11 (dd, J = 13.1, 4.9 Hz, 2H), 4.53-4.36 (m, 3H), 4.31 (d, J = 17.4 Hz, 1H), 3.83 (d, J = 13.3 Hz, 1H), 2.98-2.89 (m, 2H), 2.72 (s, 1H), 2.63-2.58 (m, 1H), 2.43-2.39 (m, 1H), 2.34-2.36 (m, 1H), 2.06-1.97 (m, 1H), 1.76 (s, 1H), 1.65 (d, J = 6.6 Hz, 4H). Molecular Formula: C22H24N4O4, theoretical molecular weight: 408.46; LC-MS(ESI):m/z=[M+H]+: 409.46.

### Embodiment 65: compound 65 synthesis

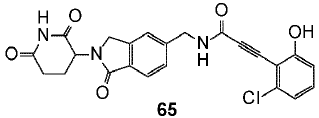

¹H NMR (400 MHz, DMSO-*d6*): 10.98 (s, 1H), 9.51 (s, 1H), 7.71-7.68 (m, 2H), 7.66 (d, J = 1.0 Hz, 1H), 7.57 (s, 1H), 7.53-7.42 (m, 3H), 5.10 (dd, J = 13.3, 5.1 Hz, 1H), 4.61 (d, J = 6.1 Hz, 2H), 4.45 (d, J = 17.4 Hz, 1H), 4.31 (d, J = 17.3 Hz, 1H), 2.95-2.86 (m, 1H), 2.62-2.56 (m, 1H), 2.43-2.38 (m, 1H), 2.03-1.95 (m, 1H). Molecular Formula: C23H18C1N3O5, theoretical molecular weight: 451.86; LC-MS(ESI):m/z = [M+H]+: 452.86.

### Embodiment 66: compound 66 synthesis

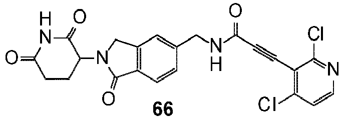

¹H NMR (400 MHz, DMSO-*d6*): 10.99 (s, 1H), 9.65 (s, 1H), 8.48 (d, J = 5.4 Hz, 1H), 7.81 (d, J = 5.4 Hz, 1H), 7.72 (d, J = 7.8 Hz, 1H), 7.53 (s, 1H), 7.45 (d, J = 7.5 Hz, 1H), 5.11 (dd, J = 13.4, 5.1 Hz, 1H), 4.52-4.42 (m, 3H), 4.32 (dd, J = 17.4, 6.5 Hz, 1H), 2.96-2.89 (m, 1H), 2.64-2.60 (m, 1H), 2.43-2.38 (m, 1H), 2.04-1.98 (m, 1H). Molecular Formula: C22H16C12N4O4, theoretical molecular weight: 471.29; LC-MS(ESI):m/z = [M+H]+: 472.29.

### Embodiment 67: compound 67 synthesis

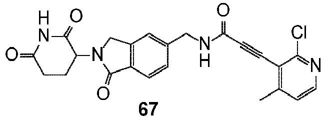

¹H NMR (400 MHz, DMSO-*d6*): 10.99 (s, 1H), 9.54 (s, 1H), 8.35 (d, J = 5.0 Hz, 1H), 7.72 (d, J = 7.9 Hz, 1H), 7.53 (s, 1H), 7.49-7.38 (m, 2H), 5.11 (dd, J = 13.3, 5.1 Hz, 1H), 4.55-4.41 (m, 3H), 4.33 (d, J = 17.4 Hz, 1H), 2.96-2.89 (m, 1H), 2.63-2.56 (m, 1H), 2.43-2.38 (m, 1H), 2.04-1.97 (m, 1H). Molecular Formula: C23H19C1N4O4, theoretical molecular weight:450.88; LC-MS(ESI):m/z= [M+H]+: 451.88.

### Embodiment 68: compound 68 synthesis

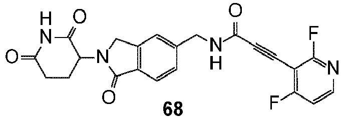

¹H NMR (400 MHz, DMSO-*d6*): 11.00 (s, 1H), 9.68 (s, 1H), 8.43 (dd, J = 8.2, 5.8 Hz, 1H), 7.71 (dd, J = 7.8, 3.6 Hz, 1H), 7.57 (dd, J = 8.2, 5.9 Hz, 1H), 7.51 (d, J = 7.0 Hz, 1H), 7.43 (d, J = 8.1 Hz, 1H), 5.11 (dd, J = 13.2, 5.0 Hz, 1H), 4.54-4.39 (m, 3H), 4.31 (dd, J = 17.4, 6.2 Hz, 1H), 2.98-2.85 (m, 1H), 2.63-2.56 (m, 1H), 2.45-2.34 (m, 1H), 2.04-1.96 (m, 1H). Molecular Formula: C22H16F2N4O4, theoretical molecular weight: 438.39; LC-MS(ESI):m/z = [M+H]+: 439.39.

### Embodiment 69: compound 69 synthesis

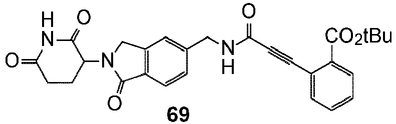

¹H NMR (400 MHz, DMSO-*d6*): 10.99 (s, 1H), 9.41 (s, 1H), 7.90 - 7.83 (m, 1H), 7.74-7.56 (m, 4H), 7.51 (s, 1H), 7.43 (dd, J = 7.9, 1.5 Hz, 1H), 5.11 (dd, J = 13.2, 5.1 Hz, 1H), 4.56-4.38 (m, 3H), 4.32 (d, J = 17.3 Hz, 1H), 2.96-2.87 (m, 1H), 2.65-2.58 (m, 1H), 2.44-2.36 (m, 1H), 2.07-1.95 (m, 1H), 1.54 (s, 9H). Molecular Formula: C28H27N3O6, theoretical molecular weight: 501.54; LC-MS(ESI):m/z = [M+H]+: 502.54.

### Embodiment 70: compound 70 synthesis

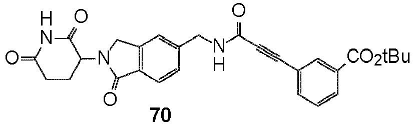

¹H NMR (400 MHz, DMSO-*d6*): 10.99 (s, 1H), 9.49 (s, 1H), 8.06-7.96 (m, 2H), 7.84 (dt, J = 7.7, 1.5 Hz, 1H), 7.71 (d, J = 7.7 Hz, 1H), 7.61 (t, J = 7.8 Hz, 1H), 7.52 (s, 1H), 7.44 (dd, J = 8.0, 1.5 Hz, 1H), 5.11 (dd, J = 13.3, 5.1 Hz, 1H), 4.52-4.41 (m, 3H), 4.32 (d, J = 17.4 Hz, 1H), 2.97-2.85 (m, 1H), 2.65-2.58 (m, 1H), 2.43-2.38 (m, 1H), 2.05-1.98 (m, 1H), 1.55 (s, 9H). Molecular Formula: C28H27N3O6, theoretical molecular weight: 501.54; LC-MS(ESI):m/z = [M+H]+: 502.54.

### Embodiment 71: compound 71 synthesis

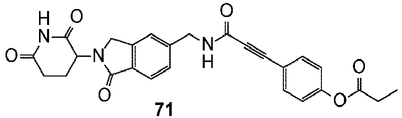

¹H NMR (400 MHz, DMSO-*d6*): 11.00 (s, 1H), 9.44 (s, 1H), 7.71 (d, J = 7.8 Hz, 1H), 7.68-7.54 (m, 2H), 7.52 (s, 1H), 7.43 (d, J = 7.8 Hz, 1H), 7.31-7.18 (m, 2H), 5.11 (dd, J = 13.3, 5.1 Hz, 1H), 4.53-4.39 (m, 3H), 4.32 (d, J = 17.4 Hz, 1H), 2.97-2.86 (m, 1H), 2.86-2.78 (m, 1H), 2.65-2.55 (m, 1H), 2.43-2.38 (m, 1H), 2.04-1.96 (m, 1H), 1.24 (s, 3H), 1.23 (s, 3H). Molecular Formula: C27H25N3O6, theoretical molecular weight: 487.51; LC-MS(ESI):m/z=[M+H]+: 488.51.

### Embodiment 72: compound 72 synthesis

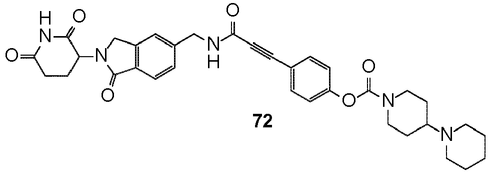

¹H NMR (400 MHz, DMSO-*d6*): 11.00 (s, 1H), 9.44 (s, 1H), 7.71 (d, J = 7.8 Hz, 1H), 7.66-7.59 (m, 2H), 7.52 (s, 1H), 7.43 (d, J = 7.8 Hz, 1H), 7.28-7.20 (m, 2H), 5.11 (dd, J = 13.3, 5.1 Hz, 1H), 4.55-4.39 (m, 3H), 4.32 (d, J = 17.4 Hz, 1H), 4.25-4.10 (m, 2H), 3.39 (d, J = 12.1 Hz, 3H), 3.09-3.0(m, 1H), 2.95-2.87 (m, 4H), 2.67-2.58 (m, 1H), 2.43-2.38 (m, 1H), 2.14 (d, J = 12.1 Hz, 2H), 2.05-1.95 (m, 1H), 1.83-1.81 (m, 4H), 1.71 (d, J = 14.2 Hz, 3H), 1.40 (s, 1H). Molecular Formula: C34H37N5O6, theoretical molecular weight: 611.7; LC-MS(ESI):m/z = [M+H]+: 612.70.

### Embodiment 73: compound 73 synthesis

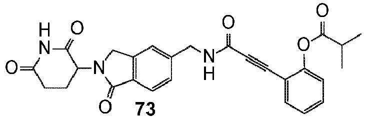

¹H NMR (400 MHz, DMSO-*d6*): 10.99 (s, 1H), 9.43 (s, 1H), 7.70 (d, J = 7.8 Hz, 1H), 7.64 (dd, J = 7.7, 1.7 Hz, 1H), 7.57 (td, J = 7.9, 1.7 Hz, 1H), 7.49 (s, 1H), 7.44-7.34 (m, 2H), 7.28 (dd, J = 8.2, 1.2 Hz, 1H), 5.12 (dd, J = 13.3, 5.1 Hz, 1H), 4.50-4.41 (m, 3H), 4.32 (d, J = 17.4 Hz, 1H), 2.97-2.89 (m, 1H), 2.89-2.82 (m, 1H), 2.63-2.57 (m, 1H), 2.43-2.38 (m, 1H), 2.03-1.96 (m, 1H), 1.26 (s, 3H), 1.25 (s, 3H). Molecular Formula: C27H25N3O6, theoretical molecular weight: 487.51; LC-MS(ESI):m/z = [M+H]+: 488.51.

### Embodiment 74: compound 74 synthesis

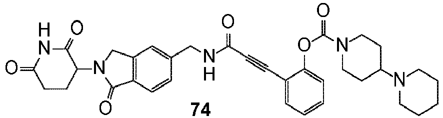

¹H NMR (400 MHz, DMSO-*d6*): 10.99 (s, 1H), 9.44 (s, 1H), 7.71 (d, J = 7.7 Hz, 1H), 7.60 (dd, J = 7.8, 1.7 Hz, 1H), 7.56-7.47 (m, 2H), 7.42 (d, J = 8.1 Hz, 1H), 7.35-7.25 (m, 2H), 5.12 (dd, J = 13.3, 5.0 Hz, 1H), 4.55-4.38 (m, 3H), 4.32 (d, J = 17.3 Hz, 1H), 4.28-4.19 (m, 1H), 4.05-3.98 (m, 1H), 3.07-2.75 (m, 4H), 2.65-2.58 (m, 1H), 2.43-2.36 (m, 4H), 2.05-1.95 (m, 1H), 1.73-1.65 (m, 2H), 1.60-1.22 (m, 9H). Molecular Formula: C34H37N5O6, theoretical molecular weight:611.7; LC-MS(ESI):m/z = [M+H]+: 612.70.

### Embodiment 75: compound 75 synthesis

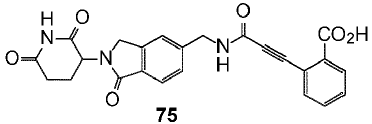

¹H NMR (400 MHz, DMSO-*d6*): 13.00 (s, 1H), 10.99 (s, 1H), 9.11 (d, J = 7.6 Hz, 1H), 8.00 (t, J = 8.2 Hz, 1H), 7.91 (t, J = 7.6 Hz, 1H), 7.79 (t, J = 7.2 Hz, 1H), 7.75-7.67 (m, 1H), 7.62-7.59 (m, 1H), 7.55 (s, 1H), 7.47 (d, J = 7.8 Hz, 1H), 5.11 (dd, J = 13.3, 5.1 Hz, 1H), 4.63-4.37 (m, 3H), 4.32 (d, J = 15.9 Hz, 1H), 2.94-2.87 (m, 1H), 2.63-2.57 (m, 1H), 2.43-2.37 (m, 1H), 2.03-1.96 (m, 1H). Molecular Formula: C24H19N3O6, theoretical molecular weight:445.43; LC-MS(ESI):m/z= [M+H]+: 446.43.

### Embodiment 76: compound 76 synthesis

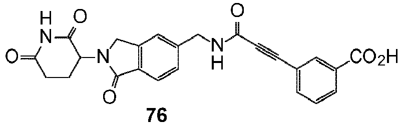

¹H NMR (400 MHz, DMSO-*d6*): 12.83 (s, 1H), 10.99 (s, 1H), 9.45 (s, 1H), 8.10 (s, 1H), 8.01 (d, J = 7.8 Hz, 1H), 7.71 (d, J = 7.8 Hz, 1H), 7.63 (d, J = 7.8 Hz, 1H), 7.52 (s, 1H), 7.50-7.40 (m, 2H), 5.11 (dd, J = 13.3, 5.1 Hz, 1H), 4.52-4.40 (m, 3H), 4.32 (d, J = 17.4 Hz, 1H), 2.94-2.86 (m, 1H), 2.63-2.57 (m, 1H), 2.41-2.37 (m, 1H), 2.05-1.95 (m, 1H). Molecular Formula: C24H19N3O6, theoretical molecular weight:445.43; LC-MS(ESI):m/z=[M+H]+: 446.43.

### Embodiment 77: compound 77 synthesis

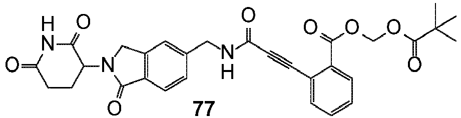

¹H NMR (400 MHz, DMSO-*d6*): 10.98 (s, 1H), 9.37 (s, 1H), 7.95 (dd, J = 8.1, 1.1 Hz, 1H), 7.81-7.69 (m, 3H), 7.65 (td, J = 7.5, 1.7 Hz, 1H), 7.53 (s, 1H), 7.51-7.41 (m, 1H) , 5.96 (s, 2H), 5.12 (dd, J = 13.3, 5.1 Hz, 1H), 4.54-4.39 (m, 3H), 4.33 (d, J = 17.3 Hz, 1H), 2.96-2.87 (m, 1H), 2.64-2.57 (m, 1H), 2.43-2.35 (m, 1H), 2.04-1.95 (m, 1H), 1.15 (s, 9H). Molecular Formula: C30H29N3O8, theoretical molecular weight: 559.58; LC-MS(ESI):m/z = [M+H]+: 560.58.

### Embodiment 78: compound 78 synthesis

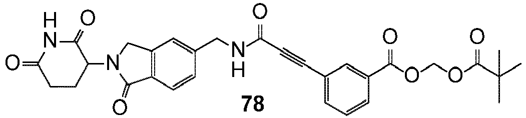

¹H NMR (400 MHz, DMSO-*d6*): 10.97 (s, 1H), 9.52 (s, 1H), 8.11 (d, J = 1.8 Hz, 1H), 8.08 (dt, J = 7.9, 1.5 Hz, 1H), 7.92 (dt, J = 7.7, 1.5 Hz, 1H), 7.72 (d, J = 7.9 Hz, 1H), 7.67 (t, J = 7.8 Hz, 1H), 7.53 (s, 1H), 7.48-7.41 (m, 1H), 5.97 (s, 2H), 5.12 (dd, J = 13.3, 5.1 Hz, 1H), 4.52-4.42 (m, 3H), 4.33 (d, J = 17.4 Hz, 1H), 2.95-2.85 (m, 1H), 2.67-2.55 (m, 1H), 2.43-2.35 (m, 1H), 2.04-1.97 (m, 1H), 1.16 (s, 9H). Molecular Formula: C30H29N3O8, theoretical molecular weight:559.58; LC-MS(ESI):m/z = [M+H]+: 560.58.

### Embodiment 79: compound 79 synthesis

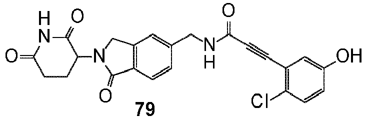

¹H NMR (400 MHz, DMSO-*d6*): 10.97 (s, 1H), 9.47 (s, 1H), 8.92 (s, 1H), 7.71 (d, J = 7.8 Hz, 1H), 7.52 (s, 1H), 7.46-7.40 (m, 1H), 7.38 (d, J = 8.8 Hz, 1H), 7.01 (d, J = 3.0 Hz, 1H), 6.91 (dd, J = 8.8, 3.0 Hz, 1H), 5.11 (dd, J = 13.3, 5.1 Hz, 1H), 4.51-4.42 (m, 3H), 4.32 (d, J = 17.3 Hz, 1H), 2.93-2.83 (m, 1H), 2.63-2.58 (m, 1H), 2.43-2.34 (m, 1H), 2.06-1.95 (m, 1H). Molecular Formula: C23H18ClN3O5, theoretical molecular weight: 451.86; LC-MS(ESI):m/z=[M+H]+: 452.86.

### Embodiment 80: compound 80 synthesis

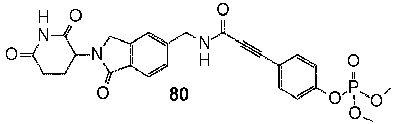

¹H NMR (400 MHz, DMSO-*d6*): 11.00 (s, 1H), 9.45 (s, 1H), 7.71 (d, J = 7.8 Hz, 1H), 7.68-7.59 (m, 2H), 7.51 (s, 1H), 7.43 (d, J = 7.4 Hz, 1H), 7.32 (d, J = 8.4 Hz, 2H), 5.11 ( dd, J = 13.3, 5.2 Hz, 1H), 4.50-4.42 (m, 3H), 4.32 (d, J = 17.4 Hz, 1H), 3.83 (s, 3H), 3.80 (s, 3H), 2.95-2.86 (m, 1H), 2.67-2.55 (m, 1H), 2.44-2.35 (m, 1H), 2.04-1.96 (m, 1H). Molecular Formula: C25H24N3O8P, theoretical molecular weight: 525.45; LC-MS(ESI):m/z = [M+H]+: 526.45.

### Embodiment 81: compound 81 synthesis

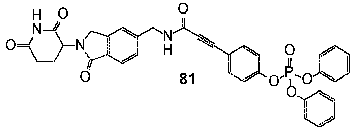

¹H NMR (400 MHz, DMSO-*d6*): 11.01 (s, 1H), 9.46 (s, 1H), 7.72 (d, J = 7.8 Hz, 1H), 7.67-7.59 (m, 2H), 7.51 (s, 1H), 7.43 (d, J = 7.4 Hz, 1H), 7.32 (d, J = 8.4 Hz, 2H), 7.27-7.22 (m, 4H), 7.21-7.18 (m, 6H), 5.11 (dd, J = 13.3, 5.2 Hz, 1H), 4.50-4.42 (m, 3H), 4.32 (d, J = 17.4 Hz, 1H), 2.95-2.86 (m, 1H), 2.67-2.55 (m, 1H), 2.44-2.35 (m, 1H), 2.04-1.96 (m, 1H). Molecular Formula: C35H28N3O8P, theoretical molecular weight: 649.6; LC-MS(ESI):m/z = [M+H]+: 650.60.

### Embodiment 82: compound 82 synthesis

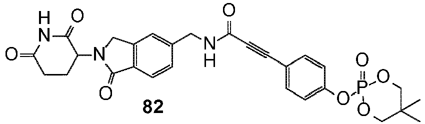

¹H NMR (400 MHz, DMSO-*d6*): 10.99 (s, 1H), 9.45 (s, 1H), 7.74-7.63 (m, 3H), 7.51 (s, 1H), 7.47-7.36 (m, 3H), 5.11 (dd, J = 13.3, 5.1 Hz, 1H), 4.53-4.40 (m, 3H), 4.38-4.26 (m, 3H), 4.13-4.07 (m, 1H), 4.07-4.00 (m, 1H), 2.95-2.84 (m, 1H), 2.67-2.55 (m, 1H), 2.44-2.35 (m, 1H), 2.04-1.96 (m, 1H), 1.20 (s, 3H), 0.86 (s, 3H). Molecular Formula: C28H28N3O8P, theoretical molecular weight: 565.52; LC-MS(ESI):m/z = [M+H]+: 566.52.

### Embodiment 83: compound 83 synthesis

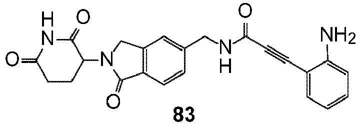

¹H NMR (400 MHz, DMSO-*d6*): 11.00 (s, 1H), 9.30 (s, 1H), 7.72 (d, J = 7.8 Hz, 1H), 7.53 (s, 1H), 7.48-7.40 (m, 1H), 7.24 (dd, J = 7.8, 1.6 Hz, 1H), 7.17-7.13 (m, 1H), 6.70 (d, J = 8.2 Hz, 1H), 6.52 (td, J = 7.6, 1.1 Hz, 1H), 5.80 (s, 2H), 5.11 (dd, J = 13.2, 5.1 Hz, 1H), 4.56-4.39 (m, 3H), 4.33 (d, J = 17.4 Hz, 1H), 2.96-2.87(m, 1H), 2.67-2.55 (m, 1H), 2.44-2.35 (m, 1H), 2.02-1.98 (m, 1H). Molecular Formula: C23H20N4O4, theoretical molecular weight: 416.44; LC-MS(ESI):m/z= [M+H]+: 417.44.

### Embodiment 84: compound 84 synthesis

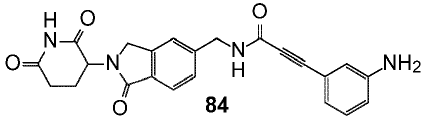

¹H NMR (400 MHz, DMSO-*d6*) : 11.01 (s, 1H), 9.30 (s, 1H), 7.73 (d, J = 7.8 Hz, 1H), 7.55 (s, 1H), 7.48-7.42 (m, 1H), 7.26 (dd, J = 7.8, 1.6 Hz, 1H), 7.15-7.13 (m, 1H), 6.68 (d, J = 8.2 Hz, 1H), 6.51 (td, J = 7.6, 1.1 Hz, 1H), 5.85 (s, 2H), 5.10 (dd, J = 13.2, 5.1 Hz, 1H), 4.58-4.39 (m, 3H), 4.31 (d, J = 17.4 Hz, 1H), 2.96-2.89 (m, 1H), 2.69-2.57 (m, 1H), 2.46-2.37 (m, 1H), 2.01-1.97 (m, 1H). Molecular Formula: C23H20N4O4, theoretical molecular weight: 416.44; LC-MS(ESI):m/z= [M+H]+: 417.44.

### Embodiment 85: compound 85 synthesis

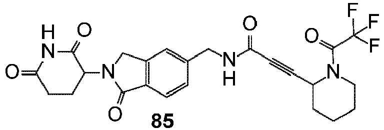

¹H NMR (400 MHz, DMSO-*d6*) : 10.99 (s, 1H), 9.31 (s, 1H), 7.70 (d, J = 7.8 Hz, 1H), 7.49 (s, 1H), 7.42-7.38 (m, 1H), 5.14 (dd, J = 13.1, 4.9 Hz, 2H), 4.53-4.39 (m, 3H), 4.33 (d, J = 17.4 Hz, 1H), 3.86 (d, J = 13.3 Hz, 1H), 3.02-2.89 (m, 2H), 2.65-2.59 (m, 1H), 2.46-2.41 (m, 1H), 2.38-2.39 (m, 1H), 2.10-1.98 (m, 1H), 1.77 (s, 1H), 1.66 (d, J = 6.6 Hz, 4H). Molecular Formula: C24H23F3N4O5, theoretical molecular weight: 504.47; LC-MS(ESI):m/z=[M+H]+: 505.47.

### Embodiment 86: compound 86 synthesis

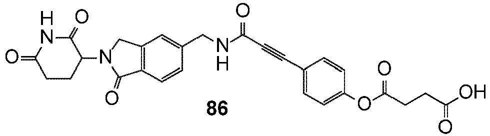

¹H NMR (400 MHz, DMSO-d6) : 11.00 (s, 1H), 10.18 (s, 1H), 9.28 (s, 1H), 7.70 (d, J = 7.8 Hz, 1H), 7.50 (s, 1H), 7.45-7.37 (m, 3H), 6.86-6.76 (m, 2H), 5.11 (dd, J = 13.4, 5.1 Hz, 1H), 4.50-4.41 (m, 3H), 4.32 (d, J = 17.4 Hz, 1H), 2.96-2.80 (m, 4H), 2.60 (d, J = 16.9 Hz, 1H), 2.43-2.35 (m, 1H), 2.33 (s, 1H), 2.03-1.96 (m, 1H). Molecular Formula: C27H23N3O8, theoretical molecular weight: 517.49; LC-MS(ESI):m/z = [M+H]+: 518.49.

### Embodiment 87: compound 87 synthesis

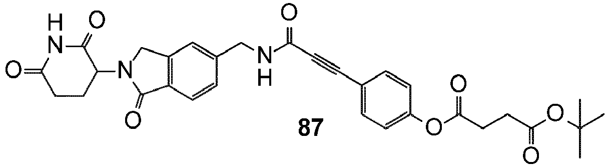

¹H NMR (400 MHz, DMSO-*d6*) : 11.00 (s, 1H), 9.44 (s, 1H), 7.74-7.60 (m, 3H), 7.52 (s, 1H), 7.43 (d, J = 8.1 Hz, 1H), 7.26-7.14 (m, 2H), 5.11 (dd, J = 13.3, 5.1 Hz, 1H), 4.52-4.42 (m, 3H), 4.32 (d, J = 17.4 Hz, 1H), 2.96-2.87 (m, 1H), 2.80 (dd, J = 7.6, 5.2 Hz, 2H), 2.63-2.55 (m, 3H), 2.43-2.35 (m, 1H), 2.04-1.96 (m, 1H), 1.40 (s, 9H). Molecular Formula: C31H31N3O8, theoretical molecular weight: 573.6; LC-MS(ESI):m/z = [M+H]+: 574.60.

### Embodiment 88: compound 88 synthesis

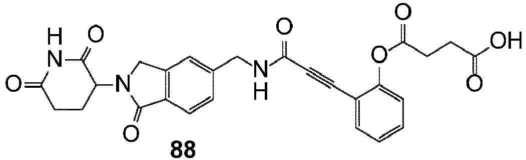

¹H NMR (400 MHz, DMSO-d6) : 12.21 (s, 1 H), 10.99 (s, 1H), 9.28 (s, 1H), 7.71 (d, J = 7.8 Hz, 1H), 7.51 (s, 1H), 7.46-7.36 (m, 2H), 7.32-7.27 (m, 1H), 6.93 (d, J = 8.1 Hz, 1H), 6.84 (t, J = 7.3 Hz, 1H), 5.11 (dd, J = 13.3, 5.0 Hz, 1H), 4.51-4.41 (m, 3H), 4.32 (d, J = 17.4 Hz, 1H), 2.93-2.86 (m, 1H), 2.67-2.56 (m, 2H), 2.44-2.38 (m, 1H), 2.36 (s, 3H), 2.04-1.96 (m, 1H). Molecular Formula: C27H23N3O8, theoretical molecular weight: 517.49; LC-MS(ESI):m/z=[M+H]+: 518.49.

### Embodiment 89: compound 89 synthesis

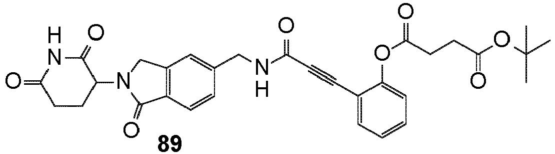

¹H NMR (400 MHz, DMSO-d6) : 10.99 (s, 1H), 9.37 (s, 1H), 7.71 (d, J = 7.8 Hz, 1H), 7.66 (dd, J = 7.7, 1.6 Hz, 1H), 7.58 (td, J = 7.8, 1.7 Hz, 1H), 7.51 (s, 1H), 7.45-7.33 (m, 2H), 7.29-7.22 (m, 1H), 5.11 (dd, J = 13.3, 5.1 Hz, 1H), 4.50-4.41 (m, 3H), 4.32 (d, J = 17.3 Hz, 1H), 2.97-2.89 (m, 1H), 2.89-2.83 (m, 2H), 2.60 (dd, J = 7.5, 5.8 Hz, 3H), 2.43-2.34 (m, 1H), 2.04-1.96 (m, 1H), 1.38 (s, 9H). Molecular Formula: C31H31N3O8, theoretical molecular weight: 573.6; LC-MS(ESI):m/z = [M+H]+: 574.60.

### Embodiment 90: compound 90 synthesis

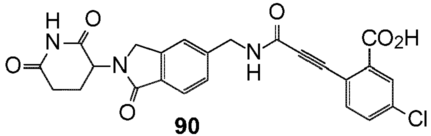

¹H NMR (400 MHz, DMSO-*d6*): 12.56 (s, 1 H), 10.99 (s, 1H), 8.77 (s, 1H), 8.19-8.03 (m, 1H), 7.97 (dd, J = 8.3, 2.0 Hz, 1H), 7.71 (dd, J = 7.8, 4.6 Hz, 1H), 7.64 (s, 1H), 7.55 (s, 1H), 7.47 (d, J = 8.0 Hz, 1H), 5.11 (dd, J = 13.2, 5.1 Hz, 1H), 4.61-4.32 (m, 4H), 2.95-2.85 (m, 1H), 2.64-2.57 (m, 1H), 2.43-2.35 (m, 1H), 2.05-1.92 (m, 1H). Molecular Formula: C24H18ClN3O6, theoretical molecular weight: 479.87; LC-MS(ESI):m/z=[M+H]+: 480.87.

### Embodiment 91: compound 91 synthesis

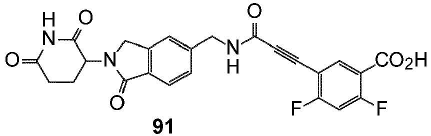

¹H NMR (400 MHz, DMSO-*d6*): 12.58 (s, 1 H), 10.99 (s, 1H), 8.77 (s, 1H), 8.19-8.08 (m, 1H), 7.97 (dd, J = 8.3, 2.0 Hz, 1H), 7.66 (s, 1H), 7.58 (s, 1H), 7.49 (t, J = 8.0 Hz, 1H), 5.10 (dd, J = 13.2, 5.1 Hz, 1H), 4.61-4.33 (m, 4H), 2.97-2.86 (m, 1H), 2.66-2.57 (m, 1H), 2.45-2.37 (m, 1H), 2.08-1.96 (m, 1H). Molecular Formula: C24H17F2N3O6, theoretical molecular weight: 481.41; LC-MS(ESI):m/z = [M+H]+: 482.41.

### Embodiment 92: compound 92 synthesis

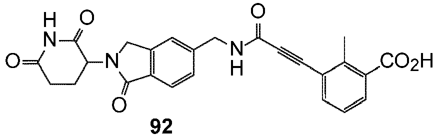

¹H NMR (400 MHz, DMSO-*d6*): 12.63 (s, 1H), 10.99 (s, 1H), 9.43 (s, 1H), 7.78 (d, J = 7.7 Hz, 1H), 7.71 (d, J = 7.8 Hz, 1H), 7.60 (d, J = 7.6 Hz, 1H), 7.53 (s, 1H), 7.44 (d, J = 7.8 Hz, 1H), 7.29 (t, J = 7.7 Hz, 1H), 5.11 (dd, J = 13.3, 5.1 Hz, 1H), 4.56-4.40 (m, 3H), 4.32 (d, J = 17.4 Hz, 1H), 2.96-2.85 (m, 1H), 2.66 (s, 3H), 2.63-2.57 (m, 1H), 2.42-2.38(m, 1H), 2.04-1.95 (m, 1H). Molecular Formula: C25H21N3O6, theoretical molecular weight: 459.46; LC-MS(ESI):m/z=[M+H]+: 460.46.

### Embodiment 93: compound 93 synthesis

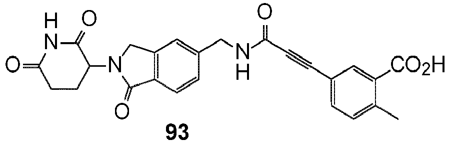

¹H NMR (400 MHz, DMSO-*d6*): 12.45 (s, 1H), 10.99 (s, 1H), 9.43 (s, 1H), 7.95 (d, J = 1.9 Hz, 1H), 7.71 (d, J = 7.8 Hz, 1H), 7.59 (d, J = 8.3 Hz, 1H), 7.52 (s, 1H), 7.43 (d, J = 7.9 Hz, 1H), 7.36 (d, J = 8.0 Hz, 1H), 5.11 (dd, J = 13.3, 5.1 Hz, 1H), 4.53-4.40 (m, 3H), 4.32 (d, J = 17.4 Hz, 1H), 2.97-2.87 (m, 1H), 2.67-2.58 (m, 1H), 2.55 (s, 3H), 2.42-2.38(m, 1H), 2.05-1.97 (m, 1H). Molecular Formula: C25H21N3O6, theoretical molecular weight: 459.46; LC-MS(ESI):m/z=[M+H]+: 460.46.

### Embodiment 94: compound 94 synthesis

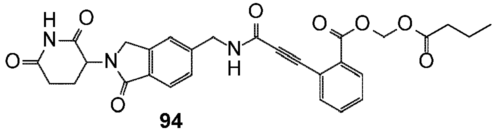

¹H NMR (400 MHz, DMSO-*d6*): 10.99 (s, 1H), 9.38 (s, 1H), 7.97 (d, J = 7.8 Hz, 1H), 7.82 - 7.69 (m, 3H), 7.65 (td, J = 7.5, 1.8 Hz, 1H), 7.53 (s, 1H), 7.45 (d, J = 7.8 Hz, 1H), 5.96 (s, 2H), 5.12 (dd, J = 13.3, 5.1 Hz, 1H), 4.57-4.39 (m, 3H), 4.33 (d, J = 17.3 Hz, 1H), 2.95-2.87 (m, 1H), 2.65-2.58 (m, 1H), 2.43-2.33 (m, 3H), 2.07-1.95 (m, 1H), 1.58-1.49 (m, 2H), 0.86 (t, J = 7.3 Hz, 3H). Molecular Formula: C29H27N3O8, theoretical molecular weight: 545.55; LC-MS(ESI):m/z = [M+H]+: 546.55.

### Embodiment 95: compound 95 synthesis

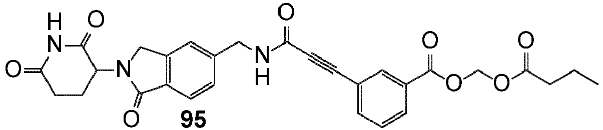

¹H NMR (400 MHz, DMSO-*d6*): 10.99 (s, 1H), 9.51 (s, 1H), 8.16-8.01 (m, 2H), 7.92 (dt, J = 7.8, 1.3 Hz, 1H), 7.72 (d, J = 7.9 Hz, 1H), 7.67 (t, J = 7.8 Hz, 1H), 7.52 (s, 1H), 7.44 (d, J = 7.8 Hz, 1H), 5.96 (s, 2H), 5.11 (dd, J = 13.3, 5.1 Hz, 1H), 4.54-4.40 (m, 3H), 4.32 (d, J = 17.4 Hz, 1H), 2.96-2.87 (m, 1H), 2.65-2.58 (m, 1H), 2.41-2.36 (m, 3H), 2.04-1.96 (m, 1H), 1.61-1.51 (m, 2H), 0.87 (t, J = 7.4 Hz, 3H). Molecular Formula: C29H27N3O8, theoretical molecular weight: 545.55; LC-MS(ESI):m/z=[M+H]+: 546.55.

### Embodiment 96: compound 96 synthesis

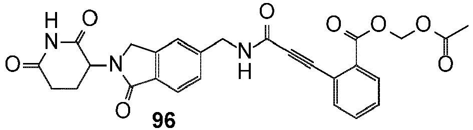

¹H NMR (400 MHz, DMSO-*d6*): 10.99 (s, 1H), 9.39 (s, 1H), 8.02-7.95 (m, 1H), 7.81-7.69 (m, 3H), 7.65 (td, J = 7.5, 1.7 Hz, 1H), 7.53 (s, 1H), 7.48-7.41 (m, 1H), 5.94 (s, 2H), 5.11 (dd, J = 13.3, 5.0 Hz, 1H), 4.53-4.41 (m, 3H), 4.33 (d, J = 17.4 Hz, 1H), 2.95-2.87 (m, 1H), 2.67-2.55 (m, 1H), 2.43-2.38 (m, 1H), 2.09 (s, 3H), 2.05-1.97 (m, 1H). Molecular Formula: C27H23N3O8, theoretical molecular weight: 517.49; LC-MS(ESI):m/z=[M+H]+: 518.49.

### Embodiment 97: compound 97 synthesis

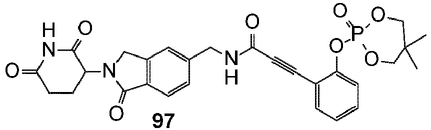

¹H NMR (400 MHz, DMSO - d6) δ 10.98 (s, 1H), 9.45 (s, 1H), 7.76-7.64 (m, 3H), 7.53 (s, 1H), 7.49-7.37 (m, 3H), 5.13 (dd, J = 13.3, 5.1 Hz, 1H), 4.53-4.48 (m, 3H), 4.37-4.29 (m, 3H), 4.13-4.08 (m, 1H), 4. 07-4.04 (m, 1H), 2.95-2.88 (m, 1H), 2.67-2.59 (m, 1H), 2.44-2.37 (m, 1H), 2.04-1.99 (m, 1H), 1.21 (s, 3H), 0.89 (s, 3H). Molecular Formula: C28H28N3O8P, theoretical molecular weight: 565.52; LC-MS(ESI):m/z=[M+H]+: 566.52.

### Embodiment 98: compound 98 synthesis

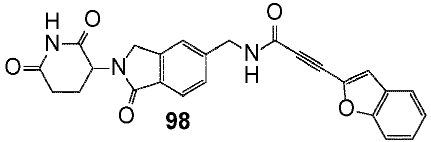

¹H NMR (400 MHz, DMSO-*d6*): 10.99 (s, 1H), 9.69 (s, 1H), 7.77-7.68 (m, 2H), 7.66-7.62 (m, 1H), 7.60 (d, J = 1.0 Hz, 1H), 7.53 (s, 1H), 7.50-7.46 (m, 1H), 7.44 (dd, J = 7.9, 1.4 Hz, 1H), 7.38-7.32 (m, 1H), 5.11 (dd, J = 13.3, 5.1 Hz, 1H), 4.57-4.41 (m, 3H), 4.33 (d, J = 17.4 Hz, 1H), 2.97-2.87 (m, 1H), 2.62-2.58 (m, 1H), 2.42-2.38 (m, 1H), 2.05-1.96 (m, 1H). Molecular Formula: C25H19N3O5, theoretical molecular weight: 441.44; LC-MS(ESI):m/z = [M+H]+: 442.44.

### Embodiment 99: compound 99 synthesis

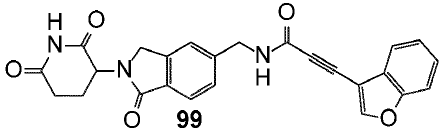

¹H NMR (400 MHz, DMSO-*d6*): 10.99 (s, 1H), 9.51 (s, 1H), 8.62 (s, 1H), 7.78 (dd, J = 7.5, 1.6 Hz, 1H), 7.72 (d, J = 7.9 Hz, 2H), 7.54 (s, 1H), 7.50-7.34 (m, 3H), 5.11 (dd, J = 13.3, 5.1 Hz, 1H), 4.57-4.39 (m, 3H), 4.33 (d, J = 17.3 Hz, 1H), 2.97-2.85 (m, 1H), 2.65-2.55 (m, 1H), 2.42-2.38 (m, 1H), 2.04-1.97 (m, 1H). Molecular Formula: C25H19N3O5, theoretical molecular weight: 441.44; LC-MS(ESI):m/z = [M+H]+: 442.44.

### Embodiment 100: compound 100 synthesis

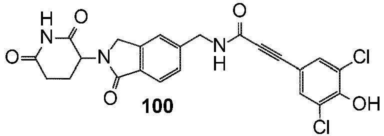

¹H NMR (400 MHz, DMSO-*d6*): 10.99 (s, 1H), 9.35 (s, 1H), 7.87 (s, 1H), 7.71 (d, J = 7.9 Hz, 1H), 7.69-7.61 (m, 1H), 7.60 (s, 1H), 7.49 (d, J = 13.1 Hz, 1H), 7.42 (d, J = 8.0 Hz, 1H), 5.11 (dd, J = 13.4, 5.1 Hz, 1H), 4.50-4.37 (m, 3H), 4.33 (d, J = 17.3 Hz, 1H), 2.97-2.86 (m, 1H), 2.63-2.56 (m, 1H), 2.42-2.36 (m, 1H), 2.05-1.97 (m, 1H). Molecular Formula: C23H17Cl2N3O5, theoretical molecular weight: 486.31; LC-MS(ESI):m/z=[M+H]+: 487.31.

### Embodiment 101: compound 101 synthesis

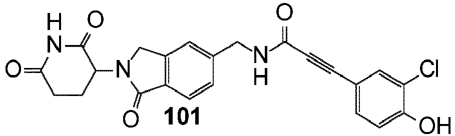

¹H NMR (400 MHz, DMSO-*d6*): 11.00 (d, J = 8.1 Hz, 2H), 9.32 (s, 1H), 7.92 (s, 1H), 7.70 (d, J = 7.8 Hz, 1H), 7.57 (d, J = 2.1 Hz, 1H), 7.50 (s, 1H), 7.46-7.33 (m, 2H), 7.02 (d, J = 8.4 Hz, 1H), 5.11 (dd, J = 13.2, 5.0 Hz, 1H), 4.51-4.38 (m, 3H), 4.32 (d, J = 17.3 Hz, 1H), 2.98-2.84 (m, 1H), 2.63-2.57 (m, 1H), 2.43-2.34 (m, 1H), 2.05-1.96 (m, 1H). Molecular Formula: C23H18ClN3O5, theoretical molecular weight: 451.86; LC-MS(ESI):m/z=[M+H]+: 452.86.

### Embodiment 102: compound 102 synthesis

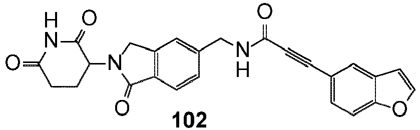

¹H NMR (400 MHz, DMSO-*d6*): 10.99 (s, 1H), 9.41 (s, 1H), 8.11 (d, J = 2.2 Hz, 1H), 7.94 (d, J = 1.7 Hz, 1H), 7.71 (dd, J = 8.2, 4.3 Hz, 2H), 7.55-7.49 (m, 2H), 7.44 (dd, J = 7.9, 1.5 Hz, 1H), 7.03 (dd, J = 2.2, 1.0 Hz, 1H), 5.11 (dd, J = 13.3, 5.1 Hz, 1H), 4.52-4.40 (m, 3H), 4.32 (d, J = 17.4 Hz, 1H), 2.97-2.85 (m, 1H), 2.65-2.56 (m, 1H), 2.43-2.34 (m, 1H), 2.05-1.96 (m, 1H). Molecular Formula: C25H19N3O5, theoretical molecular weight: 441.44; LC-MS(ESI):m/z=[M+H]+: 442.44.

### Embodiment 103: compound 103 synthesis

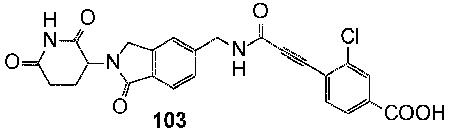

¹H NMR (400 MHz, DMSO-*d6*): 13.63 (s, 1H), 11.00 (s, 1H), 9.59 (s, 1H), 8.04 (d, J = 1.6 Hz, 1H), 7.93 (dd, J = 8.0, 1.6 Hz, 1H), 7.83 (d, J = 8.1 Hz, 1H), 7.72 (d, J = 7.8 Hz, 1H), 7.53 (s, 1H), 7.44 (d, J = 7.9 Hz, 1H), 5.11 (dd, J = 13.3, 5.1 Hz, 1H), 4.55-4.39 (m, 3H), 4.33 (d, J = 17.4 Hz, 1H), 2.97-2.86 (m, 1H), 2.64-2.55 (m, 1H), 2.47-2.32 (m, 1H), 2.06-1.95 (m, 1H). Molecular Formula: C24H18ClN3O6, theoretical molecular weight: 479.87; LC-MS(ESI):m/z=[M+H]+: 480.87.

### Embodiment 104: compound 104 synthesis

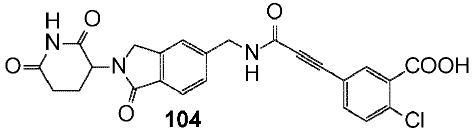

¹H NMR (400 MHz, DMSO-*d6*): 13.72 (s, 1H), 10.99 (s, 1H), 9.49 (s, 1H), 7.96 (d, J = 2.1 Hz, 1H), 7.76-7.67 (m, 2H), 7.65 (d, J = 8.4 Hz, 1H), 7.52 (s, 1H), 7.43 (d, J = 7.8 Hz, 1H), 5.11 (dd, J = 13.3, 5.1 Hz, 1H), 4.51-4.41 (m, 3H), 4.32 (d, J = 17.4 Hz, 1H), 2.96-2.85 (m, 1H), 2.64-2.56 (m, 2H), 2.43-2.35 (m, 1H), 2.03-1.96 (m, 1H). Molecular Formula: C24H18ClN3O6, theoretical molecular weight: 479.87; LC-MS(ESI):m/z=[M+H]+: 480.87.

### Embodiment 105: compound 105 synthesis

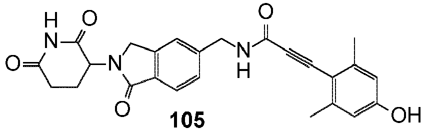

¹H NMR (400 MHz, DMSO-*d6*): 10.99 (s, 1H), 9.88 (s, 1H), 9.17 (s, 1H), 7.71 (d, J = 7.8 Hz, 1H), 7.52 (s, 1H), 7.44 (d, J = 7.9 Hz, 1H), 6.54 (s, 2H), 5.11 (dd, J = 13.3, 5.1 Hz, 1H), 4.50-4.41 (m, 3H), 4.32 (d, J = 17.4 Hz, 1H), 2.95-2.87 (m, 1H), 2.64-2.55 (m, 1H), 2.43-2.34 (m, 1H), 2.33 (s, 6H), 2.05-1.97 (m, 1H). Molecular Formula: C25H23N3O5, theoretical molecular weight: 445.48; LC-MS(ESI):m/z = [M+H]+: 446.48.

### Embodiment 106: compound 106 synthesis

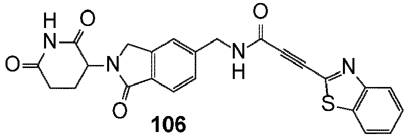

¹H NMR (400 MHz, DMSO-*d6*): 11.00 (s, 1H), 9.38 (s, 1H), 8.27-8.25 (m, 1H), 8.09-8.02 (m, 3H), 7.51-7.46 (m, 2H), 7.35-7.31 (m, 1H), 5.11 (dd, J= 13.2, 5.1 Hz, 1H), 4.52-4.41 (m, 3H), 4.32 (d, J = 17.4 Hz, 1H), 2.98-2.85 (m, 1H), 2.63-2.56 (m, 1H), 2.43- 2.35 (m, 1H), 2.04-1.96 (m, 1H). Molecular Formula: C24H18N4O4S, theoretical molecular weight: 458.49; LC-MS(ESI):m/z= [M+H]+: 459.49.

### Embodiment 107: compound 107 synthesis

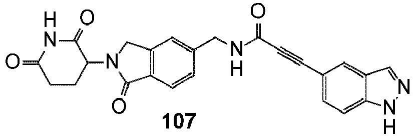

¹H NMR (400 MHz, DMSO-*d6*): 13.38 (s, 1H), 11.00 (s, 1H), 9.38 (s, 1H), 8.17 (t, J = 1.2 Hz, 1H), 8.09 (s, 1H), 7.71 (d, J = 7.8 Hz, 1H), 7.62 (d, J = 8.7 Hz, 1H), 7.55-7.41 (m, 3H), 5.11 (dd, J = 13.2, 5.1 Hz, 1H), 4.52-4.41 (m, 3H), 4.32 (d, J = 17.4 Hz, 1H), 2.98-2.85 (m, 1H), 2.63-2.56 (m, 1H), 2.43- 2.35 (m, 1H), 2.04-1.96 (m, 1H). Molecular Formula: C24H19N5O4, theoretical molecular weight:441.45; LC-MS(ESI):m/z = [M+H]+: 442.45.

### Embodiment 108: compound 108 synthesis

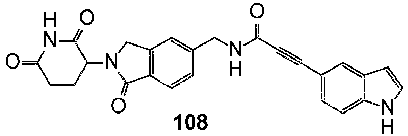

¹H NMR (400 MHz, DMSO-*d6*): 11.44 (s, 1H), 10.99 (s, 1H), 9.29 (s, 1H), 7.83 (d, J = 1.4 Hz, 1H), 7.71 (d, J = 7.8 Hz, 1H), 7.52 (s, 1H), 7.49-7.41 (m, 3H), 7.26 (dd, J = 8.3, 1.6 Hz, 1H), 6.50 (t, J = 2.6 Hz, 1H), 5.11 (dd, J = 13.3, 5.1 Hz, 1H), 4.51-4.42 (m, 3H), 4.32 (d, J = 17.4 Hz, 1H), 2.96-2.86 (m, 1H), 2.64-2.58 (m, 1H), 2.44-2.33 (m, 1H), 2.03-1.96 (m, 1H). Molecular Formula: C25H20N4O4, theoretical molecular weight: 440.46; LC-MS(ESI):m/z = [M+H]+: 441.46.

### Embodiment 109: compound 109 synthesis

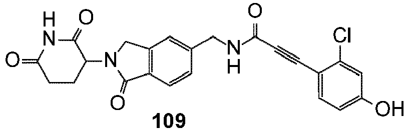

¹H NMR (400 MHz, DMSO-*d6*): 11.00 (s, 1H), 10.68 (s, 1H), 9.34 (s, 1H), 7.71 (d, J = 7.7 Hz, 1H), 7.54-7.47 (m, 2H), 7.45-7.38 (m, 1H), 7.05 (s, 1H), 6.95 (d, J = 2.3 Hz, 1H), 6.85-6.77 (m, 1H), 5.11 (dd, J = 13.3, 5.1 Hz, 1H), 4.50-4.41 (m, 3H), 4.32 (d, J = 17.4 Hz, 1H), 2.97-2.87 (m, 1H), 2.64-2.55 (m, 1H), 2.43-2.34 (m, 1H), 2.04-1.95 (m, 1H). Molecular Formula: C23H18ClN3O5, theoretical molecular weight: 451.86; LC-MS(ESI):m/z = [M+H]+: 452.86.

### Embodiment 110: compound 110 synthesis

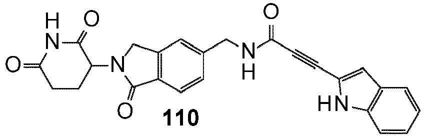

¹H NMR (400 MHz, DMSO-*d6*): 11.88 (s, 1H), 10.99 (s, 1H), 9.48 (s, 1H), 7.72 (d, J = 7.8 Hz, 1H), 7.58 (d, J = 8.0 Hz, 1H), 7.52 (s, 1H), 7.44 (d, J = 7.8 Hz, 1H), 7.36 (d, J = 8.3 Hz, 1H), 7.26-7.18 (m, 1H), 7.07 (t, J = 7.5 Hz, 1H), 6.95 (d, J = 2.0 Hz, 1H), 5.11 (dd, J = 13.3, 5.1 Hz, 1H), 4.57-4.40 (m, 3H), 4.33 (d, J = 17.4 Hz, 1H), 2.97-2.87 (m, 1H), 2.64-2.55 (m, 1H), 2.45-2.32 (m, 1H), 2.04-1.97 (m, 1H). Molecular Formula: C25H20N4O4, theoretical molecular weight: 440.46; LC-MS(ESI):m/z = [M+H]+: 441.46.

### Embodiment 111: compound 111 synthesis

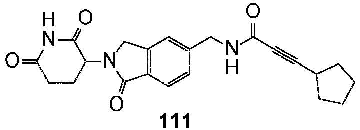

¹H NMR (400 MHz, DMSO-*d6*): 11.00 (s, 1H), 9.09 (s, 1H), 7.69 (d, J = 7.8 Hz, 1H), 7.45 (s, 1H), 7.37 (d, J = 7.8 Hz, 1H), 5.11 (dd, J = 13.3, 5.1 Hz, 1H), 4.44 (d, J = 17.4 Hz, 1H), 4.37 (d, J = 6.1 Hz, 2H), 4.30 (d, J = 17.3 Hz, 1H), 2.98-2.85 (m, 1H), 2.85-2.75 (m, 1H), 2.64-2.55 (m, 1H), 2.44-2.33 (m, 1H), 2.04-1.90 (m, 3H), 1.73-1.62 (m, 2H), 1.61-1.51 (m, 4H). Molecular Formula: C22H23N3O4, theoretical molecular weight: 393.44; LC-MS(ESI):m/z=[M+H]+: 394.44.

### Embodiment 112: compound 112 synthesis

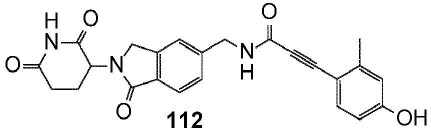

¹H NMR (400 MHz, DMSO-*d6*): 10.99 (s, 1H), 10.04 (s, 1H), 9.24 (s, 1H), 7.71 (d, J = 7.8 Hz, 1H), 7.51 (s, 1H), 7.43 (d, J = 8.0 Hz, 1H), 7.34 (d, J = 8.4 Hz, 1H), 6.71 (s, 1H), 6.64 (dd, J = 8.3, 2.5 Hz, 1H), 5.11 (dd, J = 13.3, 5.0 Hz, 1H), 4.48-4.43 (m, 3H), 4.32 (d, J = 17.4 Hz, 1H), 2.96-2.86 (m, 1H), 2.68-2.57 (m, 1H), 2.45-2.33 (m, 1H), 2.27 (s, 3H), 2.03-1.96 (m, 1H). Molecular Formula: C24H21N3O5, theoretical molecular weight: 431.45; LC-MS(ESI):m/z=[M+H]+: 432.45.

### Embodiment 113: compound 113 synthesis

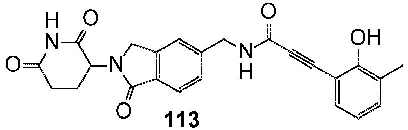

¹H NMR (400 MHz, DMSO-*d6*): 11.00 (s, 1H), 9.21 (s, 1H), 9.04 (s, 1H), 7.72 (d, J = 7.8 Hz, 1H), 7.53 (s, 1H), 7.45 (d, J = 8.0 Hz, 1H), 7.29-7.19 (m, 2H), 6.79 (t, J = 7.6 Hz, 1H), 5.11 (dd, J = 13.3, 5.0 Hz, 1H), 4.53-4.41 (m, 3H), 4.32 (d, J = 17.4 Hz, 1H), 2.98-2.85 (m, 1H), 2.63-2.57 (m, 1H), 2.43-2.35 (m, 1H), 2.18 (s, 3H), 2.03-1.95 (m, 1H). Molecular Formula: C24H21N3O5, theoretical molecular weight: 431.45; LC-MS(ESI):m/z=[M+H]+: 432.45.

### Embodiment 114: compound 114 synthesis

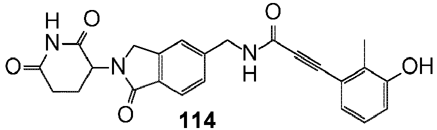

¹H NMR (400 MHz, DMSO-*d6*): 10.99 (s, 1H), 9.73 (s, 1H), 9.37 (s, 1H), 7.71 (d, J = 7.7 Hz, 1H), 7.52 (s, 1H), 7.43 (d, J = 8.0 Hz, 1H), 7.06 (t, J = 7.8 Hz, 1H), 6.98 (d, J = 7.4 Hz, 1H), 6.91 (d, J = 8.1 Hz, 1H), 5.11 (dd, J = 13.1, 5.1 Hz, 1H), 4.50-4.43 (m, 3H), 4.32 (d, J = 17.4 Hz, 1H), 2.93-2.86 (m, 1H), 2.63-2.57 (m, 1H), 2.42-2.36 (m, 1H), 2.26 (s, 3H), 2.05-1.95 (m, 2H). Molecular Formula: C24H21N3O5, theoretical molecular weight: 431.45; LC-MS(ESI):m/z=[M+H]+: 432.45.

### Embodiment 115: compound 115 synthesis

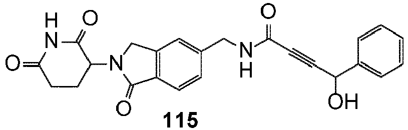

¹H NMR (400 MHz, DMSO-*d6*): 11.02 (s, 1H), 9.39 (s, 1H), 8.20 (s, 1H), 8.07-8.05 (m, 1H), 7.50 (s, 1H), 7.45-7.32 (m, 6H), 5.36 (s, 1H), 5.10 (dd, J = 13.2, 5.1 Hz, 1H), 4.51-4.44 (m, 3H), 4.35 (d, J = 17.4 Hz, 1H), 2.98-2.87 (m, 1H), 2.65-2.56 (m, 1H), 2.43- 2.37 (m, 1H), 2.05-1.96 (m, 1H). Molecular Formula: C24H21N3O5, theoretical molecular weight: 431.45; LC-MS(ESI):m/z= [M+H]+: 432.45.

### Embodiment 116: compound 116 synthesis

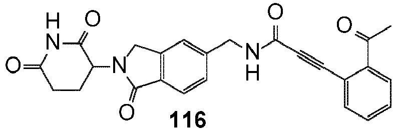

¹H NMR (400 MHz, DMSO-*d6*): 10.99 (s, 1H), 8.41 (s, 1H), 7.91 (d, J = 7.6 Hz, 1H), 7.83 (d, J = 7.7 Hz, 1H), 7.69 (d, J = 7.9 Hz, 1H), 7.66-7.51 (m, 3H), 7.46 (d, J = 7.9 Hz, 1H), 5.10 (dd, J = 13.1, 5.0 Hz, 1H), 4.56-4.38 (m, 3H), 4.31 (d, J = 17.3 Hz, 1H), 2.96-2.85 (m, 1H), 2.64-2.55 (m, 1H), 2.51 (s, 1H), 2.43-2.34 (m, 1H), 2.05-1.94 (m, 1H). Molecular Formula: C25H21N3O5, theoretical molecular weight: 443.46; LC-MS(ESI):m/z=[M+H]+: 444.46.

### Embodiment 117: compound 117 synthesis

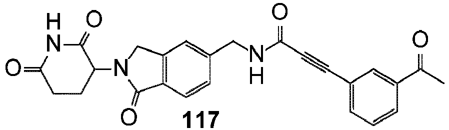

¹H NMR (400 MHz, DMSO-*d6*): 11.00 (s, 1H), 9.53 (s, 1H), 8.14 (s, 1H), 8.06 (d, J = 7.3 Hz, 1H), 7.84 (d, J = 7.7 Hz, 1H), 7.74 (dd, J = 16.1, 7.8 Hz, 1H), 7.68-7.58 (m, 1H), 7.54 (d, J = 12.6 Hz, 1H), 7.47-7.41 (m, 1H), 5.11 (dd, J = 13.3, 5.1 Hz, 1H), 4.54-4.39 (m, 3H), 4.32 (d, J = 16.9 Hz, 1H), 2.97-2.86 (m, 1H), 2.61 (s, 3H), 2.60-2.53 (m, 2H), 2.44-2.36 (m, 1H), 2.05-1.94 (m, 1H). Molecular Formula: C25H21N3O5, theoretical molecular weight: 443.46; LC-MS(ESI):m/z = [M+H]+: 444.46.

### Embodiment 118: compound 118 synthesis

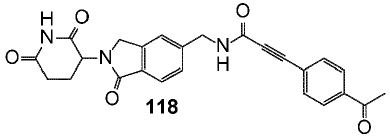

¹H NMR (400 MHz, DMSO-*d6*): 11.00 (s, 1H), 9.53 (s, 1H), 8.02 (d, J = 8.4 Hz, 2H), 7.73 (t, J = 8.1 Hz, 3H), 7.52 (s, 1H), 7.44 (d, J = 7.9 Hz, 1H), 5.11 (dd, J = 13.3, 5.1 Hz, 1H), 4.51-4.42 (m, 3H), 4.32 (d, J = 17.4 Hz, 1H), 2.98-2.85 (m, 1H), 2.61 (s, 3H), 2.60-2.57 (m, 1H), 2.44-2.36 (m, 1H), 2.05-1.92 (m, 1H). Molecular Formula: C25H21N3O5, theoretical molecular weight: 443.46; LC-MS(ESI):m/z = [M+H]+: 444.46.

### Embodiment 119: compound 119 synthesis

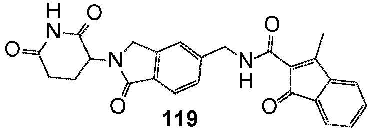

¹H NMR (400 MHz, DMSO-*d6*): 10.99 (s, 1H), 8.41 (s, 1H), 7.98-7.88 (m, 1H), 7.83 (d, J = 7.1 Hz, 1H), 7.70 (d, J = 7.9 Hz, 1H), 7.66-7.55 (m, 2H), 7.53 (s, 1H), 7.46 (d, J = 7.8 Hz, 1H), 5.11 (dd, J = 13.2, 5.1 Hz, 1H), 4.59-4.39 (m, 3H), 4.31 (d, J = 17.3 Hz, 1H), 2.98-2.84 (m, 1H), 2.63-2.57 (m, 1H), 2.51 (s, 3H), 2.46-2.34 (m, 1H), 2.03-1.96 (m, 1H). Molecular Formula: C25H21N3O5, theoretical molecular weight: 443.46; LC-MS(ESI):m/z=[M+H]+: 444.46.

### Embodiment 120: compound 120 synthesis

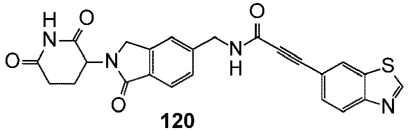

¹H NMR (400 MHz, DMSO-*d6*): 10.98 (s, 1H), 9.38 (s, 1H), 9.24 (s, 1H), 7.98 (s, 1H), 7.74 (dd, J = 8.2, 4.8 Hz, 2H), 7.55 (s, 1H), 7.46 (d, J = 7.9 Hz, 1H), 7.47-7.39 (m, 1H), 5.12 (dd, J = 13.3, 5.1 Hz, 1H), 4.53-4.41 (m, 3H), 4.32 (d, J = 17.2 Hz, 1H), 2.99-2.88 (m, 1H), 2.67-2.58 (m, 1H), 2.44-2.37 (m, 1H), 2.06-1.97 (m, 1H). Molecular Formula: C24H18N4O4S, theoretical molecular weight: 458.49; LC-MS(ESI):m/z = [M+H]+: 459.49.

### Embodiment 121: compound 121 synthesis

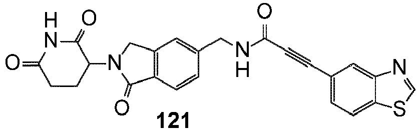

¹H NMR (400 MHz, DMSO-*d6*): 11.00 (s, 1H), 9.39 (s, 1H), 9.23 (s, 1H), 7.97 (s, 1H), 7.75 (dd, J = 8.2, 4.8 Hz, 2H), 7.56 (s, 1H), 7.48 (d, J = 7.9 Hz, 1H), 7.45-7.39 (m, 1H), 5.13 (dd, J = 13.3, 5.1 Hz, 1H), 4.53-4.46 (m, 3H), 4.31 (d, J = 17.2 Hz, 1H), 3.00-2.89 (m, 1H), 2.69-2.58 (m, 1H), 2.44-2.39 (m, 1H), 2.06-1.99 (m, 1H). Molecular Formula: C24H18N4O4S, theoretical molecular weight: 458.49; LC-MS(ESI):m/z = [M+H]+: 459.49.

### Embodiment 122: compound 122 synthesis

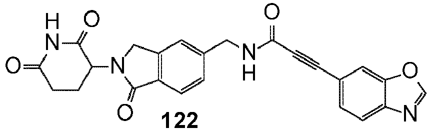

¹H NMR (400 MHz, DMSO-*d6*): 11.01 (s, 1H), 9.32 (s, 1H), 9.25 (s, 1H), 8.03 (s, 1H), 7.77 (dd, J = 8.3, 4.9 Hz, 2H), 7.54 (s, 1H), 7.46 (d, J = 7.8 Hz, 1H), 7.46-7.40 (m, 1H), 5.12 (dd, J = 13.6, 5.3 Hz, 1H), 4.55-4.46 (m, 3H), 4.34 (d, J = 17.1 Hz, 1H), 3.00-2.91 (m, 1H), 2.69-2.59 (m, 1H), 2.47-2.39 (m, 1H), 2.08-1.99 (m, 1H). Molecular Formula: C24H18N4O5, theoretical molecular weight: 442.43; LC-MS(ESI):m/z = [M+H]+: 443.43.

### Embodiment 123: compound 123 synthesis

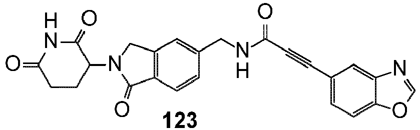

¹H NMR (400 MHz, DMSO-*d6*): 11.00 (s, 1H), 9.31 (s, 1H), 9.25 (s, 1H), 8.06 (s, 1H), 7.79 (dd, J = 8.4, 5.0 Hz, 2H), 7.58 (s, 1H), 7.47 (d, J = 7.9 Hz, 1H), 7.48-7.40 (m, 1H), 5.10 (dd, J = 13.5, 5.4 Hz, 1H), 4.54-4.47 (m, 3H), 4.32 (d, J = 17.0 Hz, 1H), 3.01-2.93 (m, 1H), 2.69-2.61 (m, 1H), 2.48-2.38 (m, 1H), 2.08-1.97 (m, 1H). Molecular Formula: C24H18N4O5, theoretical molecular weight: 442.43; LC-MS(ESI):m/z = [M+H]+: 443.43.

### Embodiment 124: compound 124 synthesis

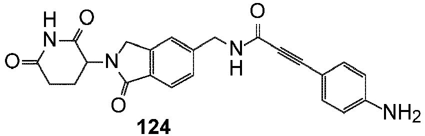

¹H NMR (400 MHz, DMSO-*d6*): 11.02 (s, 1H), 9.31 (s, 1H), 7.75 (d, J = 7.8 Hz, 1H), 7.54 (s, 1H), 7.48-7.42 (m, 1H), 7.25 (dd, J = 7.8, 1.6 Hz, 1H), 7.18-7.12 (m, 1H), 6.71 (d, J = 8.2 Hz, 1H), 6.53 (td, J = 7.8, 1.3 Hz, 1H), 5.82 (s, 2H), 5.10 (dd, J = 13.5, 5.2 Hz, 1H), 4.58-4.42 (m, 3H), 4.35 (d, J = 17.4 Hz, 1H), 2.93-2.87(m, 1H), 2.67-2.60 (m, 1H), 2.44-2.37 (m, 1H), 2.02-1.95 (m, 1H). Molecular Formula: C23H20N4O4, theoretical molecular weight: 426.44; LC-MS(ESI):m/z= [M+H]+: 417.44.

### Embodiment 125: compound 125 synthesis

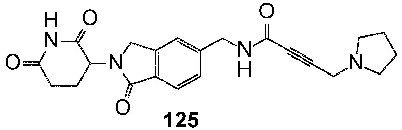

¹H NMR (400 MHz, DMSO-*d6*): 10.98 (s, 1H), 9.20 (s, 1H), 7.69 (d, J = 7.8 Hz, 1H), 7.47 (s, 1H), 7.39 (d, J = 7.8 Hz, 1H), 5.10 (dd, J = 13.3, 5.1 Hz, 1H), 4.48-4.36 (m, 3H), 4.31 (d, J = 17.3 Hz, 1H), 3.53 (s, 2H), 2.98-2.87 (m, 1H), 2.62-2.57 (m, 1H), 2.56-2.51 (m, 4H), 2.42-2.36 (m, 1H), 2.04-1.95 (m, 1H), 1.78-1.61 (m, 4H). Molecular Formula: C22H24N4O4, theoretical molecular weight: 408.46; LC-MS(ESI):m/z = [M+H]+: 409.46.

### Embodiment 126: compound 126 synthesis

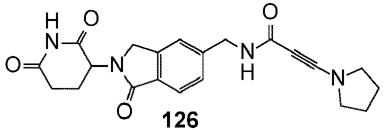

¹H NMR (400 MHz, DMSO-*d6*): 11.03 (s, 1H), 9.07 (s, 1H), 7.67 (d, J = 7.8 Hz, 1H), 7.45 (s, 1H), 7.38 (d, J = 7.8 Hz, 1H), 5.11 (dd, J = 13.3, 5.1 Hz, 1H), 4.44 (d, J = 17.4 Hz, 1H), 4.37 (d, J = 6.1 Hz, 2H), 4.30 (d, J = 17.3 Hz, 1H), 2.97-2.85 (m, 4H), 2.85-2.78 (m, 1H), 2.64-2.57 (m, 1H), 2.44-2.35 (m, 1H), 2.04-1.92 (m, 1H), 1.62-1.55 (m, 4H). Molecular Formula: C21H22N4O4, theoretical molecular weight: 394.43; LC-MS(ESI):m/z = [M+H]+: 395.43.

### Embodiment 127: compound 127 synthesis

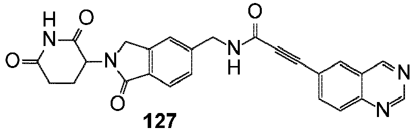

¹H NMR (400 MHz, DMSO-*d6*): 10.98 (s, 1H), 9.67 (s, 1H), 9.58 (s, 1H), 9.38 (s, 1H), 8.49 (d, J = 1.8 Hz, 1H), 8.15-8.05 (m, 2H), 7.73 (d, J = 7.9 Hz, 1H), 7.54 (s, 1H), 7.46 (d, J = 7.9 Hz, 1H), 5.12 (dd, J = 13.3, 5.1 Hz, 1H), 4.56-4.41 (m, 3H), 4.33 (d, J = 17.4 Hz, 1H), 2.96-2.87 (m, 1H), 2.64-2.57 (m, 1H), 2.45-2.35 (m, 1H), 2.05-1.96 (m, 1H). Molecular Formula: C25H19N5O4, theoretical molecular weight: 453.46; LC-MS(ESI):m/z=[M+H]+: 454.46.

### Embodiment 128: compound 128 synthesis

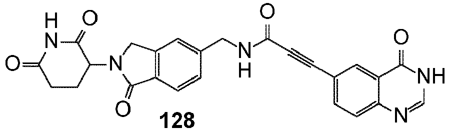

¹H NMR (400 MHz, DMSO-d6) : 12.47 (s, 1H), 10.98 (s, 1H), 9.48 (s, 1H), 8.28 (d, J = 2.0 Hz, 1H), 8.18 (s, 1H), 7.95 (dd, J = 8.5, 2.0 Hz, 1H), 7.72 (d, J = 8.2 Hz, 2H), 7.53 (s, 1H), 7.45 (d, J = 7.9 Hz, 1H), 5.11 (dd, J = 13.3, 5.1 Hz, 1H), 4.52-4.42 (m, 3H), 4.33 (d, J = 17.3 Hz, 1H), 2.96-2.86 (m, 1H), 2.64-2.56 (m, 1H), 2.45-2.34 (m, 1H), 2.02-1.98 (m, 1H). Molecular Formula: C25H19N5O5, theoretical molecular weight: 469.46; LC-MS(ESI):m/z = [M+H]+: 470.46.

### Embodiment 129: compound 129 synthesis

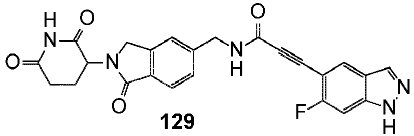

¹H NMR (400 MHz, DMSO-*d6*) : 13.43 (s, 1H), 10.98 (s, 1H), 9.44 (s, 1H), 8.20-8.12 (m, 2H), 7.71 (d, J = 7.8 Hz, 1H), 7.51 (d, J = 9.5 Hz, 2H), 7.44 (d, J = 7.9 Hz, 1H), 5.11 (dd, J = 13.3, 5.1 Hz, 1H), 4.51-4.42 (m, 3H), 4.33 (d, J = 17.3 Hz, 1H), 2.97-2.87 (m, 1H), 2.64-2.56 (m, 1H), 2.45-2.35 (m, 1H), 2.04-1.97 (m, 1H). Molecular Formula: C24H18FN5O4, theoretical molecular weight: 459.44; LC-MS(ESI):m/z= [M+H]+: 460.44.

### Embodiment 130: compound 130 synthesis

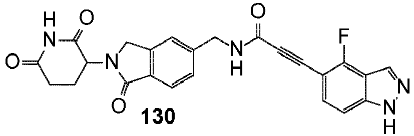

¹H NMR (400 MHz, DMSO-*d6*): 13.71 (s, 1H), 10.98 (s, 1H), 9.44 (s, 1H), 8.32 (s, 1H), 7.71 (d, J = 7.8 Hz, 1H), 7.53 (s, 1H), 7.52-7.37 (m, 3H), 5.11 (dd, J = 13.3, 5.1 Hz, 1H), 4.55-4.40 (m, 3H), 4.33 (d, J = 17.4 Hz, 1H), 2.97-2.87 (m, 1H), 2.62-2.57 (m, 1H), 2.44-2.34 (m, 1H), 2.06-1.98 (m, 1H). Molecular Formula: C24H18FN5O4, theoretical molecular weight: 459.44; LC-MS(ESI):m/z= [M+H]+: 460.44.

### Embodiment 131: compound 131 synthesis

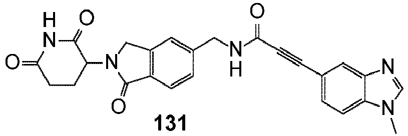

¹H NMR (400 MHz, DMSO-*d6*): 11.00 (s, 1H), 9.37 (s, 1H), 8.35 (s, 1H), 7.93 (s, 1H), 7.74 (dd, J = 8.2, 4.8 Hz, 2H), 7.55 (s, 1H), 7.46 (d, J = 7.8 Hz, 1H), 7.45-7.38 (m, 1H), 5.12 (dd, J = 13.2, 5.3 Hz, 1H), 4.54-4.46 (m, 3H), 4.30 (d, J = 17.2 Hz, 1H), 3.87 (s, 3H), 2.97-2.87 (m, 1H), 2.64-2.58 (m, 1H), 2.44-2.39 (m, 1H), 2.03-1.97 (m, 1H). Molecular Formula: C25H21N5O4, theoretical molecular weight: 455.47; LC-MS(ESI):m/z=[M+H]+: 456.47.

### Embodiment 132: compound 132 synthesis

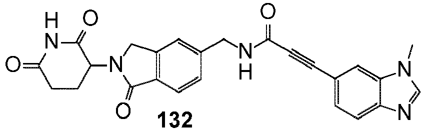

¹H NMR (400 MHz, DMSO-*d6*): 10.98 (s, 1H), 9.38 (s, 1H), 8.34 (s, 1H), 7.90 (s, 1H), 7.71 (dd, J = 8.1, 4.9 Hz, 2H), 7.53 (s, 1H), 7.45 (d, J = 7.9 Hz, 1H), 7.42-7.33 (m, 1H), 5.11 (dd, J = 13.3, 5.1 Hz, 1H), 4.54-4.41 (m, 3H), 4.33 (d, J = 17.2 Hz, 1H), 3.86 (s, 3H), 2.96-2.87 (m, 1H), 2.63-2.57 (m, 1H), 2.43-2.37 (m, 1H), 2.04-1.97 (m, 1H). Molecular Formula: C25H21N5O4, theoretical molecular weight: 455.47; LC-MS(ESI):m/z=[M+H]+: 456.47.

### Embodiment 133: compound 133 synthesis

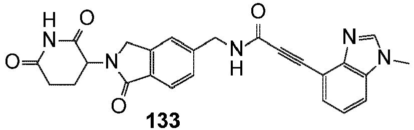

¹H NMR (400 MHz, DMSO-*d6*) : 10.98 (s, 1H), 9.45 (s, 1H), 8.32 (s, 1H), 7.73 (dd, J = 10.5, 8.0 Hz, 2H), 7.53 (s, 1H), 7.46 (t, J = 6.9 Hz, 2H), 7.33 (t, J = 7.8 Hz, 1H), 5.11 (dd, J = 13.2, 5.2 Hz, 1H), 4.51-4.43 (m, 3H), 4.33 (d, J = 17.3 Hz, 1H), 3.87 (s, 3H), 2.95-2.86 (m, 1H), 2.64-2.57 (m, 1H), 2.43-2.35 (m, 1H), 2.05-1.98 (m, 1H). Molecular Formula: C25H21N5O4, theoretical molecular weight: 455.47; LC-MS(ESI):m/z = [M+H]+: 456.47.

### Test case 1: Regulatory effect of the present invention compound on GSPT1 in MV-4-11 cells

MV-4-11 (human myeloid monocytic leukemia cells) and NB-4 (human acute promyelocytic leukemia cells) cells were treated with medium RPMI 1640+10% FBS at 1*10⁶/well was seeded in 6-well plates, then compounds were added for 4 h treatment, DMSO was used as a blank control, and cells were collected by centrifugation. Western blot analysis was performed with antibody labeling (Anti-GSPT1: CellSignaling Technology #14980s; Anti-GAPDH: HuabioET601-4; Goatanti-RabbitIgG-HRPantibody: HuabioHA 100 1) for detection on a Bio-Rad or MINICHEMITM imaging system_{∘}

The results showed in Figure 1, 10 µM compound **1** induced completely degradation of GSPT1 protein in MV-4-11 cells. Compound **12** induced the degradation of GSPT1 more as the concentration increases from 1.52 nM in MV-4-11 cells. Compound **83** induced significantly degradation of GSPT1 in NB-4 cells at 0.01 µM, and pretreatment with the proteasome inhibitor MG132 or NEDD8 activating enzyme inhibitor MLN4924 completely blocked the compound-induced degradation of GSPT1.

### Test case 2: The inhibitory effect of the disclosed compound on tumor cell proliferation

MV-4-11 cells were diluted with medium (RPM1640 + 10% FBS + 1% PS) and plated at 4000 cells/well in a white-walled clear-bottom 96-well plate. MV-4-11 cells were plated and incubated in a 5% CO2, 37°C incubator for 2 hours. Cells were treated with the indicated concentration of compound or DMSO (control), and placed in a 5% CO2, 37°C incubator for 72 h after dosing. After the end of the culture, equilibrate at room temperature for 30 min, add 100 µl of CellTiter-Glo^{®} Reagent to each well, and shake the microplate shaker for 2 min. After 10 min of incubation at room temperature, the luminescence was measured by a microplate reader, and the cell-free wells were used as the background wells. The IC50 values of the compound for the proliferative inhibitory activity of MV-4-11 cells are shown in Table 1. CC-90009 (celegen patent phase I) was used as a control, and its structural formula was as follows:

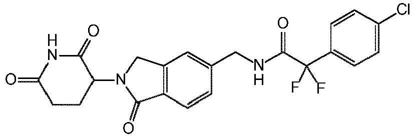

**Table 1: In vitro determination of the IC50 values of each embodiment compound on the inhibitory activity of tumor cell proliferation**

| | IC50 | Co mpo und num ber | IC50 | Com poun d numb er | IC50 | Com poun d numb er | IC50 | Co mp oun d nu mb er | IC5 0 | compo und numbe ring | IC50 | Co mp oun d nu mb er | IC50 |
|---|---|---|---|---|---|---|---|---|---|---|---|---|---|
| CC-900 09 | ++ | 20 | + | 39 | +++ | 58 | +++ | 77 | + | 96 | + | 115 | + |
| 1 | ++++ | 21 | ++++ | 40 | +++ | 59 | ++ | 78 | + | 97 | +++ | 116 | +++ |
| 2 | ++++ | 22 | + | 41 | +++ | 60 | ++ | 79 | ++ + | 98 | +++ | 117 | ++++ |
| 3 | ++++ | 23 | + | 42 | + | 61 | + | 80 | ++ | 99 | ++++ | 118 | +++ |
| 4 | ++++ | 24 | ++++ | 43 | + | 62 | +++ + | 81 | ++ ++ | 100 | + | 119 | + |
| 5 | ++++ | 25 | ++++ | 44 | + | 63 | +++ + | 82 | ++ | 101 | ++ | 120 | +++ |
| 6 | ++++ | 26 | + | 45 | + | 64 | + | 83 | ++ + | 102 | ++++ | 121 | ++++ |
| 7 | ++++ | 27 | +++ | 46 | ++++ | 65 | +++ | 84 | ++ + | 103 | ++ | 122 | ++ |
| 8 | ++++ | 28 | +++ | 47 | + | 66 | +++ | 85 | ++ | 104 | ++++ | 123 | ++++ |
| 9 | ++++ | 29 | + | 48 | +++ | 67 | +++ | 86 | ++ | 105 | +++ | 124 | ++ |
| 10 | ++++ | 30 | +++ | 49 | +++ | 68 | ++ | 87 | ++ | 106 | ++ | 125 | + |
| 11 | ++++ | 31 | ++ | 50 | +++ | 69 | +++ + | 88 | ++ + | 107 | ++ | 126 | + |
| 12 | +++ | 32 | +++ | 51 | ++++ | 70 | +++ + | 89 | ++ + | 108 | +++ | 127 | + |
| 13 | ++++ | 33 | + | 52 | ++++ | 71 | ++ | 90 | + | 109 | +++ | 128 | ++ |
| 14 | + | 34 | ++ | 53 | ++ | 72 | + | 91 | ++ + | 110 | +++ | 129 | ++ |
| 15 | +++ | 35 | ++ | 54 | ++++ | 73 | +++ | 92 | + | 111 | +++ | 130 | +++ |
| 16 | ++ | 36 | ++++ | 55 | ++++ | 74 | + | 93 | ++ | 112 | +++ | 131 | +++ |
| 18 | ++++ | 37 | + | 56 | ++++ | 75 | + | 94 | + | 113 | ++++ | 132 | ++ |
| 19 | +++ | 38 | +++ | 57 | ++ | 76 | + | 95 | + | 114 | +++ | 133 | +++ |

For IC50 values, where "++++" denotes IC50<1 nM;"+++" indicates that the IC50 is between 1 nM and 10 nM, and "++" indicates that the IC50 is between 10 and 100 nM; "+" indicates IC50>100 nM.

### Test Case 3: hERG inhibition assay

The fast-activating potassium channel encoded by the human ether-a-go-go-related gene (hERG) is an important ion channel involved in the formation of phase 3 repolarization of myocardial action potentials. Drug blockade of the hERG channel can lead to prolonged cardiac repolarization, which is called long QT syndrome on ECG. Drug-induced delayed ventricular repolarization may cause a fatal arrhythmia-torsade de pointes in some cases. This test example is used to measure the effect of the compound on the hERG potassium channel of the clone expressed in human embryonic kidney cells (HEK293).

Assay: cells are placed in HEPES-buffered saline solution in glass-lined 96-well plates and loaded with appropriate amounts of test and control solution (0.3% DMSO solution) at each concentration for 3 min exposure time. Dilute the compound to be tested in 0.3% DMSO. An automated parallel patch-clamp system QPatch 48 X (Sophion) was used to test the inhibition of hERG by compounds at a concentration of 3 µM.

The calculation method of the inhibition rate is as follows: (peak tail current in the control group - peak tail current in the test group)/(peak tail current in the control group)×100%.

Results: At a concentration of 3 µM, compound **12, 27, 31, 38, 57, 58, 59, 68, 71, 72, 80, 82,** and **83** inhibited hERG less than 20%, indicating that these compounds had almost no hERG inhibitory activity. Compound CC-90009 produces a significant 64% inhibition of hERG at 3 µM. It can be seen that the compound of the present invention can significantly reduce the inhibitory activity of hERG channel.

| Compound | Inhibition rate (3 µM) |
|---|---|
| CC-90009 | 64.48% |
| **12** | -3.22% |
| **27** | -2.36% |
| **31** | -0.61% |
| **38** | 1.85% |
| **57** | -0.68% |
| **58** | 5.39% |
| **59** | 4.65% |
| **68** | -3.00% |
| **71** | 12.86% |
| **72** | 2.25% |
| **80** | -5.31% |
| **82** | 3.91% |
| **83** | -0.53% |

## Claims

1. A compound of formula (I), or a pharmaceutically acceptable salt, tautomer, mesomer, racemate, stereoisomer, metabolite, metabolic precursor, or prodrug thereof. 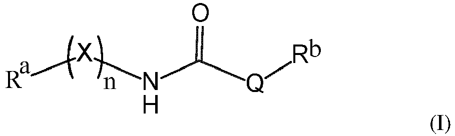 wherein
R^{a} is presented by the formula (II): 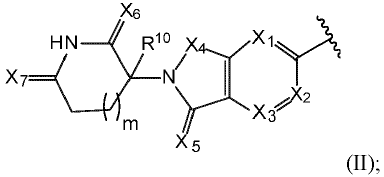
X₁ is Nor CR¹;
X₂ is N or CR²;
X₃ is N or CR³;
X₄ is CH₂, O, S, NR⁴, C=O, or C=S;
X₁, X₂, X₃, and X₄ are not N at the same time;
X₅ and X₇ are each independently O or S;
X₆ is O, S, or X₆ is absent;
R¹, R², R³, and R⁴ are each independently selected from hydrogen, deuterium, C₁₋₃ alkyl, C₁₋₃ alkoxy, C₃₋₆ cycloalkyl, 3- to 6- membered heterocycloalkyl, 4- to 7- membered aryl, 4- to 7- membered heteroaryl, hydroxyl, halogen, cyano, or -N(R⁵)(R⁶);
R⁵ and R⁶ are each independently selected from hydrogen, C₁₋₃ alkyl, C₁₋₃ alkoxy, C₃₋₆ cycloalkyl, 4- to 7- membered heterocycloalkyl, 4- to 7- membered heteroaryl, or R⁵ and R⁶ together with the nitrogen atom to which they are attached form a 3- to 6- membered mono saturated nitrogen-containing ring;
R¹⁰ is hydrogen, deuterium, C₁₋₃ alkyl, C₁₋₃ alkoxy, hydroxyl, halogen, or cyano;
m is 0, 1, 2 or 3;
R^{b} is C₁₋₁₂ alkyl, Si₁₋₃ alkyl, C₁₋₁₂ alkoxy, C₂₋₁₂ alkenyl, C₂₋₁₂ alkyne, C₃₋₁₂ cycloalkyl, 3- to 12-membered heterocycloalkyl, 5- to 12- membered aryl, 5- to 12- membered heteroaryl, 6- to 12- membered dicyclic carbon ring, 6- to 12- membered partially unsaturated bicyclic carbon ring, 8- to 12- membered benzoheterocyclic, 6- to 12- membered bicyclic heteroaryl, 10- to 15- membered tricyclic carbon ring or 10- to 15- membered partially unsaturated tricyclic carbon ring, wherein the C₁₋₁₂ alkyl, C₁₋₁₂ alkoxy, C₂₋₁₂ alkenyl, C₂₋₁₂ alkyne, C₃₋₁₂ cycloalkyl, 3- to 12- membered heterocycloalkyl, 5- to 12- membered aryl, 5- to 12- membered heteroaryl, 6- to 12- membered bicyclic carbon rings, 6- to 12- membered partially unsaturated bicyclic carbon rings, 6- to 12- membered bicyclic heteroaryl, 10- to 15- membered tricyclic carbon rings, and the 10- to 15- membered partially unsaturated tricyclic carbon rings, are optionally substituted with one or more R;
X is methylene, each hydrogen on the methylene is substituted with one or more deuterium or halogen;
Q is a bond, -CR⁷=C-, -C=C-,-C(R⁷)=N-,-N=C(R⁷)- or -N=N-; preferably, Q is -C≡C-;
R⁷ is selected from hydrogen, deuterium, C₁₋₆ alkyl, C₁₋₆ alkoxy, C₃₋₉ cycloalkyl, 3- to 9- membered heterocycloalkyl, 5- to 9- membered aryl, 5- to 9- membered heteroaryl, hydroxyl, halogen, cyano, wherein the C₁₋₆ alkyl, C₁₋₆ alkoxy, C₃₋₉ cycloalkyl, 3- to 9- membered heterocycloalkyl, 5- to 9-membered aryl, and 5- to 9- membered heteroaryl are optionally substituted with one or more R;
R is selected from deuterium, phosphonates, C₁₋₆ alkyl, C₁₋₆ alkoxy, Si₁₋₆ alkoxy, C₁₋₆ alkoxy carbonyl, -C(O)-C₁₋₆ alkyl, -C(O)-C₁₋₆ alkoxy, -O-C(O)-C₁₋₆ alkyl, -O-C(O)-3- to 9- membered heterocycloalkyl, C₃₋₉ cycloalkyl, 3- to 9- membered heterocycloalkyl, 5- to 9- membered aryl, 5- to 9-membered heteroaryl, carboxy, hydroxyl, halogen, cyano, amino, nitro, or -N(R⁸)(R⁹), wherein C₁₋₆ alkyl, Si₁₋₆ alkoxy, ₋C(O)-C₁₋₆ alkyl, -C(O)-C₁₋₆ alkoxy, ₋O-C(O)-C₁₋₆ alkyl, -O-C(O)-C₆₋₁₂ heterocycloalkyl, 5-to 9- membered aryl, and 3- to 9- membered heterocycloalkyl are optionally substituted with one or more halogen, carboxyl, nitro, amino, C₁₋₆ alkyl, and 6-membered heterocycloalkyllor -O-C(O)-C₁₋₆ alkyl; and H atoms on the phosphonate are optionally substituted with one or more R¹¹;
R¹¹ is selected from methyl or phenyl, or two R¹¹ together with the atoms to which they are attached O and P form a dioxophosphate heterocyclic alkyl ring, wherein the dioxophosphate heterocyclic alkyl ring is optionally substituted with one or more methly;
R⁸ and R⁹ are each independently selected from hydrogen, C₁₋₆ alkyl, C₁₋₆ alkoxy, C₃₋₉ cycloalkyl, 4-to 7- membered heterocycloalkyl or 4- to 7- membered heteroaryl, wherein the C₁₋₆ alkyl is optionally substituted with one or more hydroxyl, halogen, cyano or C₁₋₃ alkoxy; or R⁸ and R⁹ together with the nitrogen atom to which they are attached form 3- to 6- membered mono saturated nitrogen-containing ring;
n is an integer from 0 to 5.

2. The compound according to claim 1, or a pharmaceutically acceptable salt, tautomer, mesomer, racemate, stereoisomer, metabolite, metabolic precursor or prodrug thereof, wherein R¹⁰ is hydrogen, deuterium or halogen, and preferably, R¹⁰ is hydrogen or fluorine;
preferably, m is an integer from 0 to 2, and preferably, m is 1;
preferably, X is methylene, and one or more hydrogen atoms on the methylene are optionally substituted with deuterium or fluorine;
preferably, Q is -C≡C-;
preferably, n is an integer from 0 to 3, preferably, n is an integer from 0 to 2, preferably, n is 0 or 1, and preferably, n is 1;
preferably, R^{a} is defined as in formula (IIa) or (IIb), 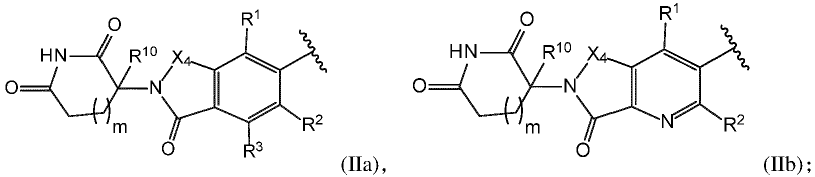
wherein X4 is selected from -CH₂ or -C=O;
R¹, R², R³, m are defined as in formula (II);
preferably, R^{a} is selected from the following groups:

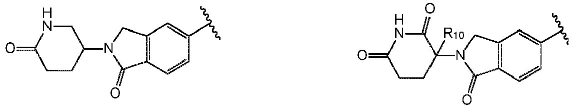

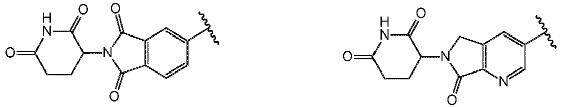

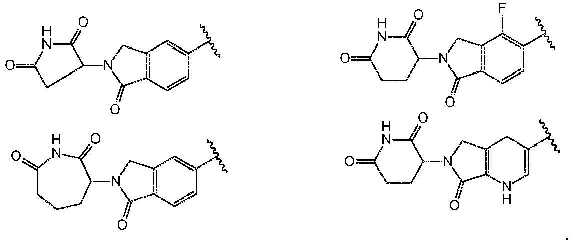

3. The compound according to claims 1-2, or a pharmaceutically acceptable salt, tautomer, mesomer, racemate, stereoisomer, metabolite, metabolic precursor or prodrug there, wherein R^{b} is Si₁₋₃ alkyl, C₁₋₆ alkyl, C₁₋₆ alkoxy, C_{2 -6} alkenyl, C₂₋₆ alkynyl, C₃₋₈ cycloalkyl, 5- to 8- membered heterocyclic alkyl, 5- to 7- membered aryl, 5- to 7- membered heteroaryl, 8- to 12- membered bicyclic carbon ring, 8- to 12-membered partially unsaturated bicyclic carbon ring, 8- to 12- membered benzoheterocyclics, 8- to 12-membered bicyclic heteroaryl, or 10- to 15- membered tricyclic carbon rings, wherein C₁₋₆ alkyl, C₁₋₆ alkoxy, C₂₋₆ alkenyl, C₂₋₆ alkyne, C₃₋₈ cycloalkyl, 5- to 8- membered heterocycloalkyl, 5- to 7- membered aryl, 5- to 7- membered heteroaryl, 8- to 12- membered bicyclic carbon ring, 8- to 12- membered partially unsaturated bicyclic carbon ring, 8- to 12- membered bicyclic heteroaryl, and 10- to 15- membered tricyclic carbon ring are optionally substituted with one or more R;
preferably, R^{b} is silyl, C₁₋₆ alkyl, C₃₋₆ cycloalkyl, 5- to 6- membered heterocyclicalkyl, 5- to 6-membered aryl, 5- to 6- membered heteroaryl, 8- to 12- membered benzoheterocyclic, or 10 membered tricyclic carbon ring, wherein C₁₋₆ alkyl, C3-6 cycloalkyl, 5- to 6- membered heterocyclicalkyl, 5- to 6-membered aryl, 5- to 6- membered heteroaryl, 8- to 12- membered benzoheterocyclic, and 10-membered tricyclic carbon ring are optionally substituted with one or more R groups;
preferably, R^{b} is methyl, ethyl, propyl, tert-butyl, methoxy, trimethylsilyl, cyclopropyl, cyclopentyl, cyclohexyl, pyrrolidyl, piperidinyl, phenyl, pyridyl, pyrimidinyl, thienyl, pyrazolyl, imidazolyl, pyrazinyl, benzofuranyl, benzothiazolyl, benzimidazolyl, benzoxazolyl, indolyl, indazolyl, quinolinyl, quinolinonyl, 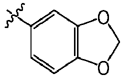 or 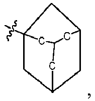 wherein methyl, ethyl, propyl, tert-butyl, methoxy, phenyl, pyridyl, pyrimidinyl, pyrazinyl, thienyl, pyrazolyl, imidazolyl, pyrazinyl, benzofuranyl, benzothiazolyl, benzimidazoly, benzoxazolyl, indolyl, indazolyl, quinolinyl, and quinolinonyl are optionally substituted with one or more R groups;
preferably, R^{b} is phenyl or thiophene.

4. The compound according to claims 1-3, or a pharmaceutically acceptable salt, tautomer, mesoform, racemate, stereoisomer, metabolite, metabolic precursor or prodrug thereof, wherein, R is selected from deuterium, phosphate ester group, C₁₋₄ alkyl, Si₁₋₄ alkoxy, C₁₋₄ alkoxy, C₁₋₄ alkoxycarbonyl, -C(O)-C₁₋₄ alkyl, -C(O)-C₁₋₄ alkoxy, -OC(O)-C₁₋₄ alkyl, -OC(O)-6-membered heterocycloalkyl, pyrrolidinyl, Phenyl, carboxyl, hydroxyl, halogen, cyano, amino or nitro; said C1-4 alkyl, Si 1 -4 alkoxy, -C(O)-C₁₋₄ alkyl, -C(O)-C₁₋₄ alkoxy, -OC(O)-C₁₋₄ alkyl, -OC(O)-6-membered heterocycloalkyl and phenyl are optionally substituted by one or more halogen , methyl, tert-butyl, carboxyl, nitro, amino, piperidinyl or -OC(O)-C₁₋₄ alkyl;
preferably, R is selected from methyl, ethyl, hydroxyl, cyano, amino, nitro, methoxycarbonyl, ethoxycarbonyl, tert-butoxycarbonyl, carboxy, phenyl, fluorine, chlorine, bromine, trifluoromethyl, nitrophenyl, aminophenyl, -CH₂CF₂, -C(O)CH₃, -C(O) CF₃, 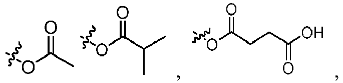 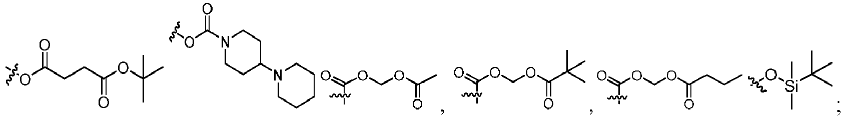
preferably, R is selected from methyl, ethyl, fluorine, chlorine, bromine, nitro, amino, ethoxycarbonyl, trifluoromethyl, methoxy, tert-butoxycarbonyl, nitrophenyl, aminophenyl, -CH₂CF₂, or -C(O)CH₃.

5. The compound according to claims 1-4, or a pharmaceutically acceptable salt, tautomer, mesomer, racemate, stereoisomer, metabolite, metabolic precursor, or prodrug thereof, wherein, R^{b} is -CH₃, - CH₂CH₃, -CH₂CH₂CH₃, -CH₂CH₂CH₂CH₂CH₃, -C(CH₃)₃, -CH₂OH, cyclopropyl, cyclopentyl, cyclohexyl, phenyl, trimethylsilyl 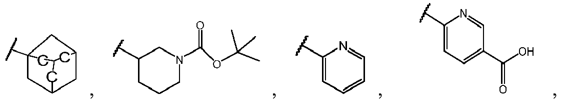 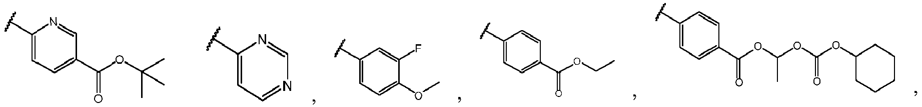 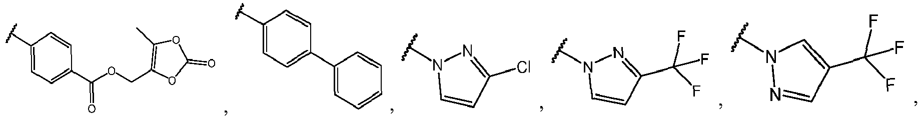 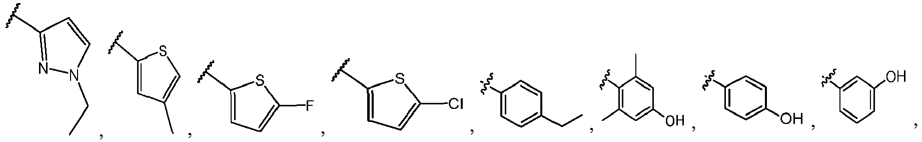 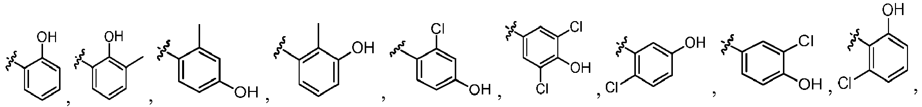 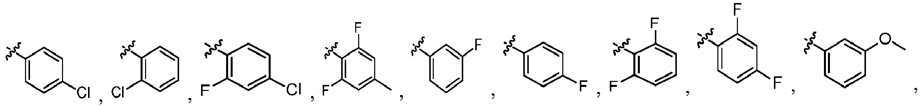 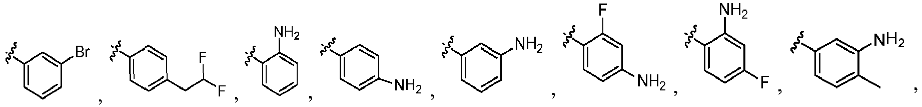 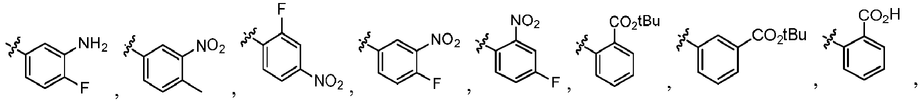 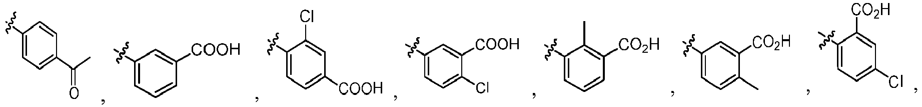 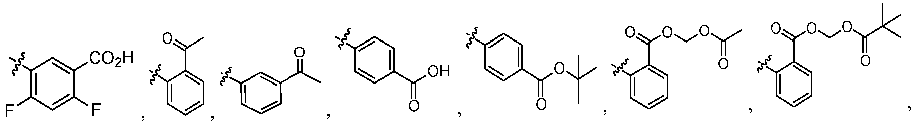 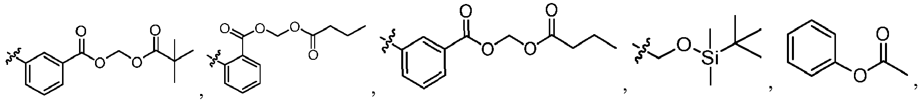 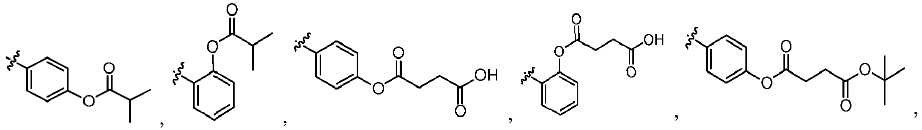 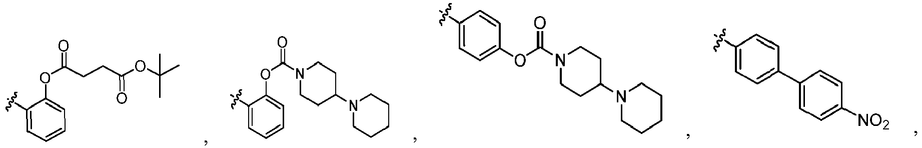 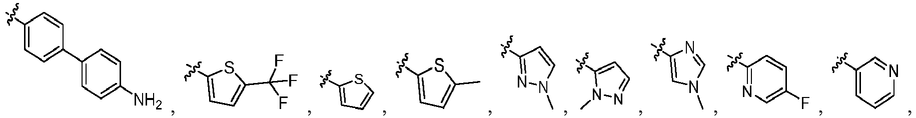 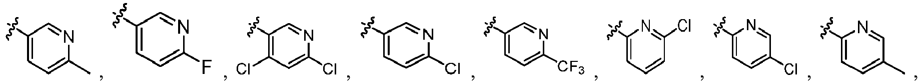 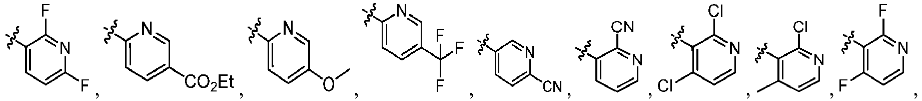 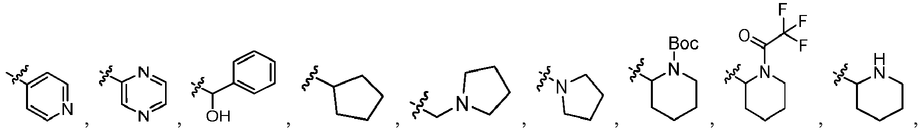 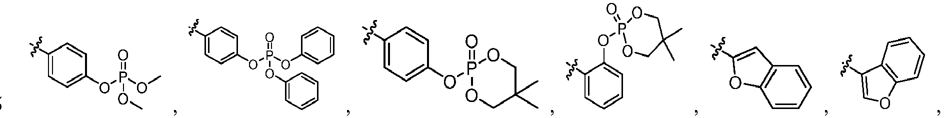 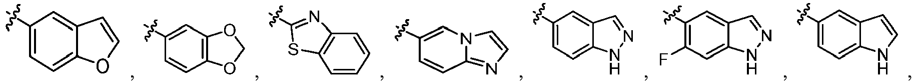 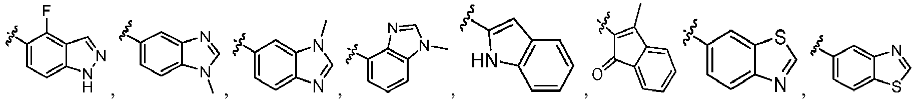 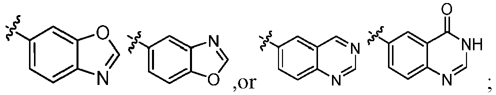 preferably, R^{b} is ethyl, -CH₂OH, trimethylsilyl, cyclopropyl, cyclopentyl, cyclohexyl, phenyl, 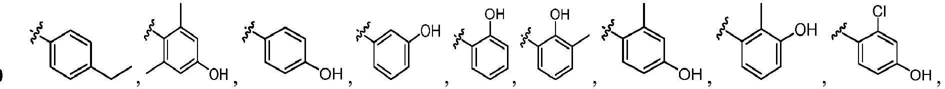 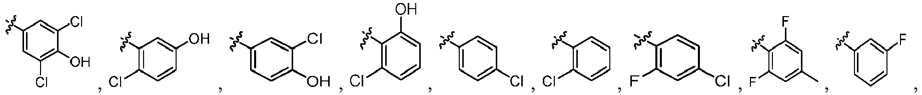 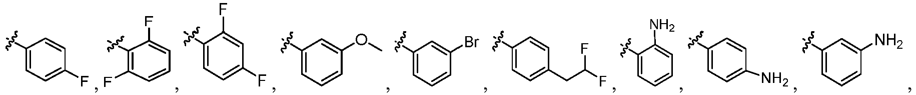 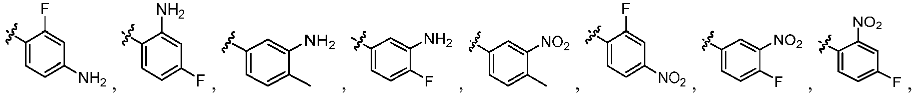 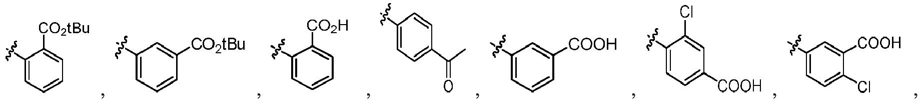 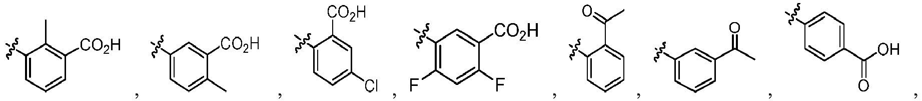 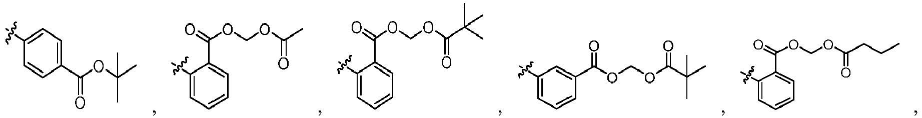 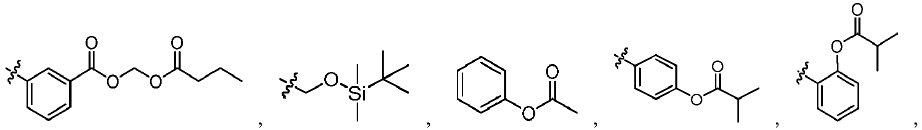 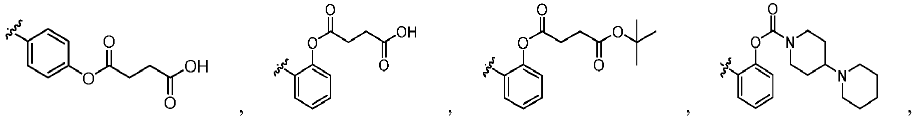 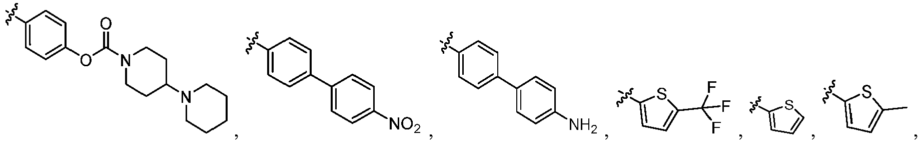 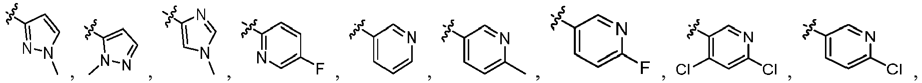 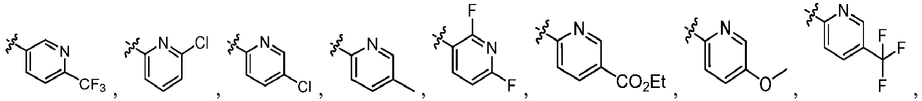 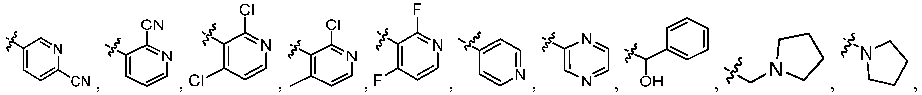 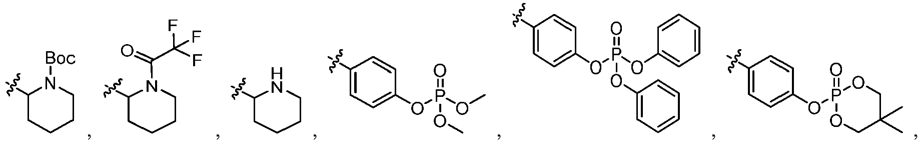 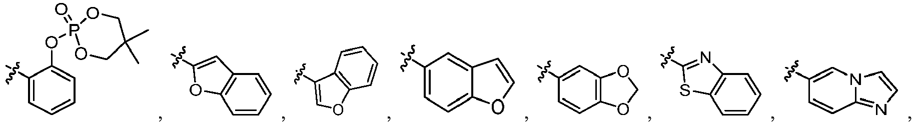 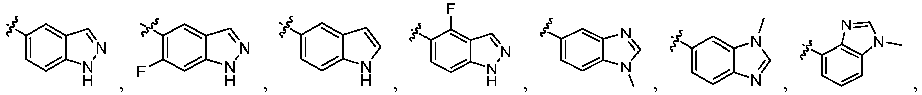 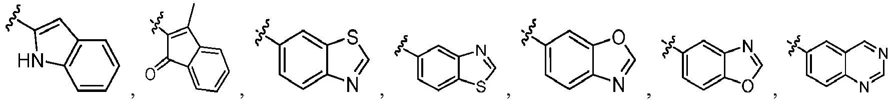 or

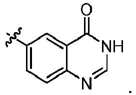

6. The compound according to claims 1-5 are defined as in formula (III), or a pharmaceutically acceptable salt, tautomer, racemate, racemate, stereoisomer, metabolite, metabolic precursor or prodrug thereof. 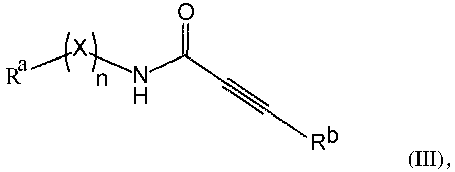
wherein, n is 0, 1 or 2, preferably n is 0 or 1, and preferably n is 1
preferably, the compound (I) is defined as in formula (I). 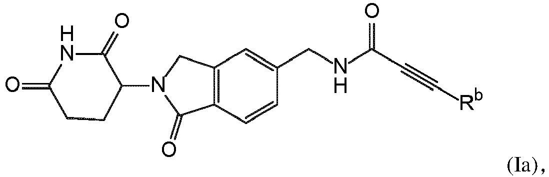
wherein, R^{b} is defined as in formula (I);
preferably, R^{b} is a R-substituted phenyl, wherein R is selected from methyl, ethyl, carboxyl, hydroxyl, halogen, cyano, amino, nitro, methoxycarbonyl, methoxycarbonyl, ethoxycarbonyl, tert-butoxycarbonyl, carboxy, phenyl, fluorine, chlorine, bromine, trifluoromethyl, nitrophenyl, aminophenyl, -CH₂CF₂, -C(O)CH₃,-C(O)CF₃, 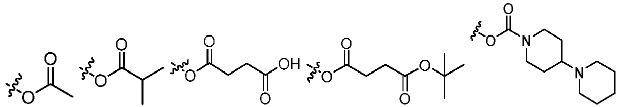 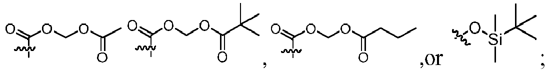
preferably, R is selected from, ethyl, fluorine, chlorine, bromine, nitro, amino, ethoxycarbonyl, trifluoromethyl, methoxy, tert-butoxycarbonyl, nitrophenyl, aminophenyl, -CH₂CF₂, or -C(O)CH₃.

7. The compound selected from the following compounds, or a pharmaceutically acceptable salt, tautomer, racemate, racemate, stereoisomer, metabolite, metabolic precursor, or prodrug thereof:

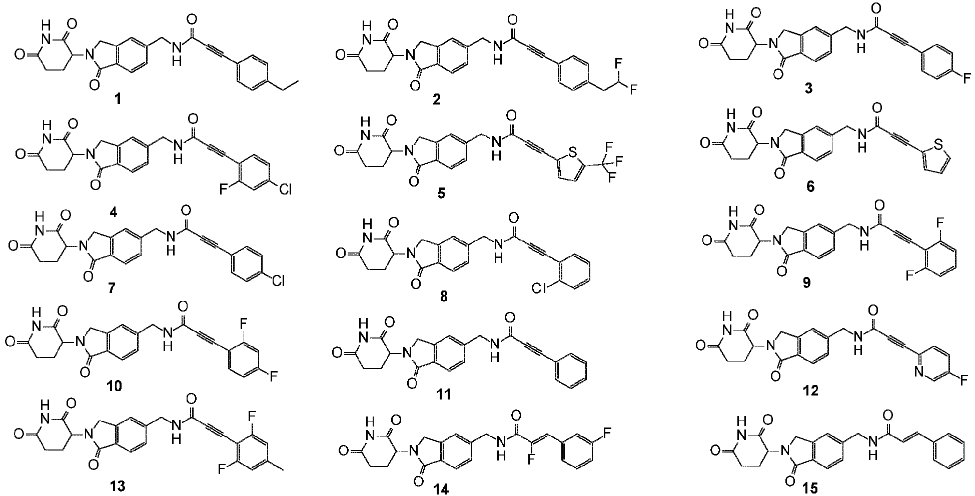

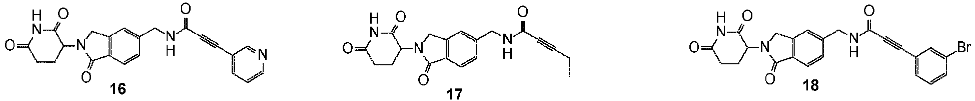

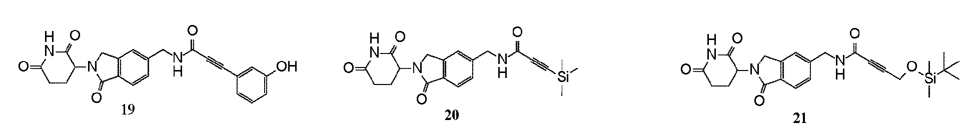

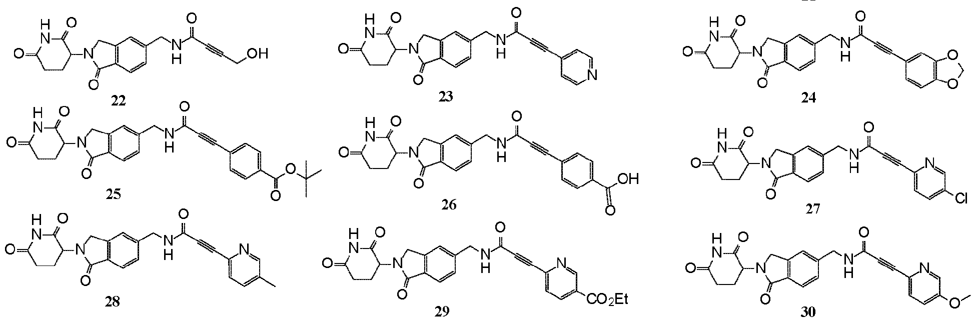

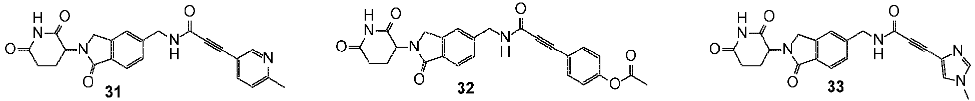

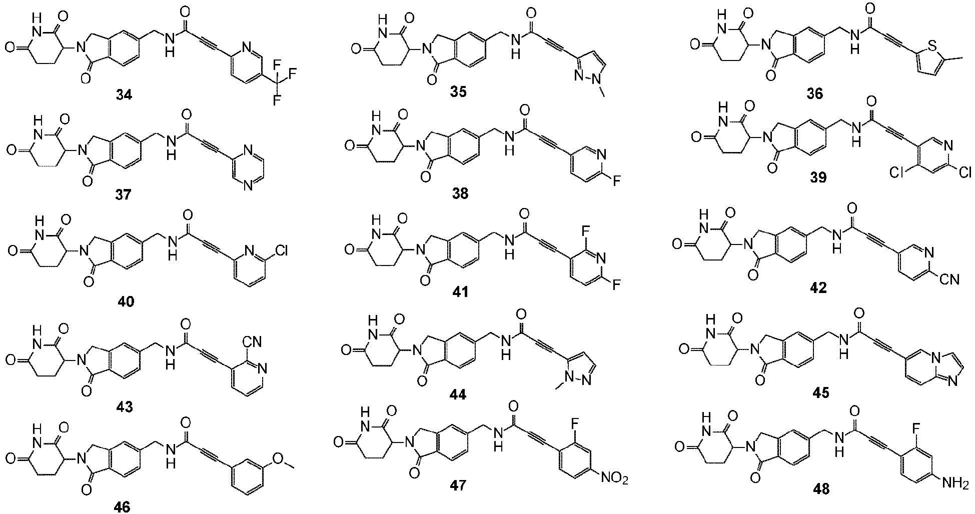

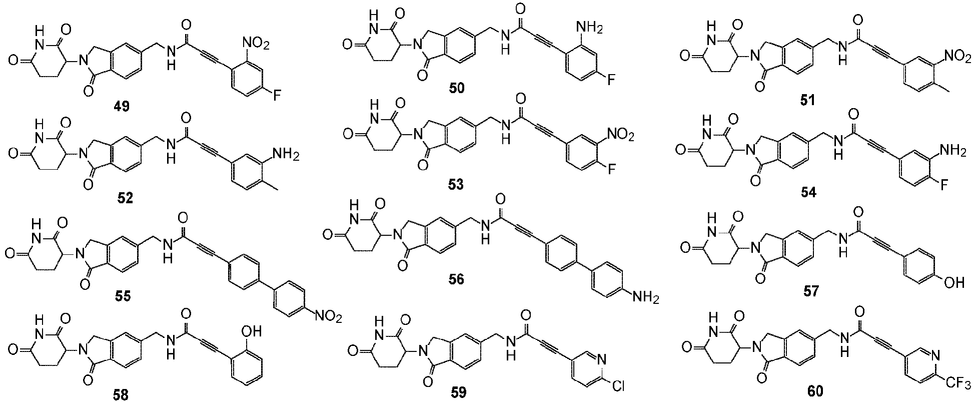

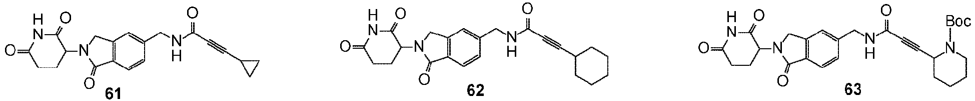

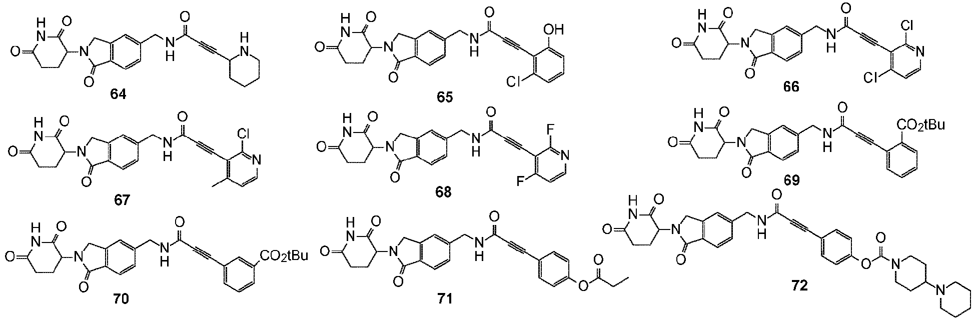

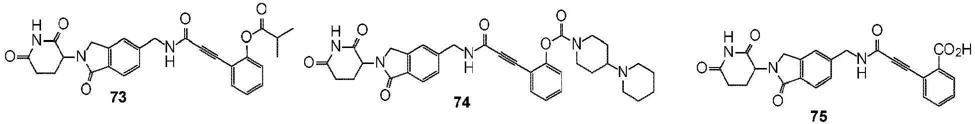

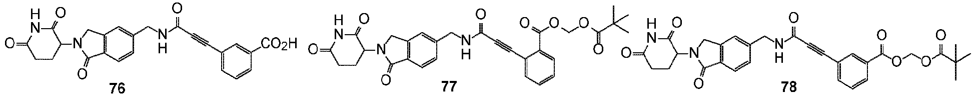

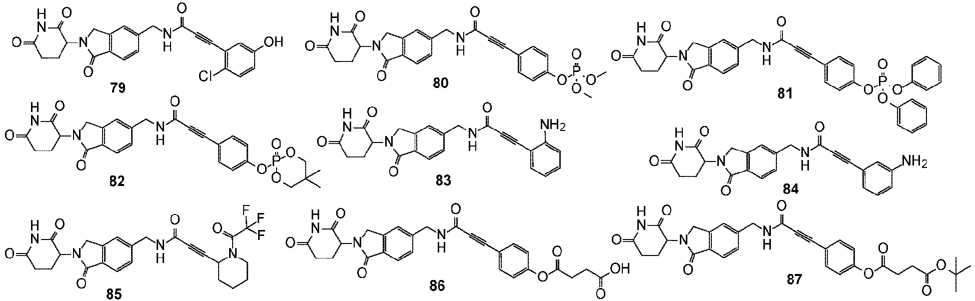

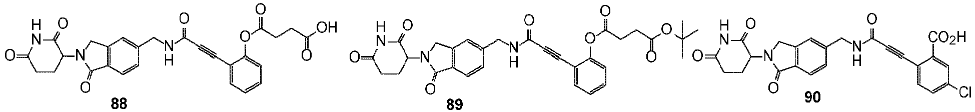

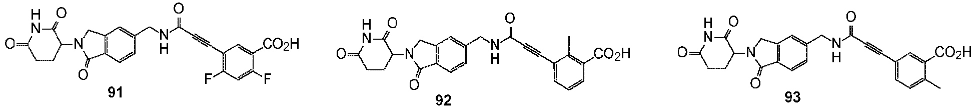

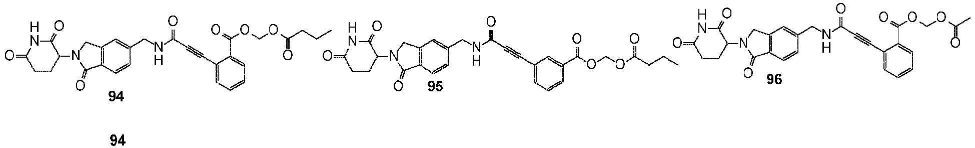

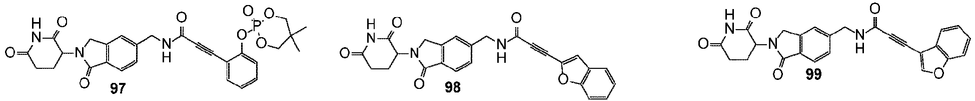

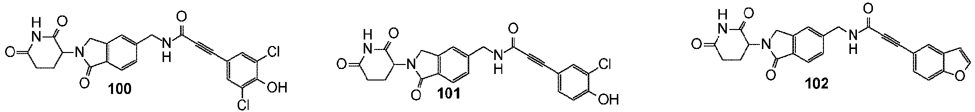

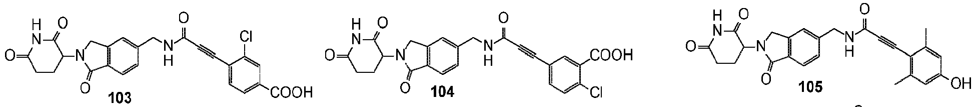

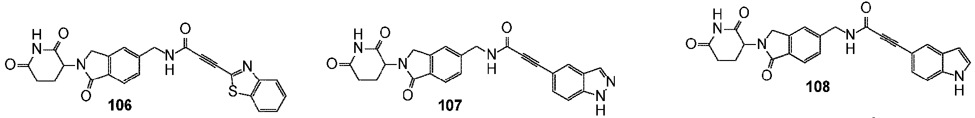

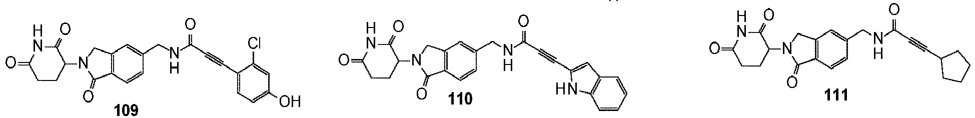

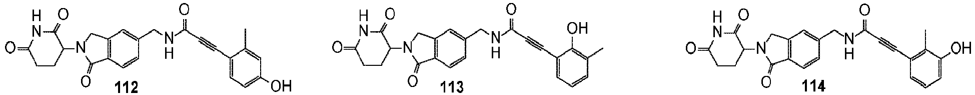

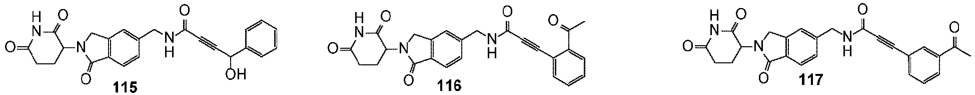

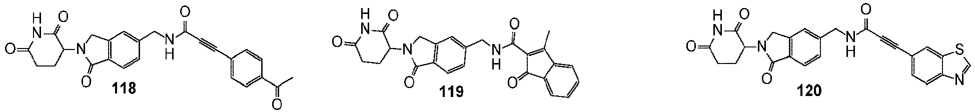

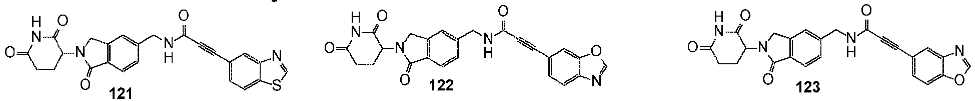

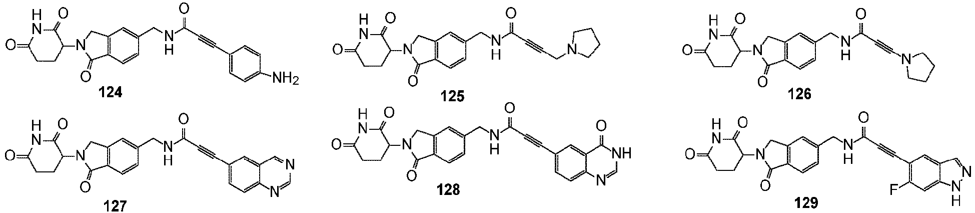

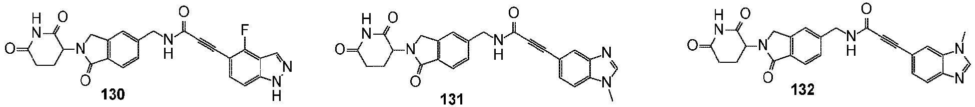

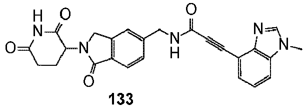

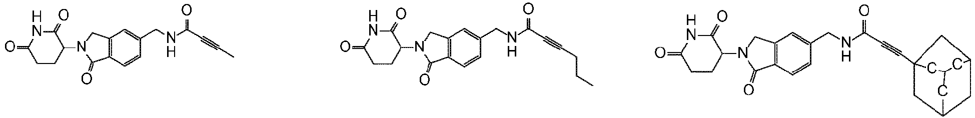

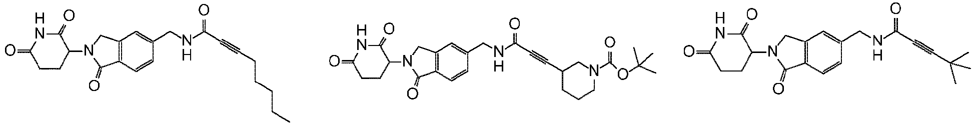

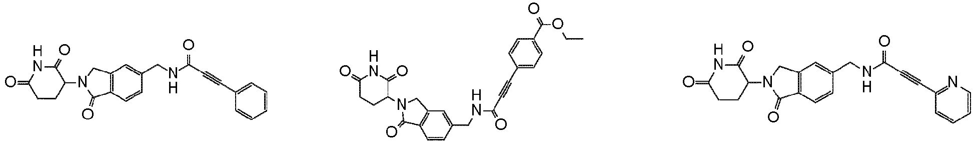

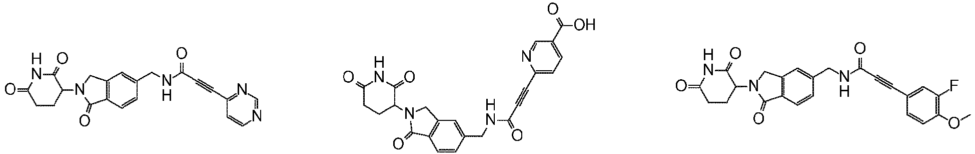

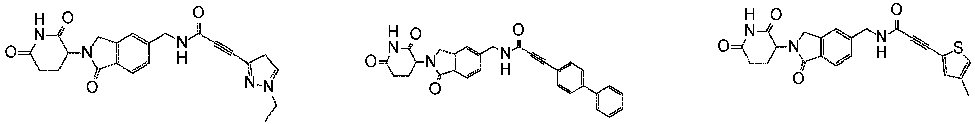

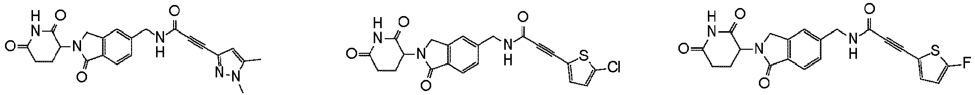

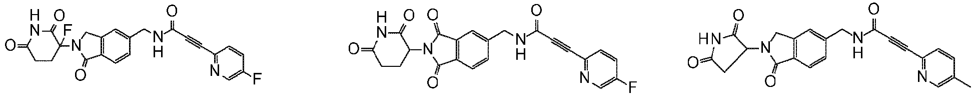

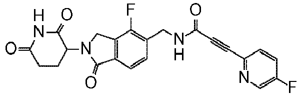

8. The method for preparing any one of the compounds of claims 1-7, wherein the following steps are comprising: 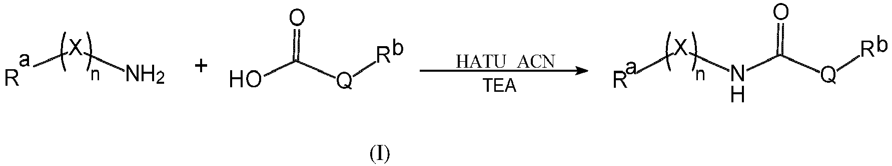 Amine compound, carboxylic acid compound, HATU, and TEA are dissolved in acetonitrile, then stirred and reacted at room temperature, the reaction solution is concentrated under reduced pressure, extracted, combined with organic phases, concentrated, and purified to obtain the compound of formula (I).

9. A pharmaceutical composition comprising the compound according to any of claims 1 to 8, or a pharmaceutically acceptable salt, a tautomer, a racemate, a racemate, a stereoisomer, a metabolite, a metabolic precursor or prodrug thereof, and a pharmaceutically acceptable excipient.

10. The use of the compound according to claims 1-7, or a pharmaceutically acceptable salt, tautomer, mesomer, racemate, stereoisomer, metabolite, metabolic precursor, or prodrug thereof, or the pharmaceutical composition comprising the compound according to claim 9, in the preparation of a drug for preventing or treating GSPT1-mediated diseases or disorders;
preferably, the GSPT1-mediated diseases or disorders include cancer, viral infection, aging, immune diseases, and neurological diseases, wherein the cancers are selected from acute myeloid leukemia, liver cancer, acute lymphoblastic leukemia, bladder cancer, bone cancer, brain cancer, breast cancer, cervical cancer, chorionic cancer, chronic lymphocytic leukemia (CLL), chronic myeloid leukemia (CML), colon cancer, endometrial cancer, esophageal cancer, gallbladder cancer, gastric cancer, gastrointestinal stromal tumor, head and neck cancer, Hodgkin lymphoma, Laryngeal cancer, leukemia, lung cancer, melanoma, multiple myeloma, ovarian cancer, pancreatic cancer, prostate cancer, rectal cancer, kidney cancer, sarcoma, skin cancer, small cell lung cancer, testicular cancer, throat cancer, thyroid cancer, uterine cancer;
preferably, the lung cancer is non-small cell lung cancer;
preferably, the sarcoma is selected from Kaposi's sarcoma, soft tissue sarcoma, mesothelioma, osteosarcoma, non-Hodgkin lymphoma;
preferably, the viral infections include SARS-CoV-2, HCoV-229E, HCoV-OC43, HCoV-NL63, HCoV-HKU1, SARS-CoV, MERS-CoV, SADS-COV, PEDV, PDCoV, FIPV virus, hepatitis B virus, HIV virus, Ebola virus, ASFV virus infection;
preferably, the GSPT1-mediated condition or disorder is selected from acute myeloid leukemia or liver cancer or coronavirus infection.

11. The use of compound according to claims 1-7, or a pharmaceutically acceptable salt, tautomer, racemate, racemate, stereoisomer, metabolite, metabolic precursor, prodrug thereof, or the pharmaceutical composition comprising the compound according to claim 9 for the manufacture of GSPT1 degrader.

12. A method of degrading GSPT1 protein in patient, comprising administering to patient a compound of any one according to claims 1-8, or a pharmaceutically acceptable salt, tautomer, racemate, racemate, stereoisomer, metabolite, metabolic precursor, or prodrug thereof, or a pharmaceutical composition comprising the compound according to claim 11.

13. A method for the treatment of a condition or disorder caused by the accumulation of GSPT1 protein in patient, comprising administering to the patient a compound according to claims 1-7, or a pharmaceutically acceptable salt, tautomer, racemate, racemate, stereoisomer, metabolite, metabolic precursor, or prodrug thereof, or a pharmaceutical composition comprising the compound according to claim 9;
preferably, the GSPT1-mediated diseases or disorders include cancer, viral infection, aging, immune diseases, and neurological diseases, wherein the cancers are selected from acute myeloid leukemia, liver cancer, acute lymphoblastic leukemia, bladder cancer, bone cancer, brain cancer, breast cancer, cervical cancer, chorionic cancer, chronic lymphocytic leukemia (CLL), chronic myeloid leukemia (CML), colon cancer, endometrial cancer, esophageal cancer, gallbladder cancer, gastric cancer, gastrointestinal stromal tumor, head and neck cancer, Hodgkin lymphoma, Laryngeal cancer, leukemia, lung cancer, melanoma, multiple myeloma, ovarian cancer, pancreatic cancer, prostate cancer, rectal cancer, kidney cancer, sarcoma, skin cancer, small cell lung cancer, testicular cancer, throat cancer, thyroid cancer, uterine cancer;
preferably, the lung cancer is non-small cell lung cancer;
preferably, the sarcoma is selected from Kaposi's sarcoma, soft tissue sarcoma, mesothelioma, osteosarcoma, non-Hodgkin lymphoma;
preferably, the viral infections include SARS-CoV-2, HCoV-229E, HCoV-OC43, HCoV-NL63, HCoV-HKU1, SARS-CoV, MERS-CoV, SADS-COV, PEDV, PDCoV, FIPV virus, hepatitis B virus, HIV virus, Ebola virus, ASFV virus infection;
preferably, the GSPT1-mediated condition or disorder is selected from acute myeloid leukemia or liver cancer or coronavirus infection.
